(19) Europäisches Patentamt
European Patent Office
Office européen des brevets

(11) Publication number: **0 345 068 B1**

# (12) EUROPEAN PATENT SPECIFICATION

(45) Date of publication of patent specification: **15.03.95**

(51) Int. Cl.[6]: **C07C 237/04**, C07C 229/14, C07C 223/02, C07C 215/06, A61K 31/165, A61K 31/195, A61K 31/215, C07D 333/20

(21) Application number: **89305551.7**

(22) Date of filing: **02.06.89**

The file contains technical information submitted after the application was filed and not included in this specification

## (54) Agonist of gamma-aminobutyric acid.

(30) Priority: **03.06.88 GB 8813185**

(43) Date of publication of application:
**06.12.89 Bulletin 89/49**

(45) Publication of the grant of the patent:
**15.03.95 Bulletin 95/11**

(84) Designated Contracting States:
**AT BE CH DE ES FR GR IT LI LU NL SE**

(56) References cited:
**EP-A- 99 685**
**EP-A- 0 056 082**
**GB-A- 1 493 416**
**EP-A- 289 070**
**FR-A- 2 531 950**
**US-A- 3 712 924**

**JOURNAL OF MEDICINAL CHEMISTRY, vol. 18, no. 3, 1975, pages 278-284, Washington, DC, US; K. J. HOFFMANN et al, "Fibrin-stabilizing factor inhibitors. 12.5-Dibenzylaminopentylamine and related compounds, a new type of FSF inhibitors"**

(73) Proprietor: **JOHN WYETH & BROTHER LIMITED**
**Huntercombe Lane South**
**Taplow**
**Maidenhead**
**Berkshire, SL6 0PH (GB)**

(72) Inventor: **Minchin, Michael Christopher Warren**
**149 Divinity Road**
**Oxford**
**Oxfordshire OX4 1LP (GB)**
Inventor: **White, John Frederick**
**22 Starling Close**
**Woosehill**
**Wokingham**
**Berkshire (GB)**

(74) Representative: **Wileman, David Francis Dr. et al**
**c/o Wyeth Laboratories**
**Huntercombe Lane South**
**Taplow**
**Maidenhead**
**Berkshire SL6 OPH (GB)**

**EP 0 345 068 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, vol. 96, no. 15, 12 April 1982, page 61. column 1, abstract no. 115 836b, Columbus, Ohio, USA; R. K. SATSANGI, "Pharmacological evaluation of six new derivatives of gamme-aminobutyric acid, especially as antitremor agents"**

**ARCHIV DER PHARMAZIE, vol. 314, no. 7, July 1981, pages 648-649, Weinheim, DE; K. DELLER et al, "Eine neue Synthese von 4-Aminobuttersäureamid"**

**CHEMICAL ABSTRACTS, vol. 106, no. 11, 16 March 1987, page 17, column 2, abstract no. 78 248p, Columbus, Ohio, USA; M. DZIEDUSZYCKA et al, "New N-amino acid derivatives of daunorubicin"**

**BRITISH JOURNAL OF PHARMACOLOGY, vol. 76, 1982, pages 291-298, The Macmillan Press, Ltd.; J. M. BOWDLER et al, "Regional rat brain benzodiazepine receptor number and gamma-aminobutyric acid concentration following a convulsion"**

**THE JOURNAL OF PHARMACOLOGY AND EXPERIMENTAL THERAPEUTICS, vol. 241, no. 1, 1987, pages 251 - 257; B. SCATTON et al, "Fengabine, a novel antidepressant GABAergic agent. II. Effect on cerebral noradrenergic, serotonergic and GABAergic transmission in the rat"**

**MOLECULAR PHARMACOLOGY, vol. 19, 1981, pages 27 - 30; M. J. W. BRENNAN et al, "Inhibition of gamma-aminobutyric acid release by gamma-aminobutyric acid agonist drugs"**

**Bull. Soc. Chim. Belg., 1977, 86 (1-2), 3508**

## Description

This invention relates to use of a new pharmacological activity and to certain amines possessing said pharmacological activity, to processes for preparing them and to pharmaceutical compositions containing them. More particularly this invention relates to the treatment of depression.

In the UK the annual referral rate for depression is around 300-400 per $10^5$ population of whom 10-15% require hospitalisation. At present the most effective and safe treatment for severe depression involves electroconvulsive therapy (ECT) where the patient receives a series of controlled electric shocks. However such treatment understandably engenders an atavistic fear and apprehension in many patients. It also has undesirable side-effects, notably disturbance of memory.

ECT is also expensive and time-consuming to administer requiring the presence of specialist doctors such as psychiatrists and anaesthetists. As an alternative to ECT, drug therapy provides a more acceptable treatment for the patient but at the present time such therapy has not displaced ECT as the optimal treatment in severe cases because it is not always effective. There is therefore a need for new drugs for the treatment of depression, especially drugs having new modes of action mimicking ECT.

The mode of action of ECT remains unknown but in recent years much has been learnt about the biological effects of electroconvulsive shock (ECS) in animals. In particular, repeated ECS, given in ways closely mimicking those used to administer ECT clinically elicits in rodents changes in monoamine functions.

These include: increased 5-HT-mediated behaviour, increased dopaminergic behaviour and depressed beta-adrenoceptor binding and sensitivity of the coupled adenylate cyclase. The last is also seen following chronic treatment with a number of antidepressant drugs.

The effects of repeated ECS are presumably a response or adaptation to the acute effects of the seizures. Among these acute effects are a marked change in the release, synthesis and level of gamma aminobutyric acid (GABA) in the brain. - see Green A.R. et al, British J. Pharmacol., 92, 5-11 and 13-18 (1987) and Bowdler et al, ibid, 76, 291-298 (1982).

GABA is one of the most widespread and abundant transmitters in the mammalian central nervous system and plays a major role in the control of brain excitability. It is similarly implicated in the benzodiazepine-mediated relief of anxiety. Recently, evidence has come to light which suggests that GABA transmission may also be involved in the therapeutic effects of some antidepressant treatments. In particular, new compounds designed as GABA agonists (eg. fengabine and progabide) have been shown in preliminary clinical trials to have antidepressant activity (vide infra). Taken together, these findings suggest that interventions directed specifically at GABA transmission may provide the basis of novel therapies for the treatment of affective disorders.

At present three GABA receptors have been identified in the central nervous system. These are (1) a $GABA_A$-receptor known to be mainly postsynaptic and mediating inhibition of neuronal firing - see for example Stephenson, F.A. Biochem, J., 249 pp 21-32 (1988); (2) a $GABA_B$ receptor located presynaptically and mediating the inhibition of release of a number of neuro-transmitters, eg. noradrenaline and aspartic acid, but not GABA - see for example Bowery, N.G. et al, Nature, 283, 92-94 (1980); and (3) a GABA autoreceptor which modulates the release of GABA from neurones - see for example Mitchell, P.R., and Martin, I.L. Nature, 274 904-905 (1978); Arbilla, S. Kanal, J.L and Langer, S.Z. Eur. J.Pharmac., 57, 211-217 (1979) and Brennan M.J.W. et al, Molec. Pharmac., 19, 27-30 (1981).

The pharmacological importance of these receptors is currently a subject of investigation with a major part of the work involving the search for anticonvulsant drugs with a mode of action involving $GABA_A$ receptors. Two drugs acting on GABA receptors, progabide and fengabine, have also been shown to possess antidepressant effects in preliminary clinical trials - see P.L. Morselli et al, L.E.R.S. Vol 4 (1986) pp 119-126 and B.Scatton et al, Journal of Pharm. and Exp. Therapeutics., 241, 251-257 (1987). The latter workers showed that fengabine possessed a biochemical mode of action different from that of conventional antidepressants but that the mechanism whereby fengabine exerted its antidepressant actions was not yet clear. It was thought to derive from a GABAergic action, most likely at $GABA_A$ receptors.

In the case of progabide, Morselli et al also attributed the antidepressant effect to an increased GABAergic transmission.

We provide evidence herein that the antidepressant effect of progabide and fengabine is in fact due to their agonist action at the GABA autoreceptor.

The GABA autoreceptor is capable of regulating the release of GABA from GABAergic neurons which means an agonist at the autoreceptor would decrease the GABA release hence decreasing GABA function ie. an action opposite to that of $GABA_A$ agonists.

As far as we are aware it has not been suggested even remotely that the GABA autoreceptor is linked to an antidepressant effect. Previously the autoreceptor was believed to have the same pharmacology as the $GABA_A$ site - see Molec. Pharm, 19,27-30 (1981). We have now surprisingly found that the GABA autoreceptor has its own distinct pharmacology and that there are compounds having selective agonist activity at the GABA autoreceptor. These compounds have valuable medical uses.

Accordingly in one aspect this invention provides use of a compound in the preparation of a medicament for treating depression characterised in that the compound has selective agonist activity at GABA autoreceptors.

There is evidence that compounds acting at the benzodiazepine receptor as inverse agonists decrease GABA function in the brain and thus increase acetylcholine transmission. In addition, probably as a consequence of these actions, they facilitate memory in animals and man (see Sarter. M. et al. Trends in Neuroscience, 11 13-17, 1988). Compounds acting selectively as GABA autoreceptor agonists would be expected to have similar actions.

Accordingly, another aspect of this invention provides use of a compound having selective agonist activity at GABA autoreceptors in the preparation of a medicament for treating senile dementia and other forms of memory impairment.

In this invention it is preferred that the compound having GABA autoreceptor agonist activity is selective in that it has little or no activity at $GABA_A$ receptors.This is because $GABA_A$ agonist activity would tend to counteract the effect of the autoreceptor agonist. $GABA_A$ antagonist activity tends to cause convulsions. For example the selectivity for the GABA autoreceptor relative to the $GABA_A$ receptor is preferably greater than 100, most preferably greater than 1000.

We have found that one general class of compounds in which selective GABA autoreceptor agonist activity can be observed is tri-substituted amines in which one of the substituents is a 3- or 4-alkanoic acid which itself may be substituted and derivatives thereof, such as an amide or substituted amide. Within such a general class the observation of GABA autoreceptor activity depends on the nature of the substituents.

More particularly this invention also relates to mono- and di-(arylalkyl)amine derivatives and related compounds possessing pharmacological activity, and intermediates of closely related structure.

Certain di-(arylalkyl)amine derivatives are known in the literature. The reaction of dibenzylamine with 3-chloropropyl cyanide to give 3-dibenzylaminopropyl cyanide and subsequent hydrolysis of the cyanide to give 4-dibenzylaminobutyric acid hydrochloride is described by M E Gittos and W Wilson in Journal of the Chemical Society, (1955) 2371-2376. The latter compound is also described in Chemical Abstracts 106, 78248(1987) where it is used to prepare derivatives of daunorublcin. 4-Dibenzylaminobutyramide and 4-dibenzylaminopropyl cyanide are disclosed in Chemical Abstracts 97, 163499m as intermediates in the preparation of 4-aminobutyric acid amide hydrochloride. Various ω-dibenzylaminoalkyl cyanides and related compounds are disclosed in J. Med. Chem., 1975 18(3) 278-284 as starting materials for fibrin stabilizing factor inhibitors.

Accordingly this invention also provides use of a compound which acts selectively as an agonist of GABA at GABA autoreceptors, said compound being of formula:

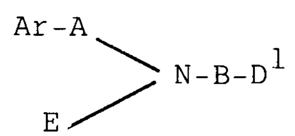

(Ia)

or a salt thereof, wherein E represents $C_{1-6}$ alkyl or a group $Ar^1$-$A^1$-; Ar and $Ar^1$ are the same or different aryl groups (including heteroaryl) which are optionally substituted, eg. by one or more substituents commonly used in pharmaceutical chemistry such as $C_{1-6}$ alkyl, $C_{1-6}$ alkoxy, halogen, halo $C_{1-6}$ alkyl, halo $C_{1-6}$ alkoxy, cyano, amino (including substituted amino eg. mono- or di-$C_{1-6}$ alkyl amino) and nitro: A and $A^1$ are the same or different alkylene groups having one or two carbon atoms linking Ar or $Ar^1$ to N and optionally substituted by $C_{1-6}$ alkyl and/or optionally substituted aryl, B is an alkylene group of 3 carbon atoms, which may be substituted by $C_{1-6}$ alkyl; $D^1$ represents $CONR^1R^2$ or COOH where $R^1$ and $R^2$ are independently hydrogen, $C_{1-6}$ alkyl or aralkyl of 7 to 12 carbon atoms, in the preparation of a medicament for the treatment or prevention of depression or senile dementia or other forms of memory impairment.

The compounds of formula Ia can be prepared from intermediates of formula Ib

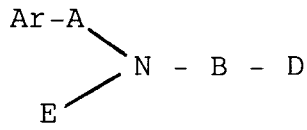

(Ib)

wherein Ar, A, E and B are as defined above and D represents CN, COhal, $CH_2OH$, CHO or an ester function COOR.

Examples of Ar and $Ar^1$ are mono- or bi-cyclic aryl groups such as carbocyclic aryl groups of 6 to 10 carbon atoms (eg. phenyl or naphthyl) and heteroaryl groups of 5 to 10 ring atoms in which the heteroatom is selected from oxygen, nitrogen and sulphur (eg. pyridine, furan, thiophene) or aromatic groups containing two or more such heteroatoms (eg. thiazolyl). Bicyclic heteroaryl groups are exemplified by quinoline and benzofuran.

Examples of A and $A^1$ are independently $-(CH_2)m-$ optionally substituted by $C_{1-6}$ alkyl and/or aryl where m is 1 or 2. Preferably A and $A^1$ are independently $-CHR^3-$ where $R^3$ is hydrogen, $C_{1-6}$ alkyl, eg. methyl or ethyl, or optionally substituted aryl as defined for Ar, eg. phenyl. Examples of B are $-CH_2CH_2CH_2-$ which may be substituted by $C_{1-6}$ alkyl such as methyl, eg. B represents $-CH(CH_3)CH_2CH_2-$ or $-CH_2CH(CH_3)CH_2-$.

Examples of $R^1$ and/or $R^2$ are hydrogen, methyl, ethyl, propyl and benzyl.

Some compounds of formula Ia and Ib are known compounds. Accordingly in a further aspect this invention provides novel compounds of formula Ia as defined above with the provisos:

(i) neither Ar-A or E is unsubstituted benzyl,

(ii) when E is $C_1$-$C_6$ alkyl, Ar is not phenyl, substituted phenyl or a 5 or 6 membered heteroaromatic group,

and

(iii) when E is phenethyl, Ar is not substituted benzyl.

This invention also provides novel compounds of formula Ib for use as intermediates for preparing the novel compounds of formula Ia as defined above with the above provisos and the additional provisos:

(i) when D is COOR then E is other than $C_1$-$C_6$ alkyl,

(ii) when D is COOR then Ar-A- and $Ar^1$-$A^1$- are not nitrobenzyl.

Compounds of formula Ib wherein D is CN, COhal, $CH_2OH$, CHO or COOR where R is as defined above are useful as intermediates to compounds of formula Ia wherein $D^1$ is COOH or $CONR^1R^2$.

The compounds of formula Ia as defined above possess pharmacological activity especially activity affecting the nervous system. In particular the compounds of formula Ia are inhibitors of gamma aminobutyric acid (GABA) release from nerve terminals via action on the GABA autoreceptor.

A number of compounds have previously been shown to be agonists at the GABA autoreceptor, for example muscimol, isoguvacine and THIP (see Merck Index 1983 No. 9214) but such compounds are non-selective in that they are also active at other GABA receptors (ie. $GABA_A$ and /or $GABA_B$). As far as we are aware no medical use has been attributed to the above-mentioned compounds based on their GABA autoreceptor activity.

Compounds showing selective properties at the GABA autoreceptor are desirable since additional activity at the other GABA receptors would cause many side effects such as sedation and adverse muscle tone actions.

The compounds of formula Ia demonstrate activity at GABA autoreceptors, more specifically they demonstrate agonist activity as shown by standard <u>in vitro</u> test procedures. Advantageously compounds of formula Ia appear to be selective in that they display little or no activity at $GABA_A$ or $GABA_B$ receptors. The following test procedures were used to measure activity at (a) GABA autoreceptors and $GABA_B$ receptors by inhibition of potassium-evoked GABA and noradrenalin release from rat cortex <u>in vitro</u> (Procedure 1);
and (b) $GABA_A$ receptors by enhancement of [3H]-flunitrazepam binding in rat cortex <u>in vitro</u> (Procedure 2):

<u>Procedure (1)</u>

Slices (0.25 x 0.25 x 2.0 mm) of rat cerebral cortex are prepared using a McIlwain tissue chopper. The slices are incubated in Krebs-Henseleit solution containing $[^3H]$-GABA ($10^{-7}$M) and $[^{14}C]$-noradrenaline

($10^{-7}$M) in the presence of amino-oxyacetic acid (AOAA) ($10^{-5}$M), pargyline ($10^{-6}$M) and ascorbic acid ($10^{-4}$M), for 20 minutes at 37°C, rinsed with 5 ml aliquots of Krebs-Henseleit solution and transferred to 10 superfusion chambers (volume 300µl). The slices are continuously superfused with Krebs-Henseleit solution (0.4 ml $min^{-1}$) containing AOAA ($10^{-5}$M) and fractions of the superfusate collected every 4 minutes. Transmitter release is induced by 4 minute exposure to a Krebs-Henseleit solution containing 25 mM potassium (with concomitant reduction in sodium to maintain osmolarity) after 68 ($S_1$) and 92 ($S_2$) minutes of superfusion. The compound under study is added to the superfusing medium 20 minutes prior to the second potassium stimulation. The residual radioactivity in the slices at the end of the experiment together with that in the superfusate fractions is measured by liquid scintillation counting using a dual label programme for tritium and carbon-14.
Calculations: The amount of radioactivity (either tritium or carbon-14) in each fraction is expressed as a percentage of the respective total radioactivity in the tissue at the start of the respective collection period. The amount of radioactivity released above basal by the increased potassium is calculated and the ratio S2/S1 obtained. The S2/S1 ratio from drug-treated slices is expressed as a percentage of the control S2/S1 ratio. For compounds achieving inhibition of 30% or more $pD_2$ values are calculated from plots of inhibition of release versus concentration of drug. Failure to inhibit the release of noradrenaline indicates that the molecule has no $GABA_B$ agonist activity.

Procedure (2)

Cortices from six or more rats are excised and homogenised in 50 volumes (volume/weight) 50mM Tris buffer (pH 7.4 at 37°C) using a Polytron homogeniser on speed 5 for 30 seconds. Tissue is kept at O°C throughout the procedure. The homogenate is centrifuged at 40,000 x g for 15 minutes and the pellet resuspended (Polytron 5, 10 sec.) in 50 volumes (original weight) of Tris buffer. Centrifugation and resuspension is repeated followed by incubation at 37°C for 10 minutes. The homogenate is centrifuged (40,000 x g, 15 min), resuspended in 10 volumes (original weight) Tris and stored below -20°C for more than 24 hours.

On the day of the experiment, membranes are thawed at 37°C and made up to 20 volumes (original weight) with Tris Krebs (20mM, see below). The membranes are homogenised (Polytron 5, 30 sec), incubated at 37°C for 15 minutes and centrifuged at 20,000 x g for 10 minutes. This wash and resuspension is repeated two more times with the final pellet resuspended in 100 volumes (original weight) Tris Krebs ready for use.

The test compound (50µl) and 20nM (final concentration) [$^3$H]-flunitrazepam (50µl) are dispensed in triplicate. The reaction is started by the addition of 900µl of membrane preparation. After a 30 minute incubation at 37°C the reaction is stopped by filtration through Whatman GF/B filters under reduced pressure using a Brandell Cell Harvester, with two 7.5 ml filter washings. The radioactivity retained on the filters is measured by liquid scintillation counting.
Tris Krebs composition:
NaCl-136mM, KCl-5mM, $MgSO_4$-2mM, $KH_2PO_4$-2mM, $CaCl_2$-2mM, Tris buffer (pH 7.4 at 37°C)-20mM, ascorbic acid-lmM, disodium ethylenediamine tetraacetic acid-lmM.

Calculations: Results are calculated as follows:

$$\frac{\text{dpm sample (enhanced binding)}}{\text{dpm control (non-enhanced binding)}} \times 100 = \%\ \text{control}$$

RESULTS

In the aforementioned tests the following representative compounds gave the results shown:

| Compound | GABA autoreceptor pD2 values | Inhibition of release of noradrenaline at $10^{-5}M$ | Enhancement of [3H]-flunitrazepam binding |
|---|---|---|---|
| 4-[N,N-Bis-(4--Chlorobenzyl)-amino]butyric acid | 7.0 | 0 | 0 |
| 4-[N,N-Bis-(4-Chlorobenzyl)-amino]butyramide | 6.6 | 0 | 0 |
| 4-(N,N-Dibenzylamino)butyramide | 7.0 | >20% | 0 |
| 4-(N,N-Dibenzylamino)butyric acid | 7.1 | 20% | 0 |

| | | | |
|---|---|---|---|
| 4-[N,N-Bis-(4-Methylbenzyl)-amino]butyric acid | 7.4 | 10% | 0 |
| 4-[N,N-Bis-(3,4-dichlorobenzyl)-amino]butyric acid | 7.5 | 0 | 0 |
| 4-[N,N-Bis-(4-methoxybenzyl)-amino]butyramide | 6.0 | 0 | 0 |
| 4-[(N-(p-chloro-benzyl)-N-methyl) amino] butyramide | 7.5 | 0 | 0 |

In the aforementioned tests fengabine was found to have a $pD_2$ value of 8.0 at the GABA autoreceptor and little or no activity at $GABA_A$ and $GABA_B$ sites in vitro. We attribute the antidepressant activity observed for fengabine to its potent selective GABA autoreceptor activity. Progabide was also active at the GABA autoreceptor. We make no claim to the use of fengabine or progabide in the preparation of a medicament for use as an antidepressant.

The compounds were also tested for their effect on a GABA synapse in vivo in the following procedure:

Procedure 3

Experiments were performed on male albino rats (240-280g) lightly anaesthetized with urethane (1.2-1.4 g/kg i.p.) or Halothane (0.7-1.0% in $0_2$). The animal's temperature was maintained between 36-38°C via a thermostatically controlled heating blanket.

The caudate nucleus and substantia nigra were approached dorsally after exposing the overlying cortex. Pulsations of the brain and cerebral oedema were minimised by allowing cerebral spinal fluid to leak from a cisternal puncture. The exposed cortex was bathed in warm liquid paraffin throughout the experiment. A coaxial bipolar stimulating electrode (tip separation 0.25mm) was positioned stereotaxically in the caudate nucleus such that the tip of the electrode corresponded to the area delineated by the co-ordinates L 2.5; A 8.5-95; D 5. (Paxinos and Watson 1986). Square wave pulses (50-300$\mu$A 0.1-0.2msec duration) were delivered at 0.5-1.0Hz via the stimulating electrode to evoke inhibitory, GABA-mediated synaptic responses in nigral neurones.

Extracellular recordings were obtained from single neurones in the ipsilateral substantia nigra via either the centre barrel (3.5M NaCl) of a multibarrelled microelectrode or a single barrelled electrode (3.5M NaCl), attached to but protruding 10-20 $\mu$ beyond, a multibarrelled electrode. The electrodes were lowered into the substantia nigra via a remote controlled stepping micromanipulator such that the electrode tip corresponded to the stereotaxic co-ordinates L1.5-2.5; A 3.0-4.0; D 7.0-8.5 (Paxinos and Watson, 1986). Unit firing was amplified, continuously monitored on an oscilloscope, electronically counted and displayed by a chart recorder and fed into a computor for the generation of peristimulus time histogrames (PSTH) of synaptic responses. Each outer barrel of the multibarrelled electrode was filled with aqueous solutions of the following which were administered in the vicinity of the recorded neurones using standard microiontophoretic techniques: Compound of Example 5 (0.1M or 0.01M in 0.9% NaCl, pH 5.5), GABA (0.2M, pH 3.5), glycine (0.2M, pH3.5), dl-homocysteate (DLH) (0.2M, pH 7.2), N-methyl-D-Aspartate (NMDA) (0.05M in

0.165M NaCl, pH 7.0), quisqualate (0.02M in 0.165M NaCl, pH7.0), kainate (0.2M in 0.165M NaCl, pH 7.0) and bicuculline methochloride (BMC) (0.005M in 0.165M NaCl, pH 5.0). Effects of agonist drugs on the GABA autoreceptor could be measured as an attenuation of the synaptic inhibition evoked by stimulation of the caudate. $GABA_A$ receptor agonism could be tested by ejecting drug directly onto the nigral meurone in the absence of synaptic inhibition.

The location of the recording and stimulating electrodes in the brain were verified after each experiment by histological examination.

Reference Paxinos G & Watson C (1986) The rat brain in stereotaxic coordinates. Academic Press.

Result

In this test the compound of Example 5 almost abolished synaptically-evoked, GABA-mediated inhibition in the substantia nigra without affecting postsynaptic $GABA_A$ receptors, when it was ejected iontophoretically in the vicinity of nigral neurones. This action of the compound of Example 5 was reversible and is entirely consistent with a selective agonist action at the GABA autoreceptor

This invention also provides processes for preparing the compounds of the invention, including processes for preparing the pharmacologically active compounds of formula Ia via intermediates of formula Ib wherein D is CN, COOR, CHO, $CH_2OH$ or COhal.

Compounds of formulae Ia and Ib may be prepared by any one of the following processes:

a) reacting a compound of formula II

Ar-A-NH-E (II)

wherein Ar, E and A are as defined above with a compound of formula III:

hal-B-CN (III)

wherein B is as defined above and hal represents chlorine or bromine, to give a compound of formula Ib wherein D is CN,

or

b) carrying out a reductive alkylation of a compound of formula II as defined above using a compound of formula IV

$OHC-B^1-CN$ (IV)

wherein $B^1$ is an alkylene chain of 2 carbon atoms optionally substituted by $C_{1-6}$ alkyl, in the presence of a reducing agent such as sodium cyanoborohydride to give a corresponding compound of formula Ib wherein D is CN and B represents $-CH_2-B^1-$;

or

(c) reducing a compound of formula (V)

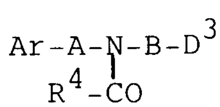

(V)

wherein $R^4$ is alkyl of 1 to 5 carbon atoms or $Ar^1-A^2-$; Ar, $Ar^1$, A and B are as defined above, $D^3$ represents CN, COOR or COOH and $A^2$ represents a direct bond or alkylene of 1 carbon atom optionally substituted by $C_{1-6}$ alkyl and/or aryl to give a corresponding compound of formula Ib wherein D is CN or COOR or formula Ia wherein $D^1$ is COOH and E is $R^4CH_2$ wherein $R^4$ is as defined above;

or

(d) reducing a compound of formula (VI)

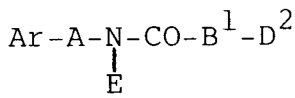

(VI)

wherein Ar, A and E are as defined above, $B^1$ is as defined in connection with formula IV and $D^2$ represents CN, COOR or CHO to give a corresponding compound of formula Ib wherein B is $-CH_2B^1-$ and D is CN, COOR or CHO,

or

(e) partially hydrolysing a compound of formula Ib wherein D is CN to give a corresponding compound of forumla Ia wherein $D^1$ is $CONH_2$;

or

(f) hydrolysing a compound of formula Ib wherein D is CN or formula Ia wherein $D^1$ is $CONH_2$ to give a corresponding compound of formula Ia wherein $D^1$ is COOH;

or

(g) reacting a compound of formula Ib wherein D is COOR or COhal with ammonia or an amine of formula $HNR^1R^2$ to give a corresponding compound of formula Ia wherein $D^1$ is $-CONR^1R^2$;

or

(h) hydrolysing an ester of formula Ib wherein D is COOR to give a carboxylic acid of formula Ia wherein $D^1$ is COOH:

or

(i) oxidising an aldehyde of formula Ib wherein D is CHO to give an acid of formula Ia wherein $D^1$ is COOH:

or

(j) oxidising an alcohol of formula Ib wherein D is $-CH_2OH$ to give an aldehyde of formula Ib wherein D is CHO or an acid of formula Ia wherein $D^1$ is COOH;,

or

(k) oxidising a compound of formula

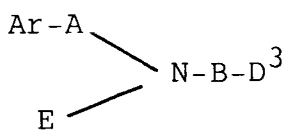

(VII)

wherein Ar, A, E and B are as defined above and $D^3$ is $-COCH_3$, $-C{\equiv}CH$ or $-CH=CH_2$ to give a compound of formula Ia wherein $D^1$ is COOH;

or

(l) hydrolysing a compound of formula

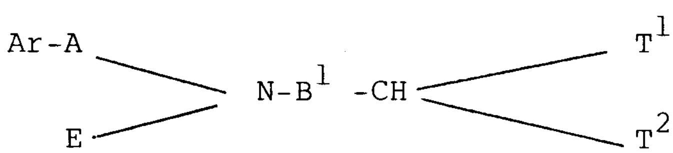

(VIII)

wherein Ar, A and E are as defined above,$T^1$ and $T^2$ are each independently an ester function, a nitrile or an acyl group, and $B^1$ is an alkylene group of 2 carbon atoms optionally substituted by $C_{1-6}$ alkyl to give a corresponding compound of formula Ia wherein B is $-B^1-CH_2-$ and $D^1$ is COOH;

or

(m) esterifying an acid of formula Ia wherein $D^1$ is COOH or acid halide of formula Ib wherein D is COhal to give an ester of formula Ib wherein D is-COOR;

or

(n) halogenating an acid of formula Ia to give an acid halide of formula Ib wherein D is COhal:

or

(o) reducing an acid of formula Ia wherein $D^1$ is COOH to give an alcohol of formula Ib wherein D is $CH_2OH$,

or

(p) acidifying a compound of formula Ia to give an acid addition salt thereof or neutralising an acid addition salt to give the free base form.

With regard to process (a) the reaction may be conveniently carried out in the presence of an inert solvent and a base such as a tertiary amine (eg. diisopropylethylamine) with heating if required. Examples of suitable inert solvents are dimethylformamide, acetonitrile and dimethylsulphoxide.

With regard to process (b) the reductive alkylation is conveniently carried out in an inert solvent, depending on the reducing agent, and without heating. When the reducing agent is sodium cyanoborohydride the solvent may be an aqueous alcohol such as aqueous ethanol. Catalytic hydrogenation may also be used, eg using Pd/C and an alcohol solvent, eg. ethanol.

Process (c) and (d) may both be carried out using a suitable reducing agent not affecting the $D^1$ or $D^2$ group for example ionic hydrogenation see Kursanor et al, Synthesis 1974, Vol 9, 633-651. Other reducing agents may be used, eg. diborane or Raney nickel.

With regard to process (e) the partial hydrolysis of the nitrile to give the amide may be carried out in conventional manner under acidic or basic conditions. Methods for carrying out the transformation are extensively described in the literature - see for example the textbook Buehler and Pearson, Survey of Organic Syntheses, Wiley Interscience, 1970 pages 903-904, and references cited therein. The preferred method is to carry out the hydrolysis using aqueous sodium hydroxide in the presence of hydrogen peroxide and a phase transfer catalyst.

Process (f) may also be carried out in conventional manner using either acid or basic conditions as described in the literature. By way of illustration reference is directed to the textbook Buehler and Pearson, ibid at pages 751-754.

Process (g) may also be carried out in conventional manner using ammonia or amine preferably in alcoholic solution. Again by way of illustration reference is drawn to the textbook Buehler and Pearson, ibid pps 899-902. Where an acid halide is used as starting material it is preferred to employ an acid salt thereof in order to minimise reaction of the starting material with itself.

The hydrolysis process (h) may be carried out under acidic or basic conditions in conventional manner eg. refluxing in 10% alkali metal hydroxide followed by acidification.

Processes (i) and (j) may be conveniently carried out using conventional chemical oxidising agents, eg. potassium dichromate, potassium permanganate and oxygen/platinum catalyst. These and other methods are given in standard textbooks, see for example Buehler and Pearson, ibid, pps 545-549 and 760-764. Where process (j) is used to provide an aldehyde, overoxidation to the acid can be avoided by selective oxidation methods as described on pages 546-8 of Buechler and Pearson.

With regard to process (k) oxidation of the compound of formula VII wherein $D^3$ is $COCH_3$ may be effected by treatment with halogen and alkali (ie. the well known haloform reaction) eg. by adding a solution of sodium hypochlorite or hypobromite to the ketone in alcoholic solvent. Oxidation of the compound of the formula VII wherein $D^3$ is $CH=CH_2$ or-$C\equiv CH$ may be effected by using potassium permanganate, potassium iodate or ozone.

Hydrolysis process (l) is conveniently carried out under acidic conditions eg. refluxing in dilute sulphuric acid.

Esterification process (m) may be effected by conventional means involving reaction with an alcohol ROH, see for example Buehler and Pearson, ibid, pps 802-809. Conveniently the reaction of the acid may be carried out with the alcohol as solvent and in the presence of mineral acid (eg. HCl). The acid halide is conveniently in the form of an acid salt prior to reaction with the alcohol and the reaction is preferably carried out in base, eg. dimethylaniline or pyridine.

Process (n) may be carried out in the usual manner using a halogenating agent, eg. phosphorus trichloride or tribromide, phosphorous pentachloride or thionyl chloride.

Reduction process (o) may be carried out using a reducing agent which reduces acids to alcohols eg. a hydride such as borane.

The starting materials of formula II used in process (a) are known compounds or can be prepared by analogous methods eg. by reducing an amide of formula Ar-A-NHCO-$E^1$ where $E^1$ has one $CH_2$ group less than E.

Compounds of formula V can be prepared by acylating a corresponding compound of formula Ar-A-NH-B-$D^1$ using an acid chloride of formula $R^4$ COCl. Compounds of formula Ar-A-NH-B-$D^3$ can themselves be prepared by alkylating amines of formula $NH_2$-B-$D^3$ using a halide of formula Ar-A-hal.

Compounds of formula VI can be prepared by acylating amines of formula Ar-A-NH-E using an acid chloride of formula ClCO.$B^1$-$D^2$ wherein the variables have the values defined in connection with process (d).

Compounds of formula VII may be prepared by a process analogous to process (a) above using a compound of formula II and compound of formula hal-B-$D^3$.

Compounds of formula VIII may be prepared by reacting a compound of formula IX:

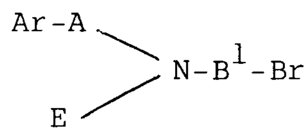

(IX)

with a compound of formula $T^1CH_2T^2$, eg a malonic acid ester in the presence of a base, eg. sodium hydride or sodium ethoxide. The compound of formula IX may itself be prepared by reaction of phosphorus tribromide and a compound of formula Ib wherein D is $-CH_2OH$.

Starting materials for the processes described herein are known compounds or can be prepared by analogous methods for known compounds.

In any of the aforementioned reactions compounds of formula Ia may be isolated in free base form or as acid addition salts as desired. Examples of such salts include salts with pharmaceutically acceptable acids such as hydrochloric, hydrobromic, hyroiodic, sulphuric, phosphoric, nitric, acetic, citric, tartaric, fumaric, succinic, malonic, formic, maleic acid or organosulphonic acids such as methane sulphonic or tosylic acid.

When acidic substituents are present it is also possible to form salts with bases eg. alkali metal (such as sodium) or ammonium salts eg. trimethylammonium. Such salts of the compounds of formula Ia are included within the scope of this invention.

This invention also provides pharmaceutical compositions comprising a compound of formula Ia or a pharmaceutically acceptable salt thereof as defined above with the provisos

(i) when E is $C_1$-$C_6$ alkyl and A is alkylene having 2 carbon atoms linking Ar to N and $D^1$ is $CONR^1R^2$ then Ar is other than phenyl or substituted phenyl,

(ii) when E is $C_1$-$C_6$ alkyl and $D^1$ is COOH the Ar is other than 3-pyridyl, and

(iii) when A is methylene and E is $C_1$-$C_6$ alkyl, benzyl or phenethyl and $D^1$ is $CONR^1R^2$ then Ar is other than substituted phenyl.

and a pharmaceutically acceptable carrier.

For the pharmaceutical compositions any suitable carrier known in the art can be used. In such a composition, the carrier may be a solid, liquid or mixture of a solid and a liquid. Solid form compositions include powders, tablets and capsules. A solid carrier can be one or more substances which may also act as flavouring agents, lubricants, solubilisers, suspending agents, binders, or tablet disintegrating agents; it can also be encapsulating material. In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from 5 to 99, preferably 10-80% of the active ingredient. Suitable solid carriers are magnesium stearate; talc, sugar; lactose, pectin, dextrin, starch; gelatin, tragacanth, methyl cellulose, sodium carboxymethyl cellulose; a low melting wax and cocoa butter. The term "composition" is intended to include the formulation of an active ingredient with encapsulating material as carrier, to give a capsule in which the active ingredient (with or without other carrier) is surrounded by carriers, which is thus in association with it. Similarly cachets are included.

Sterile liquid form compositions include sterile solutions, suspensions, emulsions, syrups, and elixirs.

The active ingredient can be dissolved or suspended in a pharmaceutically acceptable carrier, such a sterile water, sterile organic solvent or a mixture of both. The active ingredients can often be dissolved in a suitable organic solvent, for instance aqueous propylene glycol containing from 10 to 75% of the glycol by weight is generally suitable. Other compositions can be made by dispersing the finely-divided active ingredient in aqueous starch or sodium carboxymethyl cellulose solution, or in a suitable oil, for instance arachis oil. The composition may be administered orally, nasally, rectally or parenterally.

Preferably the pharmaceutical composition is in unit dosage form, the composition is sub-divided in unit doses containing appropriate quantities of the active ingredient; the unit dosage form can be a packaged composition, the package containing specific quantities of compositions, for example packeted powders or vials or ampoules. The unit dosage form can be a capsule, cachet or tablet itself, or it can be the appropriate number of any of these in packaged form. The quantity of active ingredient in a unit dose of composition may be varied or adjusted from 1 to 500 mg or more, eg. 25 mg to 250 mg, according to the particular need and the activity of the active ingredient. The invention also includes the compounds in the absence of carrier where the compounds are in unit dosage form. Based on the results from animal studies the dosage range for the treatment of humans using a compound of formula I will be in the range from about 1 mg to 2 g per day depending on the activity of the compound.

The following Examples illustrate the invention and methods for preparing compounds of the invention.

## EXAMPLE 1

### 4-[N,N-Bis-(4-Chlorobenzyl)amino]butyronitrile

Bis-(4-chlorobenzyl)amine hydrochloride [prepared by reducing N-(4-chlorobenzyl)-4-chlorobenzamide with lithium aluminium hydride in tetrahydrofuran] was basified using sodium carbonate in methylene dichloride solvent to give after rotary evaporation 3.16g of the free base as an oil. This was then mixed with 4-bromobutyronitrile (1.19ml), diisopropylethylamine (2.1ml) and 25 ml of dry dimethylformamide and the mixture stirred at 80°C under nitrogen for 24 hours. On cooling water (100cm$^3$) was added and then the mixture was extracted with 1 x 50ml and 2 x 25ml of methylene dichloride. The combined extracts were washed with water, dried ($MgSO_4$), filtered and evaporated to give an oil. This was further purified by column chromatography (silica column eluted with toluene) to give 2.83g of the title product as an oil. 2.8g of the title compound was dissolved in 10 ml hot ethanol, acidified with 0.76g (1 equiv,) of oxalic acid, diluted with ether and cooled to give, after filtration, the 1:1-ethanedioate salt (1.78g) as colourless crystals, mp 124-6° (dec).

| Analysis | | | |
|---|---|---|---|
| $C_{18}H_{18}Cl_2N_2 . C_2H_2O_4$ requires<br>Found: | C,56.8;<br>C, 57.O; | H,4.8;<br>H, 4.9; | N,6.6<br>N,6.2%. |

## EXAMPLE 2

### 4-[N,N-Bis-(4-Chlorobenzyl)amino]butyric acid

1.36g of 4-[N,N-bis-(4-chlorobenzyl)amino]butyronitrile (as produced in Example 1), water (25ml) and concentrated hydrochloric acid (10ml) were stirred and heated to reflux for 5½ hours under a nitrogen blanket. After cooling, an oil was obtained which crystallised on scratching. The crystals were filtered, washed with dilute hydrochloric acid, then with diethyl ether and recrystallised from isopropanol ether. Filtration and drying at 60°/1mm Hg gave the title compound as the hydrochloride salt (0.32g) m.p. 206-208°C(dec)

| Analysis | | | |
|---|---|---|---|
| $C_{18}H_{19}NO_2$. HCl requires<br>Found: | C,55.6;<br>C,55.4; | H,5.2;<br>H,5.2; | N,3.6<br>N,3.7%. |

EXAMPLE 3

4-[N,N-Bis-(4-chlorobenzyl)amino]butyramide

A solution of 4-[N,N-Bis-(4-chlorobenzyl)amino]butyronitrile (2.0g) prepared according to Example 1 in dichloromethane (10ml) was cooled in ice, then treated with the phase-transfer catalyst $^nBu_4NHSO_4$ (0.5g), 20% aq NaOH (3.2ml) and 30% $H_2O_2$(4.0ml). The mixture was stirred vigorously at 0° for about half an hour, then at room temperature for 23 hours. After dilution with dichloromethane the layers were separated, and the organic phase washed with water and with dil. aq. $NaHSO_3$, then dried ($MgSO_4$). Filtration and evaporation gave a syrup (2.26g) which was flash-chromatographed on neutral alumina eluted with neat toluene (to remove starting material (0.3g)) and then with 10% EtOH-toluene to recover the product (1.62g). Repeat chromatography on silica eluted with 2-5% EtOH-toluene gave the title compound (1.55g; 73.6%) as a syrup which set solid on standing. The resulting crystals had m.p. 66-76°.

| Analysis | | | |
|---|---|---|---|
| $C_{18}H_{20}N_2OCl_2$ requires:<br>Found: | C, 61.6;<br>C, 61.5; | H, 6.0;<br>H, 5.8; | N, 8.0<br>N, 8.0%. |

EXAMPLE 4

4-(N,N-Dibenzylamino)butyramide

a) By a method analogous to Example 1 dibenzylamine was reacted with 4-bromobutyronitrile to give 4-(N,N-dibenzylamino)butyronitrile, mp. 45-46°C.

b) Hydrogen peroxide (30% w/v; 5.6ml), tetra-n-butylammonium hydrogensulphate (1.70g) and 5N aqueous sodium hydroxide (4ml) were added to a stirred solution of 4-(N,N-dibenzylamino)butyronitrile (2.64g) in dichloromethane (10ml) with water cooling. The mixture was stirred vigorously at room temperature for 17 hours and dichloromethane (100ml) was added. The layers were separated and the organic phase was washed with saturated aqueous sodium chloride (10ml), dried ($Na_2SO_4$) and concentrated in vacuo to give a viscous oil (4.12g). The product was chromatographed on silica with eluant 20% ethyl acetate/toluene ----> ethyl acetate and recrystallised from cyclohexane to give the title compound (1.86g, 66%), m.p. 76° - 77°C.

| Analysis | | | |
|---|---|---|---|
| $C_{18}H_{22}N_2O$ requires:<br>Found: | C, 76.55;<br>C, 76.45; | H, 7.85,<br>H, 7.95; | N, 9.9<br>N, 9.8% |

EXAMPLE 5

4-(N,N-Dibenzylamino)butyric acid

A solution of 4-(N,N-dibenzylamino)butyronitrile (2.64g) in concentrated hydrochloric acid (50ml) was refluxed for 3 hours. The solution was concentrated in vacuo to give a solid, which was recrystallised from acetic acid and water to give the title compound as the monohydrochloride monoacetic acid salt (1.84g), m.p. 138°-139°C.

| Analysis | | | |
|---|---|---|---|
| $C_{18}H_{21}NO_2.HCl.\ C_2H_4O_2$ requires:<br>Found: | C,63.25;<br>C, 63.6; | H,6.9;<br>H, 7.2; | N,3.7<br>N, 3.9% |

EXAMPLE 6

4-[N,N-Bis-(4-methylbenzyl)amino]butyronitrile

A solution of 4-bromobutyronitrile (14.80g) in dry dimethylformamide (100ml) was added dropwise over 15 minutes to a stirred solution of bis-(4-methylbenzyl)amine (22.53g) and N,N-diisopropylethylamine (35ml 26g) in dry dimethylformamide (100ml). The solution was heated (oil bath 115°C) under nitrogen for 4 hours. The solution was poured onto a mixture of water (400ml) and saturated aqueous sodium chloride (400ml) and the mixture was extracted with ether (2 x 200ml). The extracts were dried ($Na_2SO_4$) and concentrated in vacuo to give an orange oil (39.55g). The product was chromatographed on silica with eluant diisopropyl ether, and dried at 100°C at 0.1mmHg for 4 hours to give 4-[N,N-bis-(4-methylbenzyl)-amino]butyronitrile (26g, 89%), which was used in Example 7 without further characterisation.

EXAMPLE 7

4-[N,N-Bis-(4-methylbenzyl)amino]butyramide

Hydrogen peroxide (30% w/v; 5.67ml), tetra-n-butylammonium hydrogensulpnate (1.70g) and 5N aqueous sodium hydroxide (4ml) were added successively to a stirred solution of 4-(N,N-bis-(4-methylbenzyl)-amino]butyronitrile (2.93g) in dichloromethane (10ml), with water cooling.

The mixture was stirred vigorously at room temperature for 16 hours then dichloromethane (100ml) was added. The layers were separated and the organic phase was washed with saturated aqueous sodium chloride (1x10ml), dried ($Na_2SO_4$) and concentrated in vacuo to give a viscous oil (4.61g). The product was chromatographed on silica with eluant 20% ethyl acetate/toluene ----> ethyl acetate and triturated with cyclohexane to give the title compound (1.77g), m.p. 85°-86°C

Analysis

Found: C,77.25; H, 8.35; N, 9.25; $C_{20}H_{26}N_2O$ requires C, 77.4; H, 8.45; N, 9.0%.

EXAMPLE 8

4-[N,N-Bis-(4-methylbenzyl)amino]butyric acid

4-[N,N-Bis-(4-methylbenzyl)amino]butyronitrile (2.93g) was suspended in concentrated hydrochloric acid (50ml) and the mixture was refluxed (oil bath 130°C) for 4 hours. The solution was concentrated in vacuo to give a foam (4.17g). The product was triturated with hot water and crystallised in ether at -78°C to give the title compound as the hydrochloride, three quarters hydrate (2.82g), m.p. 148°-151°C

| Analysis | | | |
|---|---|---|---|
| $C_{20}H_{25}NO_2.HCl.0.75H_2O$ requires:<br>Found: | C,66.45;<br>C, 66.2; | H,7.65;<br>H, 7.2; | N,3.9.<br>N, 3.95%. |

EXAMPLE 9

4-[N,N-Bis-(3,4-Dichlorobenzyl)amino]butyronitrile

Using a procedure analogous to Example 6, Bis-(3,4-dichlorobenzyl)amine (2.71g, 8.09mM) was reacted with 4-bromobutyronitrile (0.81ml) to give the title compound as an oil 1.97g.
Ir (film) CN 2230cm$^{-1}$.

EXAMPLE 10

4-[N,N-Bis-(3,4-dichlorobenzyl)amino]butyric acid

A mixture of 4-[N,N-bis-(3,4-dichlorobenzyl)amino]butyronitrile (1.95g), NaOH (2.0g) and ethanol (25ml) was stirred and heated to reflux for 6 hours under a nitrogen blanket. After cooling, the solvent was evaporated in vacuo to give a paste which was taken up in water (30ml), acidified with conc. HCl, stirred well and cooled in ice. The oil which had precipitated crystallised slowly. The crystals were filtered off, washed well with water, and recrystallised from dimethylformamide, diluted with a large volume of ether. The precipitated oil again crystallised slowly. The crystals were triturated with hot isopropanol, diluted with ether, collected by filtration and dried at 50°/lmm to give the title compound as the hydrochloride salt. (1.20g), m.p. 227-231° (decomp).

| Analysis | | | |
|---|---|---|---|
| $C_{18}H_{17}NO_2Cl_4$. HCl requires:<br>Found: | C, 47.2;<br>C, 47.2; | H, 4.0;<br>H, 4.0; | N, 3.1<br>N, 3.2%. |

EXAMPLE 11

4-[N,N-Bis-(4-methoxybenzyl)amino]butyronitrile

A solution of 4-bromobutyronitrile (14.80g) in dry dimethylformamide (100ml) was added dropwise over 15 minutes to a stirred solution of bis-(4-methoxybenzyl)amine (25.73g) and N,N-diisopropylethylamine (35ml, 26g) in dry dimethylformamide (100ml). The yellow solution was heated under nitrogen at 110°C for 4 hours and was allowed to cool. The solution was poured onto a mixture of water (400ml) and saturated aqueous sodium chloride (400ml) and then extracted with ether (2x200ml). The extracts were dried ($Na_2SO_4$) and concentrated in vacuo to give an oil (35.93g). The product was chromatographed on silica with eluant diisopropyl ether, and was triturated with cyclohexane to give the title compound (26.00g), m.p. 56°-58°C.

| Analysis | | | |
|---|---|---|---|
| $C_{20}H_{24}N_2O_2$ requires:<br>Found: | C, 74.05;<br>C, 73.85, | H, 7.45;<br>H 7.4; | N, 8.65.<br>N, 8.85%. |

EXAMPLE 12

4-[N,N-Bis-(4-Methoxybenzyl)amino]butyramide

Hydrogen peroxide (30% w/v; 5.67ml), tetra-n-butylammonium hydrogensulphate (1.70g) and 5N aqueous sodium hydroxide (4ml) were added successively to a stirred solution of 4-[N,N-bis-(4-methoxybenzyl)-amino]butyronitrile (3.24g) in dichloromethane (10ml), with water cooling. The mixture was stirred vigorously at room temperature for 18 hours and dichloromethane (100ml) was added. The layers were separated and the organic phase was washed with saturated aqueous sodium chloride (1x10ml), dried ($Na_2SO_4$) and concentrated in vacuo to give an oil (4.71g). The product was chromatographed on silica with eluant 20% ethyl acetate/toluene ---->ethyl acetate, and crystallised from diisopropyl ether at room temperature to give the title compound (2.21g), m.p. 53-54°C.

| Analysis: | | | |
|---|---|---|---|
| $C_{20}H_{26}N_2O_3$ requires:<br>Found: | C, 70.15;<br>C, 70.0; | H, 7:65;<br>H, 7.75; | N, 8.2<br>N, 8.15%. |

EXAMPLE 13

4-[N,N-Bis-(2-(4-chlorophenyl)ethyl)amino]butyronitrile

In a manner analogous to Example 6, 5.08g of bis-[2-(4-chlorophenyl)ethyl]amine was reacted with 1.73ml 4-bromobutyronitrile to give the title compound (5.83g).

EXAMPLE 14

4-[N,N-Bis-(2-(4-chlorophenyl)ethyl)amino]butyric acid

A solution of the 4-[N,N-bis-(2-(4-chlorophenyl)ethyl)amino]butyronitrile (5.8g) and NaOH (5.7g) in ethanol (75ml) was stirred and heated to reflux for 6 hours and, after cooling, the solvent was evaporated and the residue was taken up in water, acidified with conc. HCl and the turbid mixture maintained at 5° C overnight, during which time an oil precipitated and solidified. The solid was collected by filtration and washed with water. It was then boiled for 1 hour with conc. HBr, cooled, kept at 5° until resolidification had occurred, and the solid was collected by filtration. Recrystallisation was twice effected by dissolution of the solid in hot isopropanol containing a few drops of water, followed by dilution with ether, to give crystals of the title compound as the hydrobromide salt, (4.03g), m.p. 150-2° (dec.; "sweating" occurs above 147°).

| Analysis: | | | |
|---|---|---|---|
| $C_{20}H_{23}NO_2Cl_2$.HBr requires: | C,52.1; | H,5.2; | N,3.0 |
| Found: | C,52.2; | H,5.4; | N,3.25% |

EXAMPLE 15

Ethyl 4-[N,N-bis-(4-chlorobenzyl)amino]butanoate

a) An ice-cooled, stirred suspension of 4-[N,N-bis-(4-chlorobenzyl)amino]butyric acid, hydrochloride salt (2.05g) (prepared according to Example 2) in absolute ethanol (100ml) was treated with HCl gas for ½ hour. The solution was warmed to room temperature and allowed to stand overnight. The solvent was evaporated in vacuo, and the oily residue taken up in aqueous $NaHCO_3$ and extracted with dichloromethane (2 x 25ml). The combined extracts were washed with water and dried ($MgSO_4$). Filtration and evaporation gave an oil (1.86g) which was freed from residual acid by passage through a silica column, eluted with ethyl acetate. Evaporation of the eluate gave an oil (1.32g) which was dissolved in ethanol (5ml) and treated with oxalic acid (0.30g, 1 equiv.). Evaporation of the solvent and treatment of the residue with hot ethyl acetate caused crystallisation to occur. The crystals were collected by filtration and recrystallised from ethanol-ethyl acetate to give the title compound as the 1:1 ethanedioate salt (0.53g) as colourless crystals, m.p. 123.5-125°.

| Analysis | | | |
|---|---|---|---|
| $C_{20}H_{23}NO_2Cl_2.C_2H_2O_4$ requires: | C,56.2; | H,5.4; | N,3.0 |
| Found: | C,56.3; | H,5.5; | N,2.7% |

b) The product of step (a) is converted to 4-[N,N-bis-(4-chlorobenzyl)amino] butyramide by treatment with ammmonium hydroxide.

EXAMPLE 16

4-[N,N-Bis-(4-Chlorobenzyl)amino]butanol

A stirred suspension of 4-[N,N-bis-(4-chlorobenzyl)amino]butyric acid, hydrochloride (prepared according to Example 2) (13.44g) in dry tetrahydrofuran (250ml) was stirred and heated to reflux as borane-methyl sulphide (10 molar; 17ml; 5 equiv.) was added dropwise. The mixture was stirred and heated to reflux under

a nitrogen blanket for 17 hours. After cooling, the mixture was decomposed by the dropwise addition of 10% aq $H_2SO_4$. The organic solvent was evaporated in vacuo and the aqueous residue was diluted with 10% aq $H_2SO_4$ (120ml) and heated to reflux for 3 hours.

The cooled solution was poured onto aqueous $Na_2CO_3$ and brine and extracted with dichloromethane (2 x 100ml). The combined extracts, which showed a tendency to emulsify in the presence of water, were washed with brine and dried ($MgSO_4$). Filtration and evaporation gave a viscous syrup (11.38g) which was purified by passage through silica gel, eluted with neat toluene and then with 2-5% ethanol/toluene to give an oil (9.40g).

A solution of the oil (7.50g) in hot ethanol (25ml) was treated with 2.58g (1 equiv) of fumaric acid. The crystals which separated overnight were triturated with boiling iso-propanol, cooled and refiltered, to give crystals of the title compound as the 1½ fumaric acid salt (3.70g) mp. 141-143° (dec).

| Analysis | | | |
|---|---|---|---|
| $C_{18}H_{21}NOCl_2.1\frac{1}{2}\ C_4H_4O_4$ requires:<br>Found: | C,56.3;<br>C, 56.0; | H,5.3;<br>H,5.4; | N,2.7<br>N, 2.6% |

EXAMPLE 17

(a)

4-[(N-(1,1-diphenyl)methyl-N-benzyl)amino]butyronitrile

A solution of 4-bromobutyronitrile (7.40g) in dry dimethylformamide (50ml) was added dropwise over 5 minutes to a stirred solution of N-benzyl-1,1-diphenylmethylamine (13.67g)(prepared by reductive amination of benzophenone and benzylamine using sodium cyanoborohydride) and N,N-diisopropylethylamine (18ml, 13g) in dry dimethylformamide (50ml). The solution was heated at 120°C under nitrogen for 42 hours and was poured onto a mixture of saturated aqueous sodium chloride (200ml) and water (200ml). The mixture was extracted with ether (2 x 100ml) and the extracts were dried ($Na_2SO_4$) and concentrated in vacuo to give an oil (21.27g). The product was chromatographed on silica with eluant dichloromethane. Fractions containing the desired material were evaporated to dryness at reduced pressure and dried at 110°C at 0.1mmHg for 4 hours to give 4-[(N-(1,1-diphenyl)methyl-N-benzyl)amino]butyronitrile as an oil (14.61g).

(b)

4-[(N-(1,1-Diphenyl)methyl-N-benzyl)amino]butyric acid

A solution of the product of step (a) (3.40g) in absolute ethanol (100ml) was treated with sodium hydroxide pellets (20.0g). The mixture was refluxed for 4 hours and was concentrated in vacuo to give a solid. Water (200ml) and ice (200ml) were added and the mixture was acidified with concentrated hydrochloric acid (100ml) at 0°C. The mixture was concentrated in vacuo and the residue was triturated with isopropanol. The mixture was filtered and the solution was reconcentrated to give a foam (4.51g). The product was dissolved in hot water and the mixture was filtered through kieselguhr. The filtrate was concentrated in vacuo to give a solid (2.21g), which was triturated with ethyl acetate, to give the title compound as the hydrochloride three quarters hydrate (1.90g.), m.p. 166°-173°C.

| Analysis | | | |
|---|---|---|---|
| $C_{24}H_{25}NO_2.HCl.O.75H_2O$ requires<br>Found: | C, 70.4;<br>C,70.65; | H, 6.75;<br>H, 6.8; | N, 3.4.<br>N, 3.05%. |

EXAMPLE 18

a) 4-[N,N-Bis-(3-bromobenzyl)amino]butyronitrile

A mixture of N,N-bis-(3-bromobenzyl)amine (13.47g; 0.038mol), 4-bromobutyronitrile (3.77ml; 0.038mol), diisopropylethylamine (10ml; 0.057mol), potassium iodide (1g) and dimethylformamide (30ml) was stirred under a nitrogen blanket at 80-90° for 22 hours. After cooling, the solvent was evaporated and the residue was taken up in aq. $Na_2CO_3$ and extracted with dichloromethane. The combined extracts were washed with aq. $Na_2CO_3$ and dried ($MgSO_4$). Filtration and evaporation gave an oil (18.49g), which was chromatographed on silica eluted with 1% v/v ethanol-toluene. Evaporation of fractions containing product gave the title compound (12.36g; 77.2%) as an oil. A solution of the product (0.84g; 2mmol) in hot ethanol (5ml) was treated with oxalic acid (0.18g; 2mmol). Crystallisation occurred overnight and the crystals were collected by filtration and recrystallised from ethanol-ether to give the 1:1 ethanedioate salt (0.22g), mp. 120-122°C.

| Analysis | | | |
|---|---|---|---|
| $C_{18}H_{18}Br_2N_2.(COOH)_2$ requires:<br>Found: | C, 46.9;<br>C,47.1; | H, 3.9;<br>H,4.1; | N, 5.5.<br>N, 5.3%. |

b) 4-[N,N-Bis-(3-bromobenzyl)amino]butyric acid

A solution of 4-[N,N-bis-(3-bromobenzyl)amino]butyronitrile (12g) and ethanol (120ml) was stirred and heated to reflux for 17.5 hours under nitrogen. After cooling, the solvent was evaporated in vacuo. The residue was taken up in water (120ml) and strongly acidified with conc. HCl (36ml). The mixture was cooled in ice, causing an oil to precipitate. The oil crystallised after standing overnight and was filtered off and triturated with hot isopropanol. After cooling, the crystals were collected by filtration and washed with ether to give crystals of the title compound as the hydrochloride salt (10.87g), mp. 156-159°C.

| Analysis | | | |
|---|---|---|---|
| $C_{18}H_{19}Br_2NO_2$. HCl requires:<br>Found: | C,45.3,<br>C,45.0; | H,4.2;<br>H,4.5; | N,2.9<br>N, 2.5% |

EXAMPLE 19

a) 4-[N,N-Bis-(3-chlorobenzyl)amino]butyronitrile

A mixture of bis-(3-chlorobenzyl)amine (8.0g; 0.03 mol), potassium iodide (5.0g; 0.03 mol), diisopropylethylamine (7.0 ml; 0.04 mol), 4-bromobutyronitrile (3.0 ml; 0.03 mol) and dimethylformamide (25 ml) was stirred and heated to an oil-bath temperature of 80°C for 18 hours, under a nitrogen blanket. After cooling, the solvent was evaporated and the residue taken up in dilute aqueous $Na_2CO_3$ and extracted with dichloromethane (3x50ml). The combined extracts were washed with water and dried ($MgSO_4$). Filtration and evaporation gave an oil (11.0g) which was chromatographed on silica eluted with neat toluene and then with 2% v/v ethanol-toluene. The fractions containing product were collected and evaporated to give the title compound as an oil (8.59g).

A portion (1.1g) of the oil was converted to the 1:1 ethanedioate salt by addition of oxalic acid (one equivalent) in isopropanol. After evaporation of the solvent, the 1.1 ethanedioate salt of the title compound was obtained by crystallisation from ethyl acetate, m.p. 74-9° C.

| Analysis | | | |
|---|---|---|---|
| $C_{18}H_{18}Cl_2N_2$. $(COOH)_2$ requires:<br>Found: | C,56.7;<br>C,56.3; | H,4.8;<br>H,4.6; | N,6.6<br>N,6.7% |

b) 4-[N,N-Bis-(3-chlorobenzyl)amino]butyric acid

A solution of 4-[N,N-bis-(3-chlorobenzyl)amino]butyronitrile (8.59g; 0.026 mol), NaOH (10g; 0.25 mol) and ethanol (125 ml) was stirred and heated to reflux for 18 hours. After cooling the solvent was evaporated in vacuo and the solid residue taken up in water (75 ml). After further cooling in ice, conc. HCl was added until the mixture was strongly acid (ca. 25 ml required). The initially precipitated oil hardened to a solid, which was collected by filtration and recrystallised from dimethylformamide-ether and then from 10% v/v water-isopropanol followed by dilution with ether. After drying at 45°C/1mm the hydrochloride hemihydrate salt of the title compound was obtained (5.60g). m.p. 170-173° (dec.; softens above 166°).

| Analysis | | | |
|---|---|---|---|
| $C_{18}H_{19}Cl_2NO_2$. HCl. $\frac{1}{2}H_2O$ requires:<br>Found | C,54.4;<br>C, 54.4; | H,5.3;<br>H,5.2; | N,3.5<br>N,3.5%. |

EXAMPLE 20

4-[N,N-Bis-(2-thienylmethyl)amino]butyric acid

a) In a manner analogous to Example 19a, 4-[N,N-bis-(2-thienylmethyl)amino]butyronitrile was prepared by alkylation of bis-(2-thienylmethyl)amine using 4-bromobutyronitrile.

b) In a manner analogous to Example 2,4-[N,N-bis-(2-thienylmethyl)amino]butyronitrile (12g) was hydrolysed using concentrated hydrochloric acid (200 ml) to give the title compound as an oily-solid residue. Isopropanol (150ml) was added and the mixture cooled with stirring. Precipitated $NH_4Cl$ was removed by filtration and the filtrate was concentrated and filtered to remove further inorganic material. The filtrate was partitioned between aqueous NaOH and toluene. After standing overnight, the layers were separated, the aqueous phase was acidified to pH1, concentrated in vacuo, diluted with isopropanol, filtered to remove precipitated NaCl, and evaporated to dryness to give a solid.

The solid was chromatographed on silica eluted with 2% v/v ethanol-toluene, then with 10%- and 20%-v/v ethanol-toluene. The product-bearing fractions were evaporated, dissolved in isopropanol, filtered to remove a little solid material, concentrated in vacuo and diluted with ether. The initially-precipitated gum crystallised after standing for 72 hours. The solid was collected by filtration, purified by trituration with boiling isopropanol, cooled and refiltered to give crystals of the title compound, as the hydrochloride salt mp 158-160°.

| Analysis: | | | |
|---|---|---|---|
| $C_{14}H_{17}NO_2S_2$ HCl requires<br>Found | C, 50.7;<br>C,50.5; | H, 5.5;<br>H,5.6; | N, 4.2<br>N,4.1% |

EXAMPLE 21

4-[(N-(4-Methylphenylmethyl)-N-phenylmethyl)amino]butyric acid

a) In a manner analogous to Example 19a, 4-[(N-(4-methylphenylmethyl)-N-phenylmethyl)amino]-butyronitrile was prepared by alkylation of N-(4-methylphenylmethyl)benzenemethanamine (4.22g) using 4-bromobutyronitrile (2ml). Yield 4.45g.

b) In a manner analogous to Example 10 the nitrile from step (a) above was hydrolysed using NaOH in ethanol to give, after acidification with HBr, the title compound as the hydrobromide, quarterhydrate salt, mp 119-125°C.

| Analysis | | | |
|---|---|---|---|
| $C_{19}H_{23}NO_2$. HBr. $\frac{1}{4}H_2O$ requires:<br>Found: | C, 59.6;<br>C, 59.5; | H,6.5;<br>H, 6.75; | N, 3.7<br>N, 3.5%. |

EXAMPLE 22

4-(N,N-Dibenzylamino)-N'N'-dimethylbutyramide

A sample of 4-(N,N-dibenzylamino)butyric acid (8.51g, 0.03 moles) (prepared according to Example 5) was dissolved in dry acetonitrile (50ml) under nitrogen. 1,1'-Carbonyldiimidazole (4.85g) was dissolved in dry acetonitrile and added dropwise over 1 hour. The mixture was stirred under nitrogen for 96 hours. An excess of dimethylamine (10g. 0.22 moles) was added; and the mixture stirred for a further two hours. Evaporation gave a residue which was separated on a silica column with ethyl acetate as eluant. Ten 50 ml fractions were collected; the product appearing in fractions 5-8. Evaporation gave an oil which was dissolved in ether. Acidification with ethereal HCl to pH1 gave a precipitate which was recrystallised from ethyl acetate/isopropanol to give crystals of the title compound as the hydrochloride, quarterhydrate, mp. 166-171.

| Analysis | | | |
|---|---|---|---|
| $C_{20}$ $H_{26}$ $N_20$. HCl. $\frac{1}{4}H_20$ requires<br>Found | C, 68.5;<br>C, 68.7; | H,7.9;<br>H, 8.0; | N,8.0.<br>N, 8.0% |

EXAMPLE 23

4-[N,N-Bis-(1-Naphthalenemethyl)amino]butyric acid

a) 1-Naphthoyl chloride (5.0g, 0.026 moles) in $CH_2Cl_2$ (25ml) was added dropwise to a stirred solution of ice cooled 1-naphthalenemethylamine in $CH_2Cl_2$ (25ml). A white precipitate formed immediately. The mixture was stirred at room temperature for 3 hours then filtered. The precipitate was washed with water and hot ethanol, then dried to give N-(1-naphthalenylmethyl 1-naphthaleneacetamide (5.18g, 0.017 moles). The amide (5.18g) was dissolved in tetrahydrofuran (25ml) then added dropwise to $LiAlH_4$ (0.63g 0.016 moles) in tetrahydrofuran (25ml). The mixture was heated at reflux for 24 hours, then worked up by dropwise addition of water (0.6ml), 15% aq NaOH(0.6ml) and water (1.9ml). Filtration, drying ($MgSO_4$) and evaporation gave an oil (22.5g) which was crystallised from ice-cold ethanol to give N,N-bis-(1-naphthalenemethyl)amine (2.54g)

(b) The amine from step (a) (2.54g) was dissolved in DMF (20ml), then 4-bromobutyronitrile (0.76 ml, 0.0076 moles) and diisopropylethylamine (4.07ml 0.023 moles) in DMF (25 ml) added dropwise. Potassium iodide (1g) was added and the mixture heated at reflux for 24 hours. Evaporation gave a solid which was taken up into $Na_2CO_3$ (50ml), extracted into $CH_2Cl_2$ (3x25ml) dried ($MgSO_4$) and evaporated to give a solid. Separation on a silica column with 10% v/v ethyl acetate in toluene as eluant, followed by evaporation, gave 4-[N,N-bis(1-naphthalenemethyl)amino]butyronitrile (1.54g).

(c) The nitrile from step (b) (1.54g) was dissolved in isopropanol (25ml), then NaOH (1.0g, 0.024 moles) was added and the mixture heated at reflux for 24 hours. Evaporation gave a residue which was taken up into water and extracted with $CH_2Cl_2$ (3x30ml). Drying and evaporation gave a solid which was partially dissolved in ether, then precipitated with ethereal HCl. Filtration, trituration with HCl followed by ice-cold water gave the title compound as the hydrochloride hemihydrate, (1.62g) m.p. 187-191 °C.

| Analysis | | | |
|---|---|---|---|
| $C_{26}$ $H_{25}$ $NO_2$. HCl. $\frac{1}{2}H_2O$ requires:<br>Found: | C,72.8;<br>C, 72.7; | H,6.4;<br>H,6.6; | N,3.3.<br>N, 3.5%. |

EXAMPLE 24

4-[(N-(p-Chlorobenzyl)-N-methyl)amino]butyramide

A solution of 4-[(N-(p-chlorobenzyl)-N-methyl)amino]butyronitrile (0.95g; 4.27mmol) in dichloromethane (5ml) was cooled in ice as $^{n}Bu_4NHSO_4$ (0.3g) was added, followed quickly by 20% w/v aq NaOH (1.6ml) and 30% hydrogen peroxide (2.0ml). The mixture was stirred vigorously, briefly at 0°C and then at room temperature, for 2 hours. After dilution with more dichloromethane, the layers were separated, the organic phase was washed with water and dried ($MgSO_4$). Filtration and evaporation gave an oil (0.83g) which was chromatographed on silica eluted with toluene containing an increasing proportion of isopropanol (to 50% v-(v). The product was obtained as an oil (0.52g) which set solid on standing, to give the title compound (0.52g) m.p. 62-64°.

| Analysis | | | |
|---|---|---|---|
| $C_{12}H_{17}ClN_2O$ requires:<br>Found: | C, 59.9;<br>C, 60.0; | H, 7.1;<br>H, 7.1; | N, 11.6<br>N, 11.9% |

EXAMPLE 25

4-[N-Methyl-N-(4-chlorobenzyl)amino]butyric acid

A mixture of 4-[N-methyl-N-(4-chlorobenzyl)amino]butyronitrile (4.65g; 0.021 mol) and NaOH (5g; 0.125 mol) in ethanol (50ml) was stirred and heated to reflux under a nitrogen blanket for 21 hours. The solvent was evaporated to give a paste, which was dissolved in water (50ml) and strongly acidified with conc. HCl (ca. 12ml). The solvents were again evaporated, and the residual, semi-crystalline mass was boiled briefly with isopropanol, filtered, and the inorganic precipitate washed well with isopropanol. The filtrate was evaporated to give a syrup (7.47g) which was crystallised in several crops from isopropanol/ether. The combined crops were triturated with boiling isopropanol, cooled, filtered and dried at 50°/1mm to give the title compound as the hydrochloride salt (4.22g) m.p. 149-152°.

| Analysis | | | |
|---|---|---|---|
| $C_{12}H_{16}ClNO_2$. HCl requires<br>Found: | C, 51.8;<br>C, 51.5; | H, 6.2;<br>H, 6.2; | N, 5.0<br>N, 5.0% |

EXAMPLE 26

In a manner analogous to Examples 24 and 25, nitrates of formula X shown below were hydrolysed to the corresponding acids or partially hydrolysed to the amide as shown below:

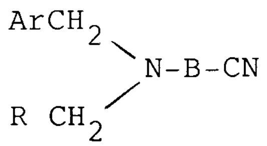

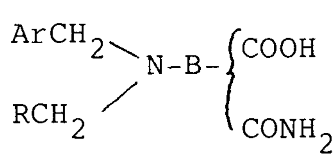

in which formulae Ar, R and B represent:

| Ar | R | B |
|---|---|---|
| 2-furanyl | 2-furanyl | $-(CH_2)_3-$ |
| 2-furanyl | hydrogen | $-(CH_2)_3-$ |
| 3-pyridyl | 3-pyridyl | $-(CH_2)_3-$ |
| 3-quinolyl | 3-quinolyl | $-(CH_2)_3-$ |
| 2-thiazolyl | 2-thiazolyl | $-(CH_2)_3-$ |

## Claims

**Claims for the following Contracting States : AT, BE, CH, DE, FR, IT, LI, LU, NL, SE**

**1.** Use of a compound which acts selectively as an agonist of gamma amino butyric acid (GABA) at GABA autoreceptors in the preparation of a medicament for the treatment of depression or senile dementia or other forms of memory impairment with the proviso that the compound is not fengabine or progabide.

**2.** A use according to Claim 1 in which the selectivity for the GABA autoreceptor relative to the $GABA_A$ receptor is greater than or equal to 100.

**3.** A use according to Claim 1 or Claim 2 wherein the GABA autoreceptor agonist is a trisubstituted amine in which one substituent is a 3- or 4- carbon alkyl group carrying a carboxylic acid or amide functional group.

**4.** A use according to Claims 1 to 3 wherein the GABA autoreceptor agonist has the formula:

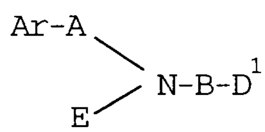

(Ia)

wherein E is $C_1$-$C_6$ alkyl or $Ar^1$-$A^1$;
Ar and $Ar^1$ are the same or different optionally substituted aryl groups;
A and $A^1$ are the same or different alkylene groups having one or two carbon atoms linking Ar or $Ar^1$ to N, each optionally substituted by $C_1$-$C_6$ alkyl or optionally substituted aryl;
B is an alkylene group of 3 carbon atoms which may be substituted by $C_1$-$C_6$ alkyl;
$D^1$ represents COOH or $CONR^1R^2$ wherein $R^1$ and $R^2$ are independently hydrogen, $C_1$-$C_6$ alkyl or aralkyl of 7 to 12 carbon atoms or a pharmaceutically acceptable salt thereof.

**5.** A use according to Claim 4 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**6.** A use according to Claim 5 wherein Ar and $Ar^1$ when present are selected from phenyl, naphthyl, pyridinyl, furanyl, thienyl, quinolinyl and benzofuranyl which groups may be optionally substituted.

**7.** A use according to Claim 6 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**8.** A use according to any one of Claims 4 to 7 wherein A and $A^1$ when present are independently selected from $-CHR^3$-where $R^3$ is hydrogen, $C_1$-$C_6$ alkyl or optionally substituted aryl as defined for Ar.

**9.** A use according to Claim 4 in which the compound of formula Ia is selected from one of the following:
4-[N,N-bis-(4-chlorobenzyl)amino]butyric acid,
4-[N,N-bis-(4-chlorobenzyl)amino]butyramide,
4-[N,N-bis-(4-methylbenzyl)amino]butyramide,

4-[N,N-bis-(3,4-dichlorobenzyl)amino]butyric acid,
4-[N,N-bis-(4-methoxybenzyl)amino]butyramide,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid,
4-[N-methyl-N-(p-chlorobenzyl)amino]butanoic acid,
4-(N,N-dibenzylamino)butyric acid,
4-[(N-(4-methylphenylmethyl)-N-phenylmethyl)amino]butanoic acid,
4-[(N-(1,1-diphenyl)methyl-N-benzyl)amino]butyric acid,
4-[(N-(p-chlorobenzyl)-N-methyl)amino]butyramide, or
4-(N,N-dibenzylamino)-N',N'-dimethylbutanamide or a pharmaceutically acceptable salt thereof.

**10.** A pharmaceutical composition comprising a compound of formula Ia as shown and defined in Claim 4 or a pharmaceutically acceptable salt thereof possessing selective GABA autoreceptor agonist activity and a pharmaceutically acceptable carrier with the provisos

(i) when E is $C_1$-$C_6$ alkyl and A is alkylene having 2 carbon atoms linking Ar to N and $D^1$ is $CONR^1R^2$ then Ar is other than phenyl or substituted phenyl,

(ii) when E is $C_1$-$C_6$ alkyl and $D^1$ is COOH the Ar is other than 3-pyridyl, and

(iii) when A is methylene and E is $C_1$-$C_6$ alkyl, benzyl or phenethyl and $D^1$ is $CONR^1R^2$ then Ar is other than substituted phenyl.

**11.** A composition according to Claim 10 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**12.** A composition according to Claim 10 wherein Ar and $Ar^1$ when present are selected from phenyl, naphthyl, pyridinyl, furanyl, thienyl, quinolinyl and benzofuranyl which groups may be optionally substituted.

**13.** A composition according to Claim 12 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**14.** A composition according to Claim 10 in which the compound of formula Ia is selected from one of the following:
4-[N,N-bis-(4-chlorobenzyl)amino]butyric acid,
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyric acid,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid, or
4-[N-methyl-N-(p-chlorobenzyl)amino]butanoic acid, or a pharmaceutically acceptable salt thereof.

**15.** A compound of formula Ia as shown and defined in Claim 4 or a pharmaceutically acceptable salt thereof possessing selective GABA autoreceptor agonist activity providing that

(i) neither Ar-A or E is unsubstituted benzyl,

(ii) when E is $C_1$-$C_6$ alkyl, Ar is not phenyl, substituted phenyl or a 5 or 6 membered heteroaromatic group,

and

(iii) when E is phenethyl, Ar is not substituted benzyl.

**16.** A compound according to Claim 15 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**17.** A compound according to Claim 16 wherein Ar and $Ar^1$ when present are selected from phenyl, naphthyl, pyridinyl, furanyl, thienyl, quinolinyl and benzofuranyl which groups may be optionally substituted.

**18.** A compound to Claim 17 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**19.** A compound according to any one of Claims 15 to 18 wherein A and $A^1$ when present are independently selected from -$CHR^3$-where $R^3$ is hydrogen, $C_1$-$C_6$ alkyl or optionally substituted aryl as defined for Ar.

**20.** A compound according to Claim 15 in which the compound of formula Ia is selected from one of the following:
4-[N,N-bis-(4-chlorobenzyl)amino]butyric acid,
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyric acid,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid, or
4-[N-methyl-N-(p-chlorobenzy)amino]butanoic acid, or a pharmaceutically acceptable salt thereof.

**21.** An intermediate for a compound of formula Ia as claimed in Claim 15, said intermediate having the formula (Ib):

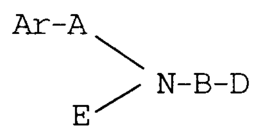

(Ib)

or a salt thereof wherein E, B, Ar, $Ar^1$, A and $A^1$ are as defined in Claim 15 and D represents CN, COhal, $CH_2OH$, CHO or an ester function COOR where R is an organic radical providing that
(i) when D is COOR then E is other than $C_1$-$C_6$ alkyl; and
(ii) when D is COOR then Ar-A and $Ar^1$-$A^1$ are not both p-nitrobenzyl.

**22.** A compound according to Claim 21 which is one of the following
4-[N,N-bis-(4-chlorobenzyl)amino]butyronitrile,
4-[N,N-bis-(4-methylbenzyl)amino]butyronitrile,
4-[N,N-bis-(3,4-dichlorobenzyl)amino]butyronitrile,
4-[N,N-bis-(4-methoxybenzyl)amino]butyronitrile,
4-[N,N-bis-(2-(4-chlorophenyl)ethyl)amino]butyronitrile,
Ethyl 4-[N,N-bis-(4-chlorobenzyl)amino]butanoate,
4-[N,N-bis-(4-chlorobenzyl)amino]butanol,
4-[N,N-bis-(3-bromobenzyl)amino]butyronitrile,
4-[N,N-bis-(3-chlorobenzyl)amino]butyronitrile,
4-[N,N-bis-(2-thienylmethyl)amino]butyronitrile,or
4-[N,N-bis-(1-naphthalenemethyl)amino]butyronitrile or

a salt thereof.

**23.** A process for preparing a compound of formula Ia or Ib as defined in Claim 15 or Claim 21 which comprises one of the following

a) reacting a compound of formula II

Ar-A-NH-E (II)

wherein Ar, E and A are as defined above with a compound of formula III:

hal-B-CN (III)

wherein B is as defined above and hal represents chlorine or bromine, to give a compound of formula Ib wherein D is CN,

or

b) carrying out a reductive alkylation of a compound of formula II as defined above using a compound of formula IV

OHC-$B^1$-CN (IV)

wherein $B^1$ is an alkylene chain of 2 carbon atoms optionally substituted by $C_1$-$C_6$ alkyl, in the presence of a reducing agent to give a corresponding compound of formula Ib wherein D is CN and B represents -$CH_2$-$B^1$-; or

(c) reducing a compound of formula (V)

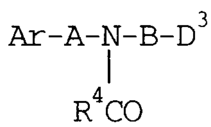

(V)

wherein $R^4$ is alkyl of 1 to 5 carbon atoms or $Ar^1$-$A^2$-; Ar, $Ar^1$, A and B are as defined above, $D^3$ represents CN, COOR or COOH and $A^2$ represents a direct bond or alkylene of 1 carbon atom optionally substituted by $C_1$-$C_6$ alkyl and/or aryl to give a corresponding compound of formula Ib wherein D is CN or COOR or formula Ia wherein $D^1$ is COOH and E is $R^4CH_2$ wherein $R^4$ is as defined above;

or

(d) reducing a compound of formula (VI)

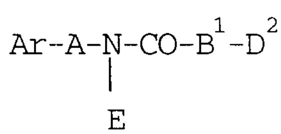

(VI)

wherein Ar, A and E are as defined above, $B^1$ is as defined in connection with formula IV and $D^2$ represents CN, COOR or CHO to give a corresponding compound of formula Ib wherein B is -$CH_2B^1$- and D is CN, COOR or CHO,

or

(e) partially hydrolysing a compound of formula Ib

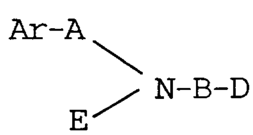

(Ib)

wherein D is CN and Ar, A, E and B are as defined above to give a corresponding compound of formula Ia wherein $D^1$ is $CONH_2$;

or

(f) hydrolysing a compound of formula Ib wherein D is CN or formula Ia wherein $D^1$ is $CONH_2$ to give a corresponding compound of formula Ia wherein $D^1$ is COOH;

or

(g) reacting a compound of formula Ib wherein D is COOR where COOR is an ester function or COhal where hal is a halogen with ammonia or an amine of formula $HNR^1R^2$ to give a corresponding compound of formula Ia wherein $D^1$ is $-CONR^1R^2$;

or

(h) hydrolysing an ester of formula Ib wherein D is COOR where COOR is an ester function to give a carboxylic acid of formula Ia wherein $D^1$ is COOH;

or

(i) oxidising an aldehyde of formula Ib wherein D is CHO to give an acid of formula Ia wherein $D^1$ is COOH; or

(j) oxidising an alcohol of formula Ib wherein D is $-CH_2OH$ to give an aldehyde formula Ib wherein D is CHO or an acid of formula Ia wherein $D^1$ is COOH;

or

(k) oxidising a compound of formula

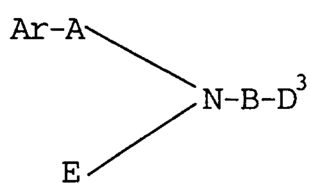

(VII)

wherein Ar, A, E and B are as defined above and $D^3$ is $-C{\equiv}CH$, $COCH_3$ or $-CH{=}CH_2$ to give a compound of formula Ia wherein $D^1$ is COOH;

or

(l) hydrolysing a compound of formula

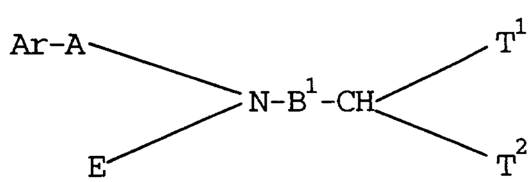

(VIII)

wherein Ar, A and E are as defined above, $T^1$ and $T^2$ are each independently an ester function, a nitrile or an acyl group, and $B^1$ is an alkylene group of 2 carbon atoms optionally substituted by $C_1$-$C_6$ alkyl to give a corresponding compound of formula Ia wherein B is $-B^1-CH_2-$ and $D^1$ is COOH;

or

(m) esterifying an acid of formula Ia wherein $D^1$ is COOH or acid halide of formula Ib wherein D is COhal to give an ester of formula Ib wherein D is-COOR;

or

(n) halogenating an acid of formula Ia to give an acid halide of formula Ib wherein $D^1$ is COhal;

or

(o) reducing an acid of formula Ia wherein $D^1$ is COOH to give an alcohol of formula Ib wherein D is $CH_2OH$,

or

(p) acidifying a compound of formula Ia to give an acid addition salt thereof or neutralising an acid addition salt to give the free base form.

**Claims for the following Contracting State : ES**

1. Use of a compound which acts selectively as an agonist of gamma amino butyric acid (GABA) at GABA autoreceptors in the preparation of a medicament for the treatment of depression or senile dementia or other forms of memory impairment with the proviso that the compound is not fengabine or progabide.

2. A use according to Claim 1 in which the selectivity for the GABA autoreceptor relative to the $GABA_A$ receptor is greater than or equal to 100.

3. A use according to Claim 1 or Claim 2 wherein the GABA autoreceptor agonist is a trisubstituted amine in which one substituent is a 3- or 4- carbon alkyl group carrying a carboxylic acid or amide functional group.

4. A use according to Claims 1 to 3 wherein the GABA autoreceptor agonist has the formula:

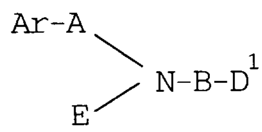

(Ia)

wherein E is $C_1$-$C_6$ alkyl or $Ar^1$-$A^1$;
Ar and $Ar^1$ are the same or different optionally substituted aryl groups;
A and $A^1$ are the same or different alkylene groups having one or two carbon atoms linking Ar or $Ar^1$ to N, each optionally substituted by $C_1$-$C_6$ alkyl or optionally substituted aryl;
B is an alkylene group of 3 carbon atoms which may be substituted by $C_1$-$C_6$ alkyl;
$D^1$ represents COOH or $CONR^1R^2$ wherein $R^1$ and $R^2$ are independently hydrogen, $C_1$-$C_6$ alkyl or aralkyl of 7 to 12 carbon atoms or a pharmaceutically acceptable salt thereof.

5. A use according to Claim 4 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

6. A use according to Claim 5 wherein Ar and $Ar^1$ when present are selected from phenyl, naphthyl, pyridinyl, furanyl, thienyl, quinolinyl and benzofuranyl which groups may be optionally substituted.

7. A use according to Claim 6 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

8. A use according to any one of Claims 4 to 7 wherein A and $A^1$ when present are independently selected from -$CHR^3$-where $R^3$ is hydrogen, $C_1$-$C_6$ alkyl or optionally substituted aryl as defined for Ar.

9. A use according to Claim 4 in which the compound of formula Ia is selected from one of the following:
4-[N,N-bis-(4-chlorobenzyl)amino]butyric acid,
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyramide,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid,
4-[N-methyl-N-(p-chlorobenzyl)amino]butanoic acid,
4-(N,N-dibenzylamino)butyric acid,

4-[(N-(4-methylphenylmethyl)-N-phenylmethyl)amino]butanoic acid,
4-[(N-(1,1-diphenyl)methyl-N-benzyl)amino]butyric acid,
4-[(N-(p-chlorobenzyl)-N-methyl)amino]butyramide, or
4-(N,N-dibenzylamino)-N',N'-dimethylbutanamide or a pharmaceutically acceptable salt thereof.

**10.** A process for preparing a pharmaceutical composition which comprises bringing a compound of formula Ia as shown and defined in Claim 4 or a pharmaceutically acceptable salt thereof possessing selective GABA autoreceptor agonist activity into association with a pharmaceutically acceptable carrier with the provisos
(i) when E is $C_1$-$C_6$ alkyl and A is alkylene having 2 carbon atoms linking Ar to N and $D^1$ is $CONR^1R^2$ then Ar is other than phenyl or
substituted phenyl,
(ii) when E is $C_1$-$C_6$ alkyl and $D^1$ is COOH the Ar is other than 3-pyridyl, and
(iii) when A is methylene and E is $C_1$-$C_6$ alkyl, benzyl or phenethyl and $D^1$ is $CONR^1R^2$ then Ar is other than substituted phenyl.

**11.** A process according to Claim 10 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**12.** A process according to Claim 10 wherein Ar and $Ar^1$ when present are selected from phenyl, naphthyl, pyridinyl, furanyl, thienyl, quinolinyl and benzofuranyl which groups may be optionally substituted.

**13.** A process according to Claim 12 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**14.** A process according to Claim 10 in which the compound of formula Ia is selected from one of the following:
4-[N,N-bis-(4-chlorobenzyl)amino]butyric acid,
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyric acid,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid, or
4-[N-methyl-N-(p-chlorobenzyl)amino]butanoic acid, or a pharmaceutically acceptable salt thereof.

**15.** A process for preparing a compound of formula Ia or Ib:

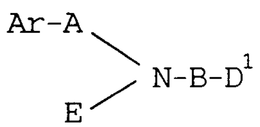
(Ia)

or

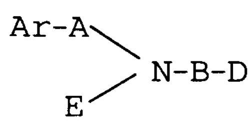
(Ib)

wherein E, B, Ar, $Ar^1$, A, $D^1$ and $A^1$ are as defined in Claim 4 and D represents CN, COhal, $CH_2OH$, CHO or an ester function COOR where R is an organic radical providing that
(i) when D is COOR then E is other than $C_1$-$C_6$ alkyl;
(ii) when D is COOR then Ar-A and $Ar^1$-$A^1$ are not both p-nitrobenzyl,
(iii) neither Ar-A or E is unsubstituted benzyl,

(iv) when E is $C_1$-$C_6$ alkyl, Ar is not phenyl, substituted phenyl or a 5 or 6 membered heteroaromatic group,

and

(v) when E is phenethyl, Ar is not substituted benzyl, which comprises one of the following:

a) reacting a compound of formula II

Ar-A-NH-E (II)

wherein Ar, E and A are as defined above with a compound of formula III:

hal-B-CN (III)

wherein B is as defined above and hal represents chlorine or bromine, to give a compound of formula Ib wherein D is CN,

or

b) carrying out a reductive alkylation of a compound of formula II as defined above using a compound of formula IV

OHC-$B^1$-CN (IV)

wherein $B^1$ is an alkylene chain of 2 carbon atoms optionally substituted by $C_1$-$C_6$ alkyl, in the presence of a reducing agent to give a corresponding compound of formula Ib wherein D is CN and B represents -$CH_2$-$B^1$-; or

(c) reducing a compound of formula (V)

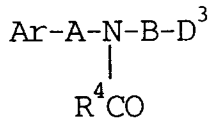

(V)

wherein $R^4$ is alkyl of 1 to 5 carbon atoms or $Ar^1$-$A^2$-;

Ar, $Ar^1$, A and B are as defined above,

$D^3$ represents CN, COOR or COOH and $A^2$ represents a direct bond or alkylene of 1 carbon atom optionally substituted by $C_1$-$C_6$ alkyl and/or aryl to give a corresponding compound of formula Ib wherein D is CN or COOR or formula Ia wherein $D^1$ is COOH and E is $R^4CH_2$ wherein $R^4$ is as defined above;

or

(d) reducing a compound of formula (VI)

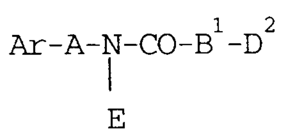

(VI)

wherein Ar, A and E are as defined above, $B^1$ is as defined in connection with formula IV and $D^2$ represents CN, COOR or CHO to give a corresponding compound of formula Ib wherein B is -$CH_2B^1$- and D is CN, COOR or CHO,

or

(e) partially hydrolysing a compound of formula Ib

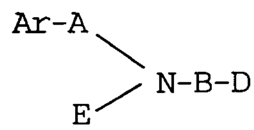

(Ib)

wherein D is CN and Ar, A, E and B are as defined above to give a corresponding compound of formula Ia wherein $D^1$ is $CONH_2$;

or

(f) hydrolysing a compound of formula Ib wherein D is CN or formula Ia wherein $D^1$ is $CONH_2$ to give a corresponding compound of formula Ia wherein $D^1$ is COOH;

or

(g) reacting a compound of formula Ib wherein D is COOR where COOR is an ester function or COhal where hal is a halogen with ammonia or an amine of formula $HNR^1R^2$ to give a corresponding compound of formula Ia wherein $D^1$ is $-CONR^1R^2$;

or

(h) hydrolysing an ester of formula Ib wherein D is COOR where COOR is an ester function to give a carboxylic acid of formula Ia wherein $D^1$ is COOH;

or

(i) oxidising an aldehyde of formula Ib wherein D is CHO to give an acid of formula Ia wherein $D^1$ is COOH; or

(j) oxidising an alcohol of formula Ib wherein D is $-CH_2OH$ to give an aldehyde formula Ib wherein D is CHO or an acid of formula Ia wherein $D^1$ is COOH;

or

(k) oxidising a compound of formula

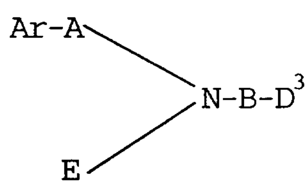

(VII)

wherein Ar, A, E and B are as defined above and $D^3$ is $-C{\equiv}CH$, $COCH_3$ or $-CH{=}CH_2$ to give a compound of formula Ia wherein $D^1$ is COOH;

or

(l) hydrolysing a compound of formula

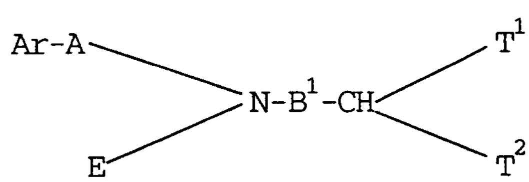

(VIII)

wherein Ar, A and E are as defined above,$T^1$ and $T^2$ are each independently an ester function, a nitrile or an acyl group, and $B^1$ is an alkylene group of 2 carbon atoms optionally substituted by $C_1$-$C_6$ alkyl to give a corresponding compound of formula Ia wherein B is $-B^1-CH_2-$ and $D^1$ is COOH;

or

(m) esterifying an acid of formula Ia wherein $D^1$ is COOH or acid halide of formula Ib wherein D is COhal to give an ester of formula Ib wherein D is-COOR;
or
(n) halogenating an acid of formula Ia to give an acid halide of formula Ib wherein $D^1$ is COhal;
or
(o) reducing an acid of formula Ia wherein $D^1$ is COOH to give an alcohol of formula Ib wherein D is $CH_2OH$,
or
(p) acidifying a compound of formula Ia to give an acid addition salt thereof or neutralising an acid addition salt to give the free base form.

**16.** A process according to Claim 15 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**17.** A process according to Claim 16 wherein Ar and $Ar^1$ when present are selected from phenyl, naphthyl, pyridinyl, furanyl, thienyl, quinolinyl and benzofuranyl which groups may be optionally substituted.

**18.** A process to Claim 17 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**19.** A process according to any one of Claims 15 to 18 wherein A and $A^1$ when present are independently selected from -$CHR^3$-where $R^3$ is hydrogen, $C_1$-$C_6$ alkyl or optionally substituted aryl as defined for Ar.

**20.** A process according to Claim 15 in which the product is one of the following
4-[N,N-bis-(4-chlorobenzyl)amino]butyronitrile,
4-[N,N-bis-(4-methylbenzyl)amino]butyronitrile,
4-[N,N-bis-(3,4-dichlorobenzyl)amino]butyronitrile,
4-[N,N-bis-(4-methoxybenzyl)amino]butyronitrile,
4-[N,N-bis-(2-(4-chlorophenyl)ethyl)amino]butyronitrile,
Ethyl 4-[N,N-bis-(4-chlorobenzyl)amino]butanoate,
4-[N,N-bis-(4-chlorobenzyl)amino]butanol,
4-[N,N-bis-(3-bromobenzyl)amino]butyronitrile,
4-[N,N-bis-(3-chlorobenzyl)amino]butyronitrile,
4-[N,N-bis-(2-thienylmethyl)amino]butyronitrile,
4-[N,N-bis-(1-naphthalenemethyl)amino]butyronitrile
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyric acid,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid, or
4-[N-methyl-N-(p-chlorobenzyl)amino]butanoic acid, or a pharmaceutically acceptable salt thereof.

**Claims for the following Contracting State : GR**

**1.** Use of a compound which acts selectively as an agonist of gamma amino butyric acid (GABA) at GABA autoreceptors in the preparation of a medicament for the treatment of depression or senile dementia or other forms of memory impairment with the proviso that the compound is not fengabine or progabide.

**2.** A use according to Claim 1 in which the selectivity for the GABA autoreceptor relative to the $GABA_A$ receptor is greater than or equal to 100.

**3.** A use according to Claim 1 or Claim 2 wherein the GABA autoreceptor agonist is a trisubstituted amine in which one substituent is a 3- or 4- carbon alkyl group carrying a carboxylic acid or amide functional group.

**4.** A use according to Claims 1 to 3 wherein the GABA autoreceptor agonist has the formula:

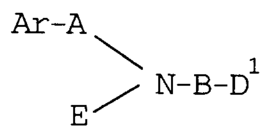

(Ia)

wherein E is $C_1$-$C_6$ alkyl or $Ar^1$-$A^1$;
Ar and $Ar^1$ are the same or different optionally substituted aryl groups;
A and $A^1$ are the same or different alkylene groups having one or two carbon atoms linking Ar or $Ar^1$ to N, each optionally substituted by $C_1$-$C_6$ alkyl or optionally substituted aryl; B is an alkylene group of 3 carbon atoms which may be substituted by $C_1$-$C_6$ alkyl;
$D^1$ represents COOH or $CONR^1R^2$ wherein $R^1$ and $R^2$ are independently hydrogen, $C_1$-$C_6$ alkyl or aralkyl of 7 to 12 carbon atoms or a pharmaceutically acceptable salt thereof.

**5.** A use according to Claim 4 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**6.** A use according to Claim 5 wherein Ar and $Ar^1$ when present are selected from phenyl, naphthyl, pyridinyl, furanyl, thienyl, quinolinyl and benzofuranyl which groups may be optionally substituted.

**7.** A use according to Claim 6 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**8.** A use according to any one of Claims 4 to 7 wherein A and $A^1$ when present are independently selected from $-CHR^3$-where $R^3$ is hydrogen, $C_1$-$C_6$ alkyl or optionally substituted aryl as defined for Ar.

**9.** A use according to Claim 4 in which the compound of formula Ia is selected from one of the following:
4-[N,N-bis-(4-chlorobenzyl)amino]butyric acid,
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyramide,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid,
4-[N-methyl-N-(p-chlorobenzyl)amino]butanoic acid,
4-(N,N-dibenzylamino)butyric acid,
4-[(N-(4-methylphenylmethyl)-N-phenylmethyl)amino]butanoic acid,
4-[(N-(1,1-diphenyl)methyl-N-benzyl)amino]butyric acid,
4-[(N-(p-chlorobenzyl)-N-methyl)amino]butyramide, or
4-(N,N-dibenzylamino)-N',N'-dimethylbutanamide or a pharmaceutically acceptable salt thereof.

**10.** A process for preparing a pharmaceutical composition which comprises bringing a compound of formula Ia as shown and defined in Claim 4 or a pharmaceutically acceptable salt thereof possessing selective GABA autoreceptor agonist activity into association with a pharmaceutically acceptable carrier with the provisos

(i) when E is $C_1$-$C_6$ alkyl and A is alkylene having 2 carbon atoms linking Ar to N and $D^1$ is $CONR^1R^2$ then Ar is other than phenyl or
substituted phenyl,
(ii) when E is $C_1$-$C_6$ alkyl and $D^1$ is COOH the Ar is other than 3-pyridyl, and
(iii) when A is methylene and E is $C_1$-$C_6$ alkyl, benzyl or phenethyl and $D^1$ is $CONR^1R^2$ then Ar is other than substituted phenyl.

**11.** A process according to Claim 10 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**12.** A process according to Claim 10 wherein Ar and $Ar^1$ when present are selected from phenyl, naphthyl, pyridinyl, furanyl, thienyl, quinolinyl and benzofuranyl which groups may be optionally substituted.

**13.** A process according to Claim 12 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**14.** A process according to Claim 10 in which the compound of formula Ia is selected from one of the following:
4-[N,N-bis-(4-chlorobenzyl)amino]butyric acid,
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyric acid,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid, or
4-[N-methyl-N-(p-chlorobenzyl)amino]butanoic acid, or a pharmaceutically acceptable salt thereof.

**15.** A process for preparing a compound of formula Ia or Ib:

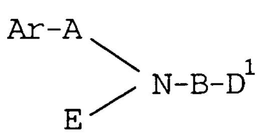

(Ia)

or

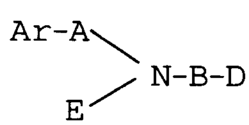

(Ib)

wherein E, B, Ar, $Ar^1$, A, $D^1$ and $A^1$ are as defined in Claim 4 and D represents CN, COhal, $CH_2OH$, CHO or an ester function COOR where R is an organic radical providing that
(i) when D is COOR then E is other than $C_1$-$C_6$ alkyl;
(ii) when D is COOR then Ar-A and $Ar^1$-$A^1$ are not both p-nitrobenzyl,
(iii) neither Ar-A or E is unsubstituted benzyl,
(iv) when E is $C_1$-$C_6$ alkyl, Ar is not phenyl, substituted phenyl or a 5 or 6 membered heteroaromatic group,
and
(v) when E is phenethyl, Ar is not substituted benzyl, which comprises one of the following:
a) reacting a compound of formula II

Ar-A-NH-E (II)

wherein Ar, E and A are as defined above with a compound of formula III:

hal-B-CN (III)

wherein B is as defined above and hal represents chlorine or bromine, to give a compound of formula Ib wherein D is CN,

or

b) carrying out a reductive alkylation of a compound of formula II as defined above using a compound of formula IV

$OHC-B^1-CN$ (IV)

wherein $B^1$ is an alkylene chain of 2 carbon atoms optionally substituted by $C_1$-$C_6$ alkyl, in the presence of a reducing agent to give a corresponding compound of formula Ib wherein D is CN and B represents $-CH_2-B^1-$; or

(c) reducing a compound of formula (V)

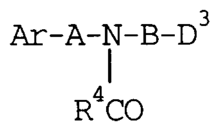

(V)

wherein $R^4$ is alkyl of 1 to 5 carbon atoms or $Ar^1-A^2-$;

Ar, $Ar^1$, A and B are as defined above,

$D^3$ represents CN, COOR or COOH and $A^2$ represents a direct bond or alkylene of 1 carbon atom optionally substituted by $C_1$-$C_6$ alkyl and/or aryl to give a corresponding compound of formula Ib wherein D is CN or COOR or formula Ia wherein $D^1$ is COOH and E is $R^4CH_2$ wherein $R^4$ is as defined above;

or

(d) reducing a compound of formula (VI)

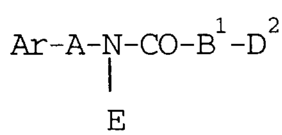

(VI)

wherein Ar, A and E are as defined above, $B^1$ is as defined in connection with formula IV and $D^2$ represents CN, COOR or CHO to give a corresponding compound of formula Ib wherein B is $-CH_2B^1-$ and D is CN, COOR or CHO,

or

(e) partially hydrolysing a compound of formula Ib

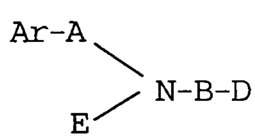

(Ib)

wherein D is CN and Ar, A, E and B are as defined above to give a corresponding compound of formula Ia wherein $D^1$ is $CONH_2$;

or

(f) hydrolysing a compound of formula Ib wherein D is CN or formula Ia wherein $D^1$ is $CONH_2$ to give a corresponding compound of formula Ia wherein $D^1$ is COOH;

or

(g) reacting a compound of formula Ib wherein D is COOR where COOR is an ester function or COhal where hal is a halogen with ammonia or an amine of formula $HNR^1R^2$ to give a

corresponding compound of formula Ia wherein $D^1$ is $-CONR^1R^2$;
or
(h) hydrolysing an ester of formula Ib wherein D is COOR where COOR is an ester function to give a carboxylic acid of formula Ia wherein $D^1$ is COOH;
or
(i) oxidising an aldehyde of formula Ib wherein D is CHO to give an acid of formula Ia wherein $D^1$ is COOH; or
(j) oxidising an alcohol of formula Ib wherein D is $-CH_2OH$ to give an aldehyde formula Ib wherein D is CHO or an acid of formula Ia wherein $D^1$ is COOH;
or
(k) oxidising a compound of formula

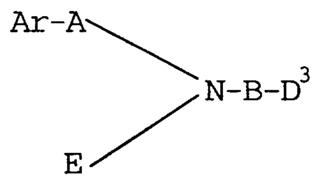

(VII)

wherein Ar, A, E and B are as defined above and $D^3$ is $-C{\equiv}CH$, $COCH_3$ or $-CH=CH_2$ to give a compound of formula Ia wherein $D^1$ is COOH;
or
(l) hydrolysing a compound of formula

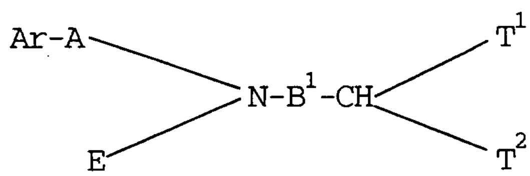

(VIII)

wherein Ar, A and E are as defined above,$T^1$ and $T^2$ are each independently an ester function, a nitrile or an acyl group, and $B^1$ is an alkylene group of 2 carbon atoms optionally substituted by $C_1$-$C_6$ alkyl to give a corresponding compound of formula Ia wherein B is $-B^1-CH_2-$ and $D^1$ is COOH;
or
(m) esterifying an acid of formula Ia wherein $D^1$ is COOH or acid halide of formula Ib wherein D is COhal to give an ester of formula Ib wherein D is-COOR;
or
(n) halogenating an acid of formula Ia to give an acid halide of formula Ib wherein $D^1$ is COhal;
or
(o) reducing an acid of formula Ia wherein $D^1$ is COOH to give an alcohol of formula Ib wherein D is $CH_2OH$,
or
(p) acidifying a compound of formula Ia to give an acid addition salt thereof or neutralising an acid addition salt to give the free base form.

**16.** A process according to Claim 15 in which Ar and $Ar^1$ when present are independently selected from mono- or bi-cyclic carbocyclic aryl groups of 6 to 10 carbon atoms and mono- or bi-cyclic heterocyclic aryl groups of 5 to 10 ring atoms in which the heteroatom(s) present is/are selected from nitrogen, oxygen and sulphur.

**17.** A process according to Claim 16 wherein Ar and $Ar^1$ when present are selected from phenyl, naphthyl, pyridinyl, furanyl, thienyl, quinolinyl and benzofuranyl which groups may be optionally substituted.

**18.** A process to Claim 17 in which the optional substituent is selected from one or more of the following $C_1$-$C_6$ alkyl, $C_1$-$C_6$ alkoxy, halogen, halo $C_1$-$C_6$ alkyl, halo $C_1$-$C_6$ alkoxy, nitro, cyano and optionally substituted amino.

**19.** A process according to any one of Claims 15 to 18 wherein A and $A^1$ when present are independently selected from -$CHR^3$-where $R^3$ is hydrogen, $C_1$-$C_6$ alkyl or optionally substituted aryl as defined for Ar.

**20.** A process according to Claim 15 in which the product is one of the following
4-[N,N-bis-(4-chlorobenzyl)amino]butyramide,
4-[N,N-bis-(4-methylbenzyl)amino]butyramide,
4-[N,N-bis-(3,4-dichlorobenzyl)amino]butyric acid,
4-[N,N-bis-(4-methoxybenzyl)amino]butyric acid,
4-[N,N-bis-(2-(4-chlorophenyl)ethyl)amino]butyric acid,
4-[N,N-bis-(3-bromobenzyl)amino]butanoic acid,
4-[N,N-bis-(3-chlorobenzyl)amino]butanoic acid,
4-[N,N-bis-(2-thienylmethyl)amino]butanoic acid,
4-[N,N-bis-(1-naphthalenemethyl)amino]butanoic acid, or
4-[N-methyl-N-(p-chlorobenzyl)amino]butanoic acid, or a pharmaceutically acceptable salt thereof.

**21.** An intermediate for a compound of formula Ia as claimed in Claim 15, said intermediate having the formula (Ib):

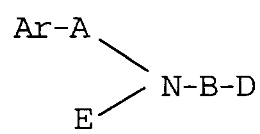

(Ib)

or a salt thereof wherein E, B, Ar, $Ar^1$, A and $A^1$ are as defined in Claim 15 and D represents CN, COhal, $CH_2OH$, CHO or an ester function COOR where R is an organic radical providing that
(i) when D is COOR then E is other than $C_1$-$C_6$ alkyl; and
(ii) when D is COOR then Ar-A and $Ar^1$-$A^1$ are not both p-nitrobenzyl.

**22.** A compound of formula Ib as claimed in Claim 21 which is one of the following:
4-[N,N-bis-(4-chlorobenzyl)amino]butyronitrile,
4-[N,N-bis-(4-methylbenzyl)amino]butyronitrile,
4-[N,N-bis-(3,4-dichlorobenzyl)amino]butyronitrile,
4-[N,N-bis-(4-methoxybenzyl)amino]butyronitrile,
4-[N,N-bis-(2-(4-chlorophenyl)ethyl)amino]butyronitrile,
Ethyl 4-[N,N-bis-(4-chlorobenzyl)amino]butanoate,
4-[N,N-bis-(4-chlorobenzyl)amino]butanol,
4-[N,N-bis-(3-bromobenzyl)amino]butyronitrile,
4-[N,N-bis-(3-chlorobenzyl)amino]butyronitrile,
4-[N,N-bis-(2-thienylmethyl)amino]butyronitrile, or
4-[N,N-bis-(1-naphthalenemethyl)amino]butyronitrile
or a pharmaceutically acceptable salt thereof.

**Patentansprüche**

**Patentansprüche für folgende Vertragsstaaten : AT, BE, CH, DE, FR, IT, LI, LU, NL, SE**

**1.** Verwendung einer Verbindung, die selektiv als Agonist von Gamma-Aminobuttersäure (GABA) an GABA-Autorezeptoren wirkt, bei der Herstellung eines Medikaments zur Behandlung von Depression oder Altersdemenz oder anderen Formen von Gedächtnisstörungen mit der Maßgabe, daß die Verbindung nicht Fengabin oder Progabid ist.

**2.** Verwendung nach Anspruch 1, wobei die Selektivität für den GABA-Autorezeptor im Verhältnis zum $GABA_A$-Rezeptor größer als oder gleich 100 ist.

**3.** Verwendung nach Anspruch 1 oder Anspruch 2, wobei der GABA-Autorezeptor-Agonist ein trisubstituiertes Amin ist, bei welchem ein Substituent eine 3- oder 4-Carbonalkylgruppe ist, die eine funktionelle Carbonsäure- oder Amid-Gruppe trägt.

**4.** Verwendung nach den Ansprüchen 1 bis 3, wobei der GABA-Autorezeptor-Agonist die Formel:

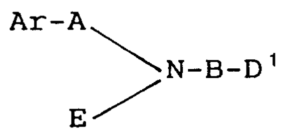

(Ia)

hat, worin E für $C_1$-$C_6$-Alkyl oder $Ar^1$-$A^1$ steht;
Ar und $Ar^1$ gleiche oder verschiedene, gegebenenfalls substituierte, Arylgruppen sind;
A und $A^1$ gleiche oder verschiedene Alkylengruppen sind, die ein oder zwei Kohlenstoffatome aufweisen, welche Ar oder $Ar^1$ mit N verbinden, wobei jedes gegebenenfalls durch $C_1$-$C_6$-Alkyl oder durch gegebenenfalls substituiertes Aryl substituiert ist;
B eine Alkylengruppe mit 3 Kohlenstoffatomen ist, die durch $C_1$-$C_6$-Alkyl substituiert sein kann;
$D^1$ für COOH oder $CONR^1R^2$ steht, worin $R^1$ und $R^2$ unabhängig voneinander Wasserstoff, $C_1$-$C_6$-Alkyl oder Aralkyl mit 7 bis 12 Kohlenstoffatomen sind, oder eines pharmazeutisch akzeptablen Salzes davon.

**5.** Verwendung nach Anspruch 4, wobei Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**6.** Verwendung nach Anspruch 5, wobei Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

**7.** Verwendung nach Anspruch 6, wobei der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**8.** Verwendung nach einem der Ansprüche 4 bis 7, wobei A und $A^1$, wenn vorhanden, unabhängig voneinander ausgewählt sind aus - $CHR^3$-, worin $R^3$ Wasserstoff, $C_1$-$C_6$-Alkyl oder gegebenenfalls substituiertes Aryl, wie für Ar definiert, ist.

**9.** Verwendung nach Anspruch 4, wobei die Verbindung der Formel Ia ausgewählt ist aus einem von Folgendem:
4-[N,N-bis-(4-Chlorbenzyl)-amino]-buttersäure,
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-butyramid,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure,
4-[N-Methyl-N-(p-Chlorbenzyl)-amino]-butansäure,
4-(N,N-Dibenzylamino)-buttersäure,
4-[(N-(4-Methylphenylmethyl)-N-phenylmethyl)-amino]-butansäure,
4-[(N-(1,1-Diphenyl)-methyl-N-benzyl)-amino]-buttersäure,
4-[(N-(p-chlorbenzyl)-N-methyl)-amino]-butyramid oder
4-(N,N-Dibenzylamino)-N',N'-dimethylbutanamid oder ein
pharmazeutisch akzeptables Salz davon.

**10.** Pharmazeutische Zusammensetzung, umfassend eine Verbindung der Formel Ia, wie in Anspruch 4 gezeigt und definiert, oder ein pharmazeutisch akzeptables Salz davon, welche(s) eine selektive GABA-Autorezeptor-Agonist-Aktivität besitzt, und einen pharmazeutisch akzeptablen Träger mit der Maßgabe, daß:

(i) wenn E $C_1$-$C_6$-Alkyl ist und A Alkylen mit 2 Kohlenstoffatomen ist, das Ar mit N verbindet, und $D^1$ $CONR^1R^2$ ist, dann Ar etwas anderes als Phenyl oder subsituiertes Phenyl ist,

(ii) wenn E $C_1$-$C_6$-Alkyl ist und $D^1$ COOH ist, dann Ar etwas anderes als 3-Pyridyl ist, und

(iii) wenn A Methylen ist und E $C_1$-$C_6$-Alkyl, Benzyl oder Phenethyl ist und $D^1$ $CONR^1R^2$ ist, dann Ar etwas anderes als substituiertes Phenyl ist.

**11.** Zusammensetzung nach Anspruch 10, worin Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**12.** Zusammensetzung nach Anspruch 10, worin Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

**13.** Zusammensetzung nach Anspruch 12, wobei der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**14.** Zusammensetzung nach Anspruch 10, wobei die Verbindung der Formel Ia ausgewählt ist aus einem von Folgendem:
4-[N,N-bis-(4-Chlorbenzyl)-amino]-buttersäure,
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-buttersäure,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure, oder
4-[N-Methyl-N-(p-Chlorbenzyl)-amino]-butansäure,
oder ein pharmazeutisch akzeptables Salz davon.

**15.** Verbindung der Formel I, wie in Anspruch 4 gezeigt und definiert, oder ein pharmazeutisch akzeptables Salz davon, welches eine selektive GABA-Autorezeptor-Agonist-Aktivität besitzt, mit der Maßgabe, daß

(i) weder Ar-A noch E unsubstituiertes Benzyl ist,

(ii) wenn E $C_1$-$C_6$-Alkyl ist, Ar nicht Phenyl, substituiertes Phenyl oder eine 5- oder 6-gliedrige heteroaromatische Gruppe ist, und

(iii) wenn E Phenethyl ist, Ar nicht substituiertes Benzyl ist.

**16.** Verbindung nach Anspruch 15, worin Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**17.** Verbindung nach Anspruch 16, worin Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

**18.** Verbindung nach Anspruch 17, worin der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**19.** Verbindung nach einem der Ansprüche 15 bis 18, wobei A und $A^1$, wenn vorhanden, unabhängig voneinander ausgewählt sind aus $-CHR^3-$, worin $R^3$ Wasserstoff, $C_1$-$C_6$-Alkyl oder gegebenenfalls substituiertes Aryl, wie für Ar definiert, ist.

**20.** Verbindung nach Anspruch 15, worin die Verbindung der Formel Ia ausgewählt ist aus einem von Folgendem:
4-[N,N-bis-(4-Chlorbenzyl)-amino]-buttersäure,
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-buttersäure,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure, oder
4-[N-Methyl-N-(p-Chlorbenzyl)-amino]-butansäure, oder ein pharmazeutisch akzeptables Salz davon.

**21.** Zwischenprodukt für eine Verbindung der Formel Ia nach Anspruch 15, wobei dieses Zwischenprodukt die Formel (Ib):

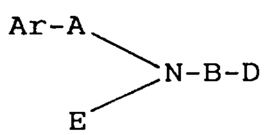

(Ib)

hat, oder ein Salz davon, worin E, B, Ar, $Ar^1$, A und $A^1$ die in Anspruch 15 angegebene Bedeutung haben und D CN, COhal, $CH_2OH$, CHO oder eine Esterfunktion COOR, worin R ein organischer Rest ist, darstellt, mit der Maßgabe, daß
(i) wenn D COOR ist, dann E etwas anderes als $C_1$-$C_6$-Alkyl ist; und
(ii) wenn D COOR ist, dann Ar-A und $Ar^1$-$A^1$ nicht beide p-Nitrobenzyl sind.

**22.** Verbindung nach Anspruch 21, welche eines von Folgendem ist:
4-[N,N-bis-(4-Chlorbenzyl)-amino]-butyronitril,
4-[N,N-bis-(4-Methylbenzyl)-amino]-butyronitril,
4-[N,N-bis-(3,4-Dichlorbenzyl)-amino]-butyronitril,
4-[N,N-bis-(4-Methoxybenzyl)-amino]-butyronitril,
4-[N,N-bis-(2-(4-Chlorphenyl)-ethyl)-amino]-butyronitril,
Ethyl-4-[N,N-bis-(4-chlorbenzyl)-amino]-butanoat,
4-[N,N-bis-(4-Chlorbenzyl)-amino]-butanol,
4-[N,N-bis-(3-Brombenzyl)-amino]butyronitril,
4-[N,N-bis-(3-Chlorbenzyl)-amino]-butyronitril,
4-[N,N-bis-(2-Thienylmethyl)-amino]-butyronitril oder
4-[N,N-bis-(1-Naphthalinmethyl)-amino]-butyronitril
oder ein Salz davon.

**23.** Verfahren zur Herstellung einer Verbindung der Formel Ia oder Ib, wie in Anspruch 15 oder Anspruch 21 definiert, welches eines von Folgendem umfaßt:
a) Umsetzung einer Verbindung der Formel II

Ar-A-NH-E ,   (II)

worin Ar, E und A die obige Bedeutung haben, mit einer Verbindung der Formel III

hal-B-CN ,   (III)

worin B die obige Bedeutung hat und hal Chlor oder Brom darstellt, zum Erhalt einer Verbindung der

Formel Ib, worin D für CN steht, oder

b) Durchführung einer reduktiven Alkylierung einer Verbindung der Formel II, wie oben definiert, unter Verwendung einer Verbindung der Formel IV

$OHC-B^1-CN$ , (IV)

worin $B^1$ eine Alkylenkette von 2 Kohlenstoffatomen, gegebenenfalls substituiert durch $C_1$-$C_6$-Alkyl, ist, in Anwesenheit eines Reduktionsmittels zum Erhalt einer entsprechenden Verbindung der Formel Ib, worin D für CN steht und B $-CH_2-B^1-$ darstellt; oder

c) die Reduktion einer Verbindung der Formel (V)

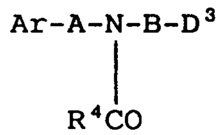

, (V)

worin $R^4$ Alkyl mit 1 bis 5 Kohlenstoffatomen oder $Ar^1-A^2$-bedeutet,
Ar, $Ar^1$, A und B die obige Bedeutung haben,
$D^3$ für CN, COOR oder COOH steht und $A^2$ eine direkte Bindung oder Alkylen mit 1 Kohlenstoffatom, gegebenenfalls substituiert durch $C_1$-$C_6$-Alkyl und/oder Aryl bedeutet, zum Erhalt einer entsprechenden Verbindung der Formel Ib, worin D für CN oder COOR oder die Formel Ia steht, worin $D^1$ für COOH steht und E $R^4CH_2$ bedeutet, worin $R^4$ die obige Bedeutung hat;

oder

(d) die Reduktion einer Verbindung der Formel (VI)

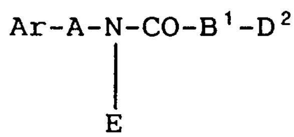

, (VI)

worin Ar, A und E die obige Bedeutung haben, $B^1$ wie in Verbindung mit Formel IV definiert ist und $D^2$ CN, COOR oder CHO darstellt, zum Erhalt einer entsprechenden Verbindung der Formel Ib, worin B $-CH_2B^1-$ bedeutet und D CN, COOR oder CHO ist,

oder

(e) teilweise Hydrolyse einer Verbindung der Formel Ib

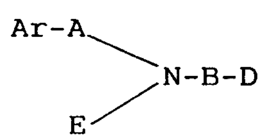

, (Ib)

worin D CN ist und Ar, A, E und B die obige Bedeutung haben, zum Erhalt einer entsprechenden Verbindung der Formel Ia, worin $D^1$ $CONH_2$ ist;

oder

(f) Hydrolyse einer Verbindung der Formel Ib, worin D CN ist oder der Formel Ia, worin $D^1$ $CONH_2$ ist, zum Erhalt einer entsprechenden Verbindung der Formel Ia, worin $D^1$ COOH ist;

oder

(g) Umsetzung einer Verbindung der Formel Ib, worin D COOR ist, worin COOR eine Esterfunktion oder COhal ist, worin hal ein Halogen bedeutet, mit Ammoniak oder einem Amin der Formel $HNR^1R^2$ zum Erhalt einer entsprechenden Verbindung der Formel Ia, worin $D^1$ $-CONR^1R^2$ ist;

oder

(h) Hydrolyse eines Esters der Formel Ib, worin D COOR ist, worin COOR eine Esterfunktion ist, zum Erhalt einer Carbonsäure der Formel Ia, worin $D^1$ COOH ist;

oder

(i) Oxidation eines Aldehyds der Formel Ib, worin D CHO ist, zum Erhalt einer Säure der Formel Ia, worin $D^1$ COOH ist;

oder

(j) Oxidation eines Alkohols der Formel Ib, worin D $-CH_2OH$ ist, zum Erhalt eines Aldehyds der Formel Ib, worin D CHO ist, oder einer Säure der Formel Ia, worin $D^1$ COOH ist;

oder

(k) Oxidation einer Verbindung der Formel

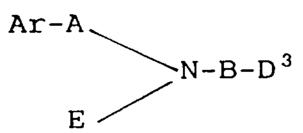

, (VII)

worin Ar, A, E und B die obige Bedeutung haben und $D^3$ $-C{\equiv}CH$, $COCH_3$ oder $-CH=CH_2$ ist, zum Erhalt einer Verbindung der Formel Ia, worin $D^1$ COOH ist;

oder

(l) Hydrolyse einer Verbindung der Formel

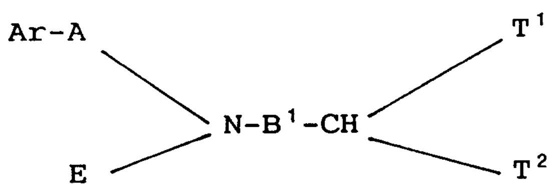

, (VIII)

worin Ar, A und E die obige Bedeutung haben, $T^1$ und $T^2$ jeweils unabhängig voneinander eine Esterfunktion, ein Nitril oder eine Acylgruppe sind und $B^1$ eine Alkylengruppe mit 2 Kohlenstoffatomen ist, gegenenfalls substituiert durch $C_1$-$C_6$-Alkyl, zum Erhalt einer entsprechenden Verbindung der Formel Ia, worin B $-B^1-CH_2$-ist und $D^1$ COOH ist,

oder

(m) Veresterung einer Säure der Formel Ia, worin $D^1$ COOH ist, oder eines Säurehalogenids der Formel Ib, worin D COhal ist, zum Erhalt eines Esters der Formel Ib, worin D -COOR ist;

oder

(n) Halogenierung einer Säure der Formel Ia zum Erhalt eines Säurehalogenids der Formel Ib, worin $D^1$ COhal ist;

oder

(o) Reduktion einer Säure der Formel Ia, worin $D^1$ COOH ist, zum Erhalt eines Alkohols der Formel Ib, worin D $CH_2OH$ ist;

oder

(p) Ansäuerung einer Verbindung der Formel Ia zum Erhalt eines Säureadditionssalzes derselben oder Neutralisierung eines Säureadditionssalzes zum Erhalt der freien Basenform.

**Patentansprüche für folgenden Vertragsstaat : ES**

1. Verwendung einer Verbindung, die selektiv als Agonist von Gamma-Aminobuttersäure (GABA) an GABA-Autorezeptoren wirkt, bei der Herstellung eines Medikaments zur Behandlung von Depression oder Altersdemenz oder anderen Formen von Gedächtnisstörungen mit der Maßgabe, daß die Verbindung nicht Fengabin oder Progabid ist.

2. Verwendung nach Anspruch 1, wobei die Selektivität für den GABA-Autorezeptor im Verhältnis zum $GABA_A$-Rezeptor größer als oder gleich 100 ist.

3. Verwendung nach Anspruch 1 oder Anspruch 2, wobei der GABA-Autorezeptor-Agonist ein trisubstituiertes Amin ist, bei welchem ein Substituent eine 3- oder 4-Carbonalkylgruppe ist, die eine funktionelle Carbonsäure- oder Amid-Gruppe trägt.

4. Verwendung nach den Ansprüchen 1 bis 3, wobei der GABA-Autorezeptor-Agonist die Formel:

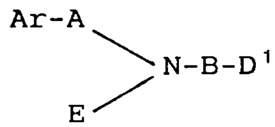

(Ia)

hat, worin E für $C_1$-$C_6$-Alkyl oder $Ar^1$-$A^1$ steht;
Ar und $Ar^1$ gleiche oder verschiedene, gegebenenfalls substituierte, Arylgruppen sind;
A und $A^1$ gleiche oder verschiedene Alkylengruppen sind, die ein oder zwei Kohlenstoffatome aufweisen, welche Ar oder $Ar^1$ mit N verbinden, wobei jedes gegebenenfalls durch $C_1$-$C_6$-Alkyl oder durch gegebenenfalls substituiertes Aryl substituiert ist;
B eine Alkylengruppe mit 3 Kohlenstoffatomen ist, die durch $C_1$-$C_6$-Alkyl substituiert sein kann;
$D^1$ für COOH oder $CONR^1R^2$ steht, worin $R^1$ und $R^2$ unabhängig voneinander Wasserstoff, $C_1$-$C_6$-Alkyl oder Aralkyl mit 7 bis 12 Kohlenstoffatomen sind, oder eines pharmazeutisch akzeptablen Salzes davon.

5. Verwendung nach Anspruch 4, wobei Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

6. Verwendung nach Anspruch 5, wobei Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

7. Verwendung nach Anspruch 6, wobei der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

8. Verwendung nach einem der Ansprüche 4 bis 7, wobei A und $A^1$, wenn vorhanden, unabhängig voneinander ausgewählt sind aus - $CHR^3$-, worin $R^3$ Wasserstoff, $C_1$-$C_6$-Alkyl oder gegebenenfalls substituiertes Aryl, wie für Ar definiert, ist.

9. Verwendung nach Anspruch 4, wobei die Verbindung der Formel Ia ausgewählt ist aus einem von Folgendem:
4-[N,N-bis-(4-Chlorbenzyl)-amino]-buttersäure,
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-butyramid,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure,
4-[N-Methyl-N-(p-Chlorbenzyl)-amino]-butansäure,
4-(N,N-Dibenzylamino)-buttersäure,
4-[(N-(4-Methylphenylmethyl)-N-phenylmethyl)-amino]-butansäure,
4-[(N-(1,1-Diphenyl)-methyl-N-benzyl)-amino]-buttersäure,
4-[(N-(p-chlorbenzyl)-N-methyl)-amino]-butyramid oder
4-(N,N-Dibenzylamino)-N',N'-dimethylbutanamid oder ein pharmazeutisch akzeptables Salz davon.

10. Verfahren zur Herstellung einer pharmazeutischen Zusammensetzung, umfassend das In-Verbindung-Bringen einer Verbindung der Formel Ia, wie in Anspruch 4 gezeigt und definiert, oder eines pharmazeutisch akzeptablen Salzes davon, welche(s) eine selektive GABA-Autorezeptor-Agonist-Aktivität besitzt, mit einem pharmazeutisch akzeptablen Träger mit der Maßgabe, daß:

(i) wenn E $C_1$-$C_6$-Alkyl ist und A Alkylen mit 2 Kohlenstoffatomen ist, das Ar mit N verbindet, und $D^1$ $CONR^1R^2$ ist, dann Ar etwas anderes als Phenyl oder subsituiertes Phenyl ist,

(ii) wenn E $C_1$-$C_6$-Alkyl ist und $D^1$ COOH ist, dann Ar etwas anderes als 3-Pyridyl ist, und

(iii) wenn A Methylen ist und E $C_1$-$C_6$-Alkyl, Benzyl oder Phenethyl ist und $D^1$ $CONR^1R^2$ ist, dann Ar etwas anderes als substituiertes Phenyl ist.

**11.** Verfahren nach Anspruch 10, worin Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**12.** Verfahren nach Anspruch 10, worin Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

**13.** Verfahren nach Anspruch 12, wobei der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**14.** Verfahren nach Anspruch 10, wobei die Verbindung der Formel Ia ausgewählt ist aus einem von Folgendem:
4-[N,N-bis-(4-Chlorbenzyl)-amino]-buttersäure,
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-buttersäure,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure, oder
4-[N-Methyl-N-(p-Chlorbenzyl)-amino]-butansäure,
oder ein pharmazeutisch akzeptables Salz davon.

**15.** Verfahren zur Herstellung einer Verbindung der Formel Ia oder Ib:

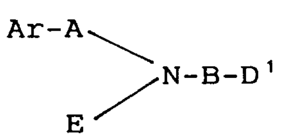
(Ia)

oder

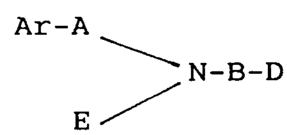
(Ib),

worin E, B, Ar, $Ar^1$, A, $D^1$ und $A^1$ wie in Anspruch 4 definiert sind und D für CN, COhal, $CH_2OH$, CHO oder eine Esterfunktion COOR steht, worin R ein organischer Rest ist, mit der Maßgabe, daß

(i) wenn D COOR ist, dann E etwas anderes als $C_1$-$C_6$-Alkyl ist;

(ii) wenn D COOR ist, dann Ar-A und $Ar^1$-$A^1$ nicht beide p-Nitrobenzyl sind,

(iii) weder Ar-A noch E unsubstituiertes Benzyl ist,

(iv) wenn E $C_1$-$C_6$-Alkyl ist, Ar nicht Phenyl, substituiertes Phenyl oder eine 5- oder 6-gliedrige heteroaromatische Gruppe ist,

und

(v) wenn E Phenethyl ist, Ar nicht substituiertes Benzyl ist, welches eines von Folgendem umfaßt:

a) Umsetzung einer Verbindung der Formel II

Ar-A-NH-E , (II)

worin Ar, E und A die obige Bedeutung haben, mit einer Verbindung der Formel III

hal-B-CN , (III)

worin B die obige Bedeutung hat und hal Chlor oder Brom darstellt, zum Erhalt einer Verbindung der Formel Ib, worin D für CN steht, oder

b) Durchführung einer reduktiven Alkylierung einer Verbindung der Formel II, wie oben definiert, unter Verwendung einer Verbindung der Formel IV

$OHC-B^1-CN$ , (IV)

worin $B^1$ eine Alkylenkette von 2 Kohlenstoffatomen, gegebenenfalls substituiert durch $C_1$-$C_6$-Alkyl, ist, in Anwesenheit eines Reduktionsmittels zum Erhalt einer entsprechenden Verbindung der Formel Ib, worin D für CN steht und B $-CH_2-B^1-$ darstellt; oder

c) die Reduktion einer Verbindung der Formel (V)

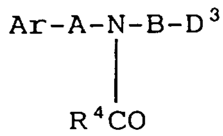

, (V)

worin $R^4$ Alkyl mit 1 bis 5 Kohlenstoffatomen oder $Ar^1$-$A^2$-bedeutet,
Ar, $Ar^1$, A und B die obige Bedeutung haben,
$D^3$ für CN, COOR oder COOH steht und $A^2$ eine direkte Bindung oder Alkylen mit 1 Kohlenstoffatom, gegebenenfalls substituiert durch $C_1$-$C_6$-Alkyl und/oder Aryl bedeutet, zum Erhalt einer entsprechenden Verbindung der Formel Ib, worin D für CN oder COOR oder die Formel Ia steht, worin $D^1$ für COOH steht und E $R^4CH_2$ bedeutet, worin $R^4$ die obige Bedeutung hat;
oder

(d) die Reduktion einer Verbindung der Formel (VI)

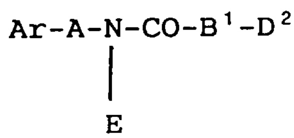

, (VI)

worin Ar, A und E die obige Bedeutung haben, $B^1$ wie in Verbindung mit Formel IV definiert ist und $D^2$ CN, COOR oder CHO darstellt, zum Erhalt einer entsprechenden Verbindung der Formel Ib, worin B $-CH_2B^1-$ bedeutet und D CN, COOR oder CHO ist,
oder

(e) teilweise Hydrolyse einer Verbindung der Formel Ib

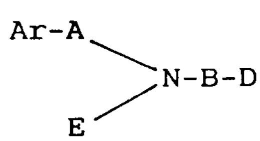

, (Ib)

worin D CN ist und Ar, A, E und B die obige Bedeutung haben, zum Erhalt einer entsprechenden Verbindung der Formel Ia, worin $D^1$ $CONH_2$ ist;
oder

(f) Hydrolyse einer Verbindung der Formel Ib, worin D CN ist oder der Formel Ia, worin $D^1$ $CONH_2$ ist, zum Erhalt einer entsprechenden Verbindung der Formel Ia, worin $D^1$ COOH ist;
oder

(g) Umsetzung einer Verbindung der Formel Ib, worin D COOR ist, worin COOR eine Esterfunktion oder COhal ist, worin hal ein Halogen bedeutet, mit Ammoniak oder einem Amin der Formel $HNR^1R^2$ zum Erhalt einer entsprechenden Verbindung der Formel Ia, worin $D^1$ $-CONR^1R^2$ ist;

oder
(h) Hydrolyse eines Esters der Formel Ib, worin D COOR ist, worin COOR eine Esterfunktion ist, zum Erhalt einer Carbonsäure der Formel Ia, worin $D^1$ COOH ist;
oder
(i) Oxidation eines Aldehyds der Formel Ib, worin D CHO ist, zum Erhalt einer Säure der Formel Ia, worin $D^1$ COOH ist;
oder
(j) Oxidation eines Alkohols der Formel Ib, worin D $-CH_2OH$ ist, zum Erhalt eines Aldehyds der Formel Ib, worin D CHO ist, oder einer Säure der Formel Ia, worin $D^1$ COOH ist;
oder
(k) Oxidation einer Verbindung der Formel

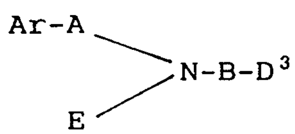

, (VII)

worin Ar, A, E und B die obige Bedeutung haben und $D^3$ $-C{\equiv}CH$, $COCH_3$ oder $-CH=CH_2$ ist, zum Erhalt einer Verbindung der Formel Ia, worin $D^1$ COOH ist;
oder
(l) Hydrolyse einer Verbindung der Formel

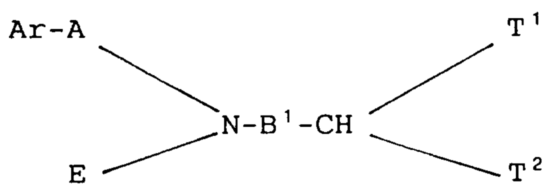

, (VIII)

worin Ar, A und E die obige Bedeutung haben, $T^1$ und $T^2$ jeweils unabhängig voneinander eine Esterfunktion, ein Nitril oder eine Acylgruppe sind und $B^1$ eine Alkylengruppe mit 2 Kohlenstoffatomen ist, gegenenfalls substituiert durch $C_1$-$C_6$-Alkyl, zum Erhalt einer entsprechenden Verbindung der Formel Ia, worin B $-B^1-CH_2-$ ist und $D^1$ COOH ist,
oder
(m) Veresterung einer Säure der Formel Ia, worin $D^1$ COOH ist, oder eines Säurehalogenids der Formel Ib, worin D COhal ist, zum Erhalt eines Esters der Formel Ib, worin D -COOR ist;
oder
(n) Halogenierung einer Säure der Formel Ia zum Erhalt eines Säurehalogenids der Formel Ib, worin $D^1$ COhal ist;
oder
(o) Reduktion einer Säure der Formel Ia, worin $D^1$ COOH ist, zum Erhalt eines Alkohols der Formel Ib, worin D $CH_2OH$ ist;
oder
(p) Ansäuerung einer Verbindung der Formel Ia zum Erhalt eines Säureadditionssalzes derselben oder Neutralisierung eines Säureadditionssalzes zum Erhalt der freien Basenform.

**16.** Verfahren nach Anspruch 15, worin Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**17.** Verfahren nach Anspruch 16, worin Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls

substituiert sein können.

**18.** Verfahren nach Anspruch 17, worin der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**19.** Verfahren nach einem der Ansprüche 15 bis 18, wobei A und $A^1$, wenn vorhanden, unabhängig voneinander ausgewählt sind aus -$CHR^3$-, worin $R^3$ Wasserstoff, $C_1$-$C_6$-Alkyl oder gegebenenfalls substituiertes Aryl, wie für Ar definiert, ist.

**20.** Verfahren nach Anspruch 15, worin das Produkt eines von Folgendem ist:
4-[N,N-bis-(4-Chlorbenzyl)-amino]-butyronitril,
4-[N,N-bis-(4-Methylbenzyl)-amino]-butyronitril,
4-[N,N-bis-(3,4-Dichlorbenzyl)-amino]-butyronitril,
4-[N,N-bis-(4-Methoxybenzyl)-amino]-butyronitril,
4-[N,N-bis-(2-(4-Chlorphenyl)-ethyl)-amino]-butyronitril,
Ethyl-4-[N,N-bis-(4-chlorbenzyl)-amino]-butanoat,
4-[N,N-bis-(4-Chlorbenzyl)-amino]-butanol,
4-[N,N-bis-(3-Brombenzyl)-amino]butyronitril,
4-[N,N-bis-(3-Chlorbenzyl)-amino]-butyronitril,
4-[N,N-bis-(2-Thienylmethyl)-amino]-butyronitril,
4-[N,N-bis-(1-Naphthalinmethyl)-amino]-butyronitril
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-buttersäure,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure oder,
4-[N-Methyl-N-(p-Chlorbenzyl)-amino]-butansäure,
oder ein pharmazeutisch akzeptables Salz davon.

**Patentansprüche für folgenden Vertragsstaat : GR**

**1.** Verwendung einer Verbindung, die selektiv als Agonist von Gamma-Aminobuttersäure (GABA) an GABA-Autorezeptoren wirkt, bei der Herstellung eines Medikaments zur Behandlung von Depression oder Altersdemenz oder anderen Formen von Gedächtnisstörungen mit der Maßgabe, daß die Verbindung nicht Fengabin oder Progabid ist.

**2.** Verwendung nach Anspruch 1, wobei die Selektivität für den GABA-Autorezeptor im Verhältnis zum $GABA_A$-Rezeptor größer als oder gleich 100 ist.

**3.** Verwendung nach Anspruch 1 oder Anspruch 2, wobei der GABA-Autorezeptor-Agonist ein trisubstituiertes Amin ist, bei welchem ein Substituent eine 3- oder 4-Carbonalkylgruppe ist, die eine funktionelle Carbonsäure- oder Amid-Gruppe trägt.

**4.** Verwendung nach den Ansprüchen 1 bis 3, wobei der GABA-Autorezeptor-Agonist die Formel:

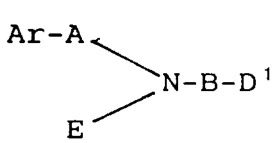

(Ia)

hat, worin E für $C_1$-$C_6$-Alkyl oder $Ar^1$-$A^1$ steht;
Ar und $Ar^1$ gleiche oder verschiedene, gegebenenfalls substituierte, Arylgruppen sind;

A und $A^1$ gleiche oder verschiedene Alkylengruppen sind, die ein oder zwei Kohlenstoffatome aufweisen, welche Ar oder $Ar^1$ mit N verbinden, wobei jedes gegebenenfalls durch $C_1$-$C_6$-Alkyl oder durch gegebenenfalls substituiertes Aryl substituiert ist;
B eine Alkylengruppe mit 3 Kohlenstoffatomen ist, die durch $C_1$-$C_6$-Alkyl substituiert sein kann;
$D^1$ für COOH oder $CONR^1R^2$ steht, worin $R^1$ und $R^2$ unabhängig voneinander Wasserstoff, $C_1$-$C_6$-Alkyl oder Aralkyl mit 7 bis 12 Kohlenstoffatomen sind, oder eines pharmazeutisch akzeptablen Salzes davon.

**5.** Verwendung nach Anspruch 4, wobei Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**6.** Verwendung nach Anspruch 5, wobei Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

**7.** Verwendung nach Anspruch 6, wobei der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**8.** Verwendung nach einem der Ansprüche 4 bis 7, wobei A und $A^1$, wenn vorhanden, unabhängig voneinander ausgewählt sind aus - $CHR^3$-, worin $R^3$ Wasserstoff, $C_1$-$C_6$-Alkyl oder gegebenenfalls substituiertes Aryl, wie für Ar definiert, ist.

**9.** Verwendung nach Anspruch 4, wobei die Verbindung der Formel Ia ausgewählt ist aus einem von Folgendem:
4-[N,N-bis-(4-Chlorbenzyl)-amino]-buttersäure,
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-butyramid,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure,
4-[N-Methyl-N-(p-Chlorbenzyl)-amino]-butansäure,
4-(N,N-Dibenzylamino)-buttersäure,
4-[(N-(4-Methylphenylmethyl)-N-phenylmethyl)-amino]-butansäure,
4-[(N-(1,1-Diphenyl)-methyl-N-benzyl)-amino]-buttersäure,
4-[(N-(p-chlorbenzyl)-N-methyl)-amino]-butyramid oder
4-(N,N-Dibenzylamino)-N',N'-dimethylbutanamid oder ein pharmazeutisch akzeptables Salz davon.

**10.** Verfahren zur Herstellung einer pharmazeutischen Zusammensetzung, umfassend das In-Verbindung-Bringen einer Verbindung der Formel Ia, wie in Anspruch 4 gezeigt und definiert, oder eines pharmazeutisch akzeptablen Salzes davon, welche(s) eine selektive GABA-Autorezeptor-Agonist-Aktivität besitzt, mit einem pharmazeutisch akzeptablen Träger mit der Maßgabe, daß:
(i) wenn E $C_1$-$C_6$-Alkyl ist und A Alkylen mit 2 Kohlenstoffatomen ist, das Ar mit N verbindet, und $D^1$ $CONR^1R^2$ ist, dann Ar etwas anderes als Phenyl oder subsituiertes Phenyl ist,
(ii) wenn E $C_1$-$C_6$-Alkyl ist und $D^1$ COOH ist, dann Ar etwas anderes als 3-Pyridyl ist, und
(iii) wenn A Methylen ist und E $C_1$-$C_6$-Alkyl, Benzyl oder Phenethyl ist und $D^1$ $CONR^1R^2$ ist, dann Ar etwas anderes als substituiertes Phenyl ist.

**11.** Verfahren nach Anspruch 10, worin Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**12.** Verfahren nach Anspruch 10, worin Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

**13.** Verfahren nach Anspruch 12, wobei der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**14.** Verfahren nach Anspruch 10, wobei die Verbindung der Formel Ia ausgewählt ist aus einem von Folgendem:
4-[N,N-bis-(4-Chlorbenzyl)-amino]-buttersäure,
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-buttersäure,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure, oder
4-[N-Methyl-N-(p-Chlorbenzyl)-amino]-butansäure,
oder ein pharmazeutisch akzeptables Salz davon.

**15.** Verfahren zur Herstellung einer Verbindung der Formel Ia oder Ib:

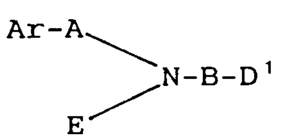
(Ia) oder

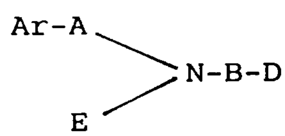
(Ib),

worin E, B, Ar, $Ar^1$, A, $D^1$ und $A^1$ wie in Anspruch 4 definiert sind und D für CN, COhal, $CH_2OH$, CHO oder eine Esterfunktion COOR steht, worin R ein organischer Rest ist, mit der Maßgabe, daß
(i) wenn D COOR ist, dann E etwas anderes als $C_1$-$C_6$-Alkyl ist;
(ii) wenn D COOR ist, dann Ar-A und $Ar^1$-$A^1$ nicht beide p-Nitrobenzyl sind,
(iii) weder Ar-A noch E unsubstituiertes Benzyl ist,
(iv) wenn E $C_1$-$C_6$-Alkyl ist, Ar nicht Phenyl, substituiertes Phenyl oder eine 5- oder 6-gliedrige heteroaromatische Gruppe ist,
und
(v) wenn E Phenethyl ist, Ar nicht substituiertes Benzyl ist, welches eines von Folgendem umfaßt:
a) Umsetzung einer Verbindung der Formel II

Ar-A-NH-E , (II)

worin Ar, E und A die obige Bedeutung haben, mit einer Verbindung der Formel III

hal-B-CN , (III)

worin B die obige Bedeutung hat und hal Chlor oder Brom darstellt, zum Erhalt einer Verbindung der Formel Ib, worin D für CN steht, oder
b) Durchführung einer reduktiven Alkylierung einer Verbindung der Formel II, wie oben definiert, unter Verwendung einer Verbindung der Formel IV

OHC-$B^1$-CN , (IV)

worin $B^1$ eine Alkylenkette von 2 Kohlenstoffatomen, gegebenenfalls substituiert durch $C_1$-$C_6$-Alkyl, ist, in Anwesenheit eines Reduktionsmittels zum Erhalt einer entsprechenden Verbindung

der Formel Ib, worin D für CN steht und B $-CH_2-B^1-$ darstellt; oder
c) die Reduktion einer Verbindung der Formel (V)

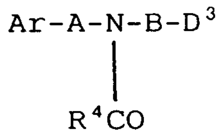

, (V)

worin $R^4$ Alkyl mit 1 bis 5 Kohlenstoffatomen oder $Ar^1-A^2$-bedeutet,
Ar, $Ar^1$, A und B die obige Bedeutung haben,
$D^3$ für CN, COOR oder COOH steht und $A^2$ eine direkte Bindung oder Alkylen mit 1 Kohlenstoffatom, gegebenenfalls substituiert durch $C_1$-$C_6$-Alkyl und/oder Aryl bedeutet, zum Erhalt einer entsprechenden Verbindung der Formel Ib, worin D für CN oder COOR oder die Formel Ia steht, worin $D^1$ für COOH steht und E $R^4CH_2$ bedeutet, worin $R^4$ die obige Bedeutung hat;
oder
(d) die Reduktion einer Verbindung der Formel (VI)

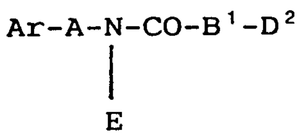

, (VI)

worin Ar, A und E die obige Bedeutung haben, $B^1$ wie in Verbindung mit Formel IV definiert ist und $D^2$ CN, COOR oder CHO darstellt, zum Erhalt einer entsprechenden Verbindung der Formel Ib, worin B $-CH_2B^1-$ bedeutet und D CN, COOR oder CHO ist,
oder
(e) teilweise Hydrolyse einer Verbindung der Formel Ib

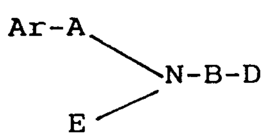

, (Ib)

worin D CN ist und Ar, A, E und B die obige Bedeutung haben, zum Erhalt einer entsprechenden Verbindung der Formel Ia, worin $D^1$ $CONH_2$ ist;
oder
(f) Hydrolyse einer Verbindung der Formel Ib, worin D CN ist oder der Formel Ia, worin $D^1$ $CONH_2$ ist, zum Erhalt einer entsprechenden Verbindung der Formel Ia, worin $D^1$ COOH ist;
oder
(g) Umsetzung einer Verbindung der Formel Ib, worin D COOR ist, worin COOR eine Esterfunktion oder COhal ist, worin hal ein Halogen bedeutet, mit Ammoniak oder einem Amin der Formel $HNR^1R^2$ zum Erhalt einer entsprechenden Verbindung der Formel Ia, worin $D^1$ $-CONR^1R^2$ ist;
oder
(h) Hydrolyse eines Esters der Formel Ib, worin D COOR ist, worin COOR eine Esterfunktion ist, zum Erhalt einer Carbonsäure der Formel Ia, worin $D^1$ COOH ist;
oder
(i) Oxidation eines Aldehyds der Formel Ib, worin D CHO ist, zum Erhalt einer Säure der Formel Ia, worin $D^1$ COOH ist;
oder
(j) Oxidation eines Alkohols der Formel Ib, worin D $-CH_2OH$ ist, zum Erhalt eines Aldehyds der Formel Ib, worin D CHO ist, oder einer Säure der Formel Ia, worin $D^1$ COOH ist;

oder
(k) Oxidation einer Verbindung der Formel

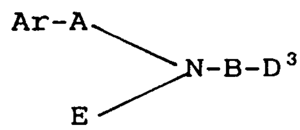

, (VII)

worin Ar, A, E und B die obige Bedeutung haben und $D^3$ -C≡CH, $COCH_3$ oder $-CH=CH_2$ ist, zum Erhalt einer Verbindung der Formel Ia, worin $D^1$ COOH ist;
oder
(l) Hydrolyse einer Verbindung der Formel

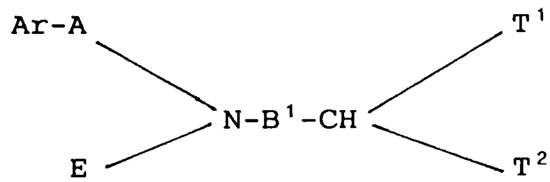

, (VIII)

worin Ar, A und E die obige Bedeutung haben, $T^1$ und $T^2$ jeweils unabhängig voneinander eine Esterfunktion, ein Nitril oder eine Acylgruppe sind und $B^1$ eine Alkylengruppe mit 2 Kohlenstoffatomen ist, gegenenfalls substituiert durch $C_1$-$C_6$-Alkyl, zum Erhalt einer entsprechenden Verbindung der Formel Ia, worin B $-B^1-CH_2-$ ist und $D^1$ COOH ist,
oder
(m) Veresterung einer Säure der Formel Ia, worin $D^1$ COOH ist, oder eines Säurehalogenids der Formel Ib, worin D COhal ist, zum Erhalt eines Esters der Formel Ib, worin D -COOR ist;
oder
(n) Halogenierung einer Säure der Formel Ia zum Erhalt eines Säurehalogenids der Formel Ib, worin $D^1$ COhal ist;
oder
(o) Reduktion einer Säure der Formel Ia, worin $D^1$ COOH ist, zum Erhalt eines Alkohols der Formel Ib, worin D $CH_2OH$ ist;
oder
(p) Ansäuerung einer Verbindung der Formel Ia zum Erhalt eines Säureadditionssalzes derselben oder Neutralisierung eines Säureadditionssalzes zum Erhalt der freien Basenform.

**16.** Verfahren nach Anspruch 15, worin Ar und $Ar^1$, wenn vorhanden, unabhängig voneinander ausgewählt sind aus mono- oder bicyclischen carbocyclischen Arylgruppen mit 6 bis 10 Kohlenstoffatomen und mono- oder bicyclischen heterocyclischen Arylgruppen mit 5 bis 10 Ringatomen, worin das (die) vorhandene(n) Heteroatom(e) ausgewählt ist (sind) aus Stickstoff, Sauerstoff und Schwefel.

**17.** Verfahren nach Anspruch 16, worin Ar und $Ar^1$, wenn vorhanden, ausgewählt sind aus Phenyl, Naphthyl, Pyridinyl, Furanyl, Thienyl, Chinolinyl und Benzofuranyl, welche Gruppen gegebenenfalls substituiert sein können.

**18.** Verfahren nach Anspruch 17, worin der fakultative Substituent ausgewählt ist aus einem oder mehreren von Folgendem: $C_1$-$C_6$-Alkyl, $C_1$-$C_6$-Alkoxy, Halogen, Halogen-$C_1$-$C_6$-Alkyl, Halogen-$C_1$-$C_6$-Alkoxy, Nitro, Cyano und gegebenenfalls substituiertem Amino.

**19.** Verfahren nach einem der Ansprüche 15 bis 18, wobei A und $A^1$, wenn vorhanden, unabhängig voneinander ausgewählt sind aus $-CHR^3-$, worin $R^3$ Wasserstoff, $C_1$-$C_6$-Alkyl oder gegebenenfalls substituiertes Aryl, wie für Ar definiert, ist.

20. Verfahren nach Anspruch 15, worin das Produkt eines von Folgendem ist:
4-[N,N-bis-(4-Chlorbenzyl)-amino]-butyramid,
4-[N,N-bis-(4-Methylbenzyl)-amino]-butyramid,
4-[N,N-bis-(3,4-Dichlorbenzyl)-amino]-buttersäure,
4-[N,N-bis-(4-Methoxybenzyl)-amino]-buttersäure,
4-[N,N-bis-(2-(4-Chlorphenyl)-ethyl)-amino]-buttersäure,
4-[N,N-bis-(3-Brombenzyl)-amino]-butansäure,
4-[N,N-bis-(3-Chlorbenzyl)-amino]-butansäure,
4-[N,N-bis-(2-Thienylmethyl)-amino]-butansäure,
4-[N,N-bis-(1-Naphthalinmethyl)-amino]-butansäure oder,
4-[N-Methyl-N-(p-Chlorbenzyl)-amino]-butansäure,
oder ein pharmazeutisch akzeptables Salz davon.

21. Zwischenprodukt für eine Verbindung der Formel Ia nach Anspruch 15, wobei dieses Zwischenprodukt die Formel Ib

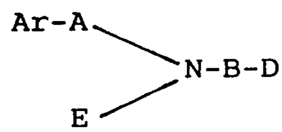

(Ib)

hat, oder ein Salz davon, worin E, B, Ar, Ar[1], A und A[1] wie in Anspruch 15 definiert sind und D für CN, COhal, $CH_2OH$, CHO oder eine Esterfunktion COOR steht, worin R ein organischer Rest ist, mit der Maßgabe, daß
(i) wenn D COOR bedeutet, dann E etwas anderes als $C_1$-$C_6$-Alkyl ist; und
(ii) wenn D COOR bedeutet, dann Ar-A und $Ar^1$-$A^1$ nicht beide p-Nitrobenzyl sind.

22. Verbindung der Formel Ib nach Anspruch 21, welche eines von Folgendem ist:
4-[N,N-bis-(4-Chlorbenzyl)-amino]-butyronitril,
4-[N,N-bis-(4-Methylbenzyl)-amino]-butyronitril,
4-[N,N-bis-(3,4-Dichlorbenzyl)-amino]-butyronitril,
4-[N,N-bis-(4-Methoxybenzyl)-amino]-butyronitril,
4-[N,N-bis-(2-(4-Chlorphenyl)-ethyl)-amino]-butyronitril,
Ethyl-4-[N,N-bis-(4-chlorbenzyl)-amino]-butanoat,
4-[N,N-bis-(4-Chlorbenzyl)-amino]-butanol,
4-[N,N-bis-(3-Brombenzyl)-amino]butyronitril,
4-[N,N-bis-(3-Chlorbenzyl)-amino]-butyronitril,
4-[N,N-bis-(2-Thienylmethyl)-amino]-butyronitril,
4-[N,N-bis-(1-Naphthalinmethyl)-amino]-butyronitril
oder ein pharmazeutisch akzeptables Salz davon.

**Revendications**
**Revendications pour les Etats contractants suivants : AT, BE, CH, DE, FR, IT, LI, LU, NL, SE**

1. Utilisation d'un composé qui agit sélectivement comme agoniste de l'acide gamma-aminobutyrique (GABA) sur les autorécepteurs GABA, dans la préparation d'un médicament pour le traitement de la dépression ou de la démence sénile ou d'autres formes de troubles de la mémoire, à la condition que le composé n'est pas la fengabine ou le progabide.

2. Utilisation suivant la revendication 1, dans laquelle la sélectivité pour l'autorécepteur GABA par rapport au récepteur $GABA_A$ est supérieure ou égale à 100.

3. Utilisation suivant la revendication 1 ou 2, dans laquelle l'agoniste de l'autorécepteur GABA est une amine trisubstituée dans laquelle un substituant est un radical alcoyle de 3 ou 4 atomes de carbone portant un groupe fonctionnel acide carboxylique ou amide.

**4.** Utilisation suivant les revendications 1 à 3, dans laquelle l'agoniste de l'autorécepteur GABA présente la formule :

```
Ar-A
    \
     N-B-D1                (Ia)
    /
   E
```

dans laquelle :

E est alcoyle en $C_{1-6}$ ou $Ar^1$-$A^1$;

Ar et $Ar^1$ sont des radicaux aryle identiques ou différents, facultativement substitués;

A et $A^1$ sont des radicaux alcoylène identiques ou différents, ayant 1 ou 2 atomes de carbone liant Ar ou $Ar^1$ à N, chacun étant facultativement substitué par alcoyle en $C_{1-6}$ ou aryle facultativement substitué;

B est un radical alcoylène de 3 atomes de carbone qui peut être substitué par alcoyle en $C_{1-6}$;

$D^1$ représente -COOH ou -$CONR^1R^2$, où $R^1$ et $R^2$ sont indépendamment, hydrogène, alcoyle en $C_{1-6}$ ou aralcoyle en 7 à 12 atomes de carbone, ou un sel pharmaceutiquement acceptable de celui-ci.

**5.** Utilisation suivant la revendication 4, dans laquelle Ar et $Ar^1$, lorsqu'ils sont présents, sont indépendamment choisis parmi les radicaux aryle carbocycliques mono- ou bicycliques de 6 à 10 atomes de carbone et les radicaux aryle hétérocycliques mono- ou bicycliques de 5 à 10 atomes cycliques dans lesquels le ou les hétéroatomes présents sont choisis parmi azote, oxygène et soufre.

**6.** Utilisation suivant la revendication 5, dans laquelle Ar et $Ar^1$, lorsqu'ils sont présents, sont choisis parmi phényle, naphtyle, pyridinyle, furannyle, thiényle, quinoléinyle et benzofurannyle, ces radicaux étant facultativement substitués.

**7.** Utilisation suivant la revendication 6, dans laquelle le substituant facultatif est choisi parmi un ou plusieurs des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**8.** Utilisation suivant l'une quelconque des revendications 4 à 7, dans laquelle A et $A^1$, lorsqu'ils sont présents, sont indépendamment choisis parmi -$CHR^3$- où $R^3$ est hydrogène, alcoyle en $C_{1-6}$ ou aryle facultativement substitué, comme défini pour Ar.

**9.** Utilisation suivant la revendication 4, dans laquelle le composé de formule (Ia) est choisi parmi l'un des suivants :

l'acide 4-[N,N-bis(4-chlorobenzyl)amino]butyrique;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique;
le 4-[N,N-bis(4-méthoxybenzyl)amino]butyramide;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque;
l'acide 4-[N-méthyl-N-(p-chlorobenayl)amino]butanoïque;
l'acide 4-(N,N-dibenzylamino)butyrique;
l'acide 4-[(N-(4-méthylphénylméthyl)-N-phénylméthyl)amino]butanoïque;
l'acide 4-[(N-(1,1-diphényl)méthyl-N-benzyl)]amino]butyrique;
le 4-[(N-(p-chlorobenzyl)-N-méthyl)amino]butyramide, on
le 4-(N,N-dibenzylamino)-N',N'-diméthylbutanamide ou un sel pharmaceutiquement acceptable de ceux-ci.

**10.** Composition pharmaceutique comprenant un composé de formule (Ia) tel que montré et défini à la revendication 4 ou un sel pharmaceutiquement acceptable de celui-ci, possédant une activité agoniste de l'autorécepteur GABA et un excipient pharmaceutiquement acceptable, avec les conditions :
(i) lorsque E est alcoyle en $C_{1-6}$, A est alcoylène ayant 2 atomes de carbone liant Ar à N et $D^1$ est $-CONR^1R^2$, alors Ar est autre que phényle ou phényle substitué;
(ii) lorsque E est alcoyle en $C_{1-6}$ et $D^1$ est -COOH, alors Ar est autre que 3-pyridyle, et
(iii) lorsque A est méthylène et E est alcoyle en $C_{1-6}$, benzyle ou phényléthyl et $D^1$ est $-CONR^1R^2$, alors Ar est autre que phényle substitué.

**11.** Composition suivant la revendication 10, dans laquelle Ar et $Ar^1$ lorsqu'ils sont présents, sont indépendamment choisis parmi des radicaux aryle carbocycliques mono- ou bicycliques de 6 à 10 atomes de carbone et des radicaux aryle hétérocycliques mono- ou bicycliques de 5 à 10 atomes cycliques, dans lesquels le ou les hétéroatomes présents sont choisis parmi azote, oxygène et soufre.

**12.** Composition suivant la revendication 10, dans laquelle Ar et $Ar^1$, lorsqu'ils sont présents, sont choisis parmi phényle, naphtyle, pyridinyle, furannyle, thiényle, quinoléinyle et benzofurannyle, ces radicaux peuvent être facultativement substitués.

**13.** Composition suivant la revendication 12, dans laquelle le substituant facultatif est choisi parmi un ou plusieurs des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**14.** Composition suivant la revendication 10, dans laquelle le composé de formule (Ia) est choisi parmi l'un des suivants :
l'acide 4-[N,N-bis(4-chlorobenzyl)amino]butyrique;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique;
l'acide 4-[N,N-bis(4-méthoxybenzyl)amino]butyrique;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque, ou
l'acide 4-[N-méthyl-N-(p-chlorobenzyl)amino]butanoïque;
ou un sel pharmaceutiquement acceptable de ceux-ci.

**15.** Composé de formule (Ia) tel que montré et défini à la revendication 4 ou un sel pharmaceutiquement acceptable de celui-ci, possédant une activité agoniste sélective de l'autorécepteur GABA pourvu que :
(i) ni Ar-A, ni E n'est benzyle non substitué;
(ii) lorsque E est alcoyle en $C_{1-6}$, Ar n'est pas phényle, phényle substitué ou un radical hétéroaromatique à 5 ou 6 membres, et
(iii) lorsque E est phénéthyle, Ar n'est pas benzyle substitué.

**16.** Composé suivant la revendication 15, dans lequel Ar et $Ar^1$, lorsqu'ils sont présents, sont indépendamment choisis parmi des radicaux aryle carbocycliques mono- ou bicycliques de 6 à 10 atomes de carbone et des radicaux aryle hétérocycliques mono- ou bicycliques de 5 à 10 atomes cycliques, dans lesquels le ou les hétéroatomes présents sont choisis parmi azote, oxygène et soufre.

**17.** Composé suivant la revendication 16, dans lequel Ar et $Ar^1$, lorsqu'ils sont présents sont choisis parmi phényle, naphtyle, pyridinyle, furannyle, thiényle, quinoléinyle et benzofurannyle, ces radicaux peuvent être facultativement substitués.

**18.** Composé suivant la revendication 17, dans lequel le substituant facultatif est choisi parmi un ou plusieurs des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**19.** Composé suivant l'une quelconque des revendications 15 à 18, dans lequel A et $A^1$, lorsqu'ils sont présents, sont indépendamment choisis parmi $-CHR^3-$ où $R^3$ est hydrogène, alcoyle en $C_{1-6}$ ou aryle facultativement substitué tel que défini pour Ar.

**20.** Composé suivant la revendication 15, dans lequel le composé de formule (Ia) est choisi parmi l'un des suivants :

l'acide 4-[N,N-bis(4-chlorobenzyl)amino]butyrique;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique;
l'acide 4-[N,N-bis(4-méthoxybenzyl)amino]butyrique;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque, ou
l'acide 4-[N-méthyl-N-(p-chlorobenzyl)amino]butanoïque;
ou un sel pharmaceutiquement acceptable de ceux-ci.

**21.** Intermédiaire pour un composé de formule (Ia) suivant la revendication 15, l'intermédiaire ayant la formule (Ib) :

```
Ar-A
    \
     N-B-D                          (Ib)
    /
   E
```

ou un sel de celui-ci, dans lequel E, B, Ar, $Ar^1$, A et $A^1$ sont tels que définis à la revendication 15 et D représente -CN, -COhal, $-CH_2OH$, -CHO ou une fonction ester, -COOR, où R est un radical organique, pourvu que :

(i) lorsque D est -COOR, alors E est autre que alcoyle en $C_{1-6}$, et
(ii) lorsque D est -COOR alors Ar-A et $Ar^1$-$A^1$ ne sont pas tous deux p-nitrobenzyle.

**22.** Composé suivant la revendication 21, qui est l'un des suivants :

le 4-[N,N-bis(4-chlorobenzyl)amino]butyronitrile;
le 4-[N,N-bis(4-méthylbenzyl)amino]butyronitrile;
le 4-[N,N-bis(3,4-dichlorobenzyl)amino]butyronitrile;
le 4-[N,N-bis(4-méthoxybenzyl)amino]butyronitrile;
le 4-[N,N-bis(2-(4-chlorophényl)éthyl)amino]butyronitrile;
le 4-[N,N-bis(4-chlorobenzyl)amino]butanoate d'éthyle;
le 4-[N,N-bis(4-chlorobenzyl)amino]butanol;
le 4-[N,N-bis(3-bromobenzyl)amino]butyronitrile;
le 4-[N,N-bis(3-chlorobenzyl)amino]butyronitrile;
le 4-[N,N-bis(2-thiénylméthyl)amino]butyronitrile, ou
le 4-[N,N-bis(1-naphtalèneméthyl)amino]butyronitrile ou un sel de ceux-ci.

**23.** Procédé de préparation d'un composé de formule (Ia) ou (Ib) tel que défini à la revendication 15 ou la revendication 21, qui comprend l'une des étapes suivantes :

a) réaction d'un composé de formule (II) :

Ar-A-NH-E (II)

dans lequel :

Ar, E et A sont tels que définis ci-dessus, avec un composé de formule (III) :

hal-B-CN (III)

dans lequel :

B est tel que défini ci-dessus et hal représente chlore ou brome, pour donner un composé de formule (Ib) dans lequel D est -CN, ou

b) la réalisation d'une alcoylation réductrice d'un composé de formule (II) tel que défini ci-dessus au moyen d'un composé de formule (IV) :

OHC-$B^1$-CN (IV)

dans lequel :

$B^1$ est une chaîne alcoylène de 2 atomes de carbone, facultativement substituée par alcoyle en $C_{1-6}$, en présence d'un agent réducteur pour donner un composé correspondant de formule (Ib) dans lequel D est -CN et B représente -$CH_2B^1$-, ou

c) la réduction d'un composé de formule (V) :

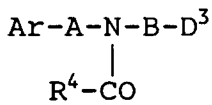

(V)

dans lequel :

$R^4$ est alcoyle en 1 à 5 atomes de carbone ou $Ar^1$-$A^2$-;

Ar, $Ar^1$, A et B sont tels que définis ci-dessus,

$D^3$ représente -CN, -COOR ou -COOH, et

$A^2$ représente un lien direct ou un alcoylène de 1 atome de carbone, facultativement substitué par alcoyle en $C_{1-6}$ et/ou aryle, pour donner un composé correspondant de formule (Ib), dans lequel D est -CN ou -COOR ou de formule (Ia), dans lequel $D^1$ est -COOH et E est $R^4CH_2$, où $R^4$ est tel que défini ci-dessus, ou

d) la réduction d'un composé de formule (VI) :

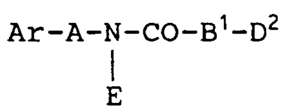

(VI)

dans lequel :

Ar, A et E sont tels que définis ci-dessus;

$B^1$ est tel que défini par rapport à la formule (IV), et

$D^2$ représente -CN, -COOR ou -CHO, pour donner un composé correspondant de formule (Ib) dans lequel B est -$CH_2B^1$- et D est -CN, -COOR ou -CHO, ou

e) l'hydrolyse partielle d'un composé de formule (Ib) :

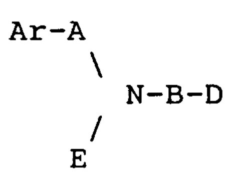

(Ib)

dans lequel D est -CN et Ar, A, E et B sont tels que définis ci-dessus, pour donner un composé correspondant de formule (Ia) dans lequel $D^1$ est -$CONH_2$, ou

f) l'hydrolyse d'un composé de formule (Ib), dans lequel D est -CN ou de formule (Ia), dans lequel $D^1$ est -$CONH_2$, pour donner un composé correspondant de formule (Ia) dans lequel $D^1$ est -COOH, ou

g) la réaction d'un composé de formule (Ib) dans lequel D est -COOR où -COOR est une fonction ester, ou -COhal où hal est un halogène, avec de l'ammoniac ou une amine de formule $HNR^1R^2$, pour donner un composé correspondant de formule (Ia) dans lequel $D^1$ est $-CONR^1R^2$, ou

h) l'hydrolyse d'un ester de formule (Ib), dans lequel D est -COOR où -COOR est une fonction ester, pour donner un acide carboxylique de formule (Ia) dans lequel $D^1$ est -COOH, ou

i) l'oxydation d'un aldéhyde de formule (Ib), dans lequel D est -CHO, pour donner un acide de formule (Ia), où $D^1$ est -COOH, ou

j) l'oxydation d'un alcool de formule (Ib), dans lequel D est $-CH_2OH$, pour donner un aldéhyde de formule (Ib), dans lequel D est -CHO ou un acide de formule (Ia), dans lequel $D^1$ est -COOH, ou

k) l'oxydation d'un composé de formule (VII) :

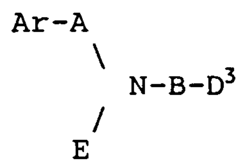

(VII)

dans lequel :

Ar, A, E et B sont tels que définis ci-dessus, et

$D^3$ est -C≡CH; $-COCH_3$ ou $-CH=CH_2$, pour donner un composé de formule (Ia), dans lequel $D^1$ est -COOH, ou

(l) l'hydrolyse d'un composé de formule (VIII) :

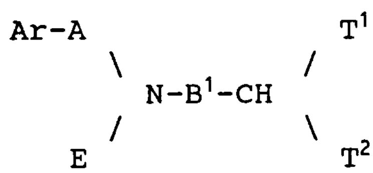

(VIII)

dans lequel :

Ar, A et E sont tels que définis ci-dessus;

$T^1$ et $T^2$ sont chacun, indépendamment, une fonction ester, un nitrile ou un radical acyle, et

$B^1$ est un radical alcoylène de 2 atomes de carbone, facultativement substitué par alcoyle en $C_{1-6}$, pour donner un composé correspondant de formule (Ia), dans lequel B est $-B^1-CH_2$ et $D^1$ est -COOH, ou

m) l'estérification d'un acide de formule (Ia), dans lequel $D^1$ est -COOH ou d'un halogénure d'acide de formule (Ib), dans lequel D est -COhal pour donner un ester de formule (Ib), dans lequel D est -COOR, ou

n) l'halogénation d'un acide de formule (Ia) pour donner un halogénure d'acide de formule (Ib), dans lequel D est -COhal, ou

o) la réduction d'un acide de formule (Ia) dans lequel $D^1$ est -COOH, pour donner un alcool de formule (Ib), dans lequel D est $-CH_2OH$, ou

p) l'acidification d'un composé de formule (Ia), pour donner un sel d'addition d'acide de celui-ci ou la neutralisation d'un sel d'addition d'acide pour donner la forme base libre.

## Revendications pour l'Etat contractant suivant : ES

1. Utilisation d'un composé qui agit sélectivement comme agoniste de l'acide gamma-aminobutyrique (GABA) sur les autorécepteurs GABA, dans la préparation d'un médicament pour le traitement de la dépression ou de la démence sénile ou d'autres formes de troubles de la mémoire, à la condition que le composé n'est pas la fengabine ou le progabide.

2. Utilisation suivant la revendication 1, dans laquelle la sélectivité pour l'autorécepteur GABA par rapport au récepteur $GABA_A$ est supérieure ou égale à 100.

**3.** Utilisation suivant la revendication 1 ou 2, dans laquelle l'agoniste de l'autorécepteur GABA est une amine trisubstituée dans laquelle un substituant est un radical alcoyle de 3 ou 4 atomes de carbone portant un groupe fonctionnel acide carboxylique ou amide.

**4.** Utilisation suivant les revendications 1 à 3, dans laquelle l'agoniste de l'autorécepteur GABA présente la formule :

Ar-A<br>
\ <br>
N-B-$D^1$ (Ia)<br>
/<br>
E

dans laquelle :

E est alcoyle en $C_{1-6}$ ou $Ar^1$-$A^1$;

Ar et $Ar^1$ sont des radicaux aryle identiques ou différents, facultativement substitués;

A et $A^1$ sont des radicaux alcoylène identiques ou différents, ayant 1 ou 2 atomes de carbone liant Ar ou $Ar^1$ à N, chacun étant facultativement substitué par alcoyle en $C_{1-6}$ ou aryle facultativement substitué;

B est un radical alcoylène de 3 atomes de carbone qui peut être substitué par alcoyle en $C_{1-6}$;

$D^1$ représente -COOH ou -$CONR^1R^2$, où $R^1$ et $R^2$ sont indépendamment, hydrogène, alcoyle en $C_{1-6}$ ou aralcoyle en 7 à 12 atomes de carbone, ou un sel pharmaceutiquement acceptable de celui-ci.

**5.** Utilisation suivant la revendication 4, dans laquelle Ar et $Ar^1$, lorsqu'ils sont présents, sont indépendamment choisis parmi les radicaux aryle carbocycliques mono- ou bicycliques de 6 à 10 atomes de carbone et les radicaux aryle hétérocycliques mono- ou bicycliques de 5 à 10 atomes cycliques dans lesquels le ou les hétéroatomes présents sont choisis parmi azote, oxygène et soufre.

**6.** Utilisation suivant la revendication 5, dans laquelle Ar et $Ar^1$, lorsqu'ils sont présents, sont choisis parmi phényle, naphtyle, pyridinyle, furannyle, thiényle, quinoléinyle et benzofurannyle, ces radicaux étant facultativement substitués.

**7.** Utilisation suivant la revendication 6, dans laquelle le substituant facultatif est choisi parmi un ou plusieurs des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**8.** Utilisation suivant l'une quelconque des revendications 4 à 7, dans laquelle A et $A^1$, lorsqu'ils sont présents, sont indépendamment choisis parmi -$CHR^3$- où $R^3$ est hydrogène, alcoyle en $C_{1-6}$ ou aryle facultativement substitué, comme défini pour Ar.

**9.** Utilisation suivant la revendication 4, dans laquelle le composé de formule (Ia) est choisi parmi l'un des suivants :

l'acide 4-[N,N-bis(4-chlorobenzyl)amino]butyrique;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique;
le 4-[N,N-bis(4-méthoxybenzyl)amino]butyramide;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque;
l'acide 4-[N-méthyl-N-(p-chlorobenzyl)amino]butanoïque;
l'acide 4-(N,N-dibenzylamino)butyrique;
l'acide 4-[(N-(4-méthylphénylméthyl)-N-phénylméthyl)amino]butanoïque;
l'acide 4-[(N-(1,1-diphényl)méthyl-N-benzyl)]amino]butyrique;

le 4-[(N-(p-chlorobenzyl)-N-méthyl)amino]butyramide, ou
le 4-(N,N-dibenzylamino)-N',N'-diméthylbutanamide ou un sel pharmaceutiquement acceptable de ceux-ci.

**10.** Procédé de préparation d'une composition pharmaceutique qui comprend la mise en commun d'un composé de formule (Ia) tel que présenté et défini à la revendication 4 ou d'un sel pharmaceutiquement acceptable de celui-ci, possédant une activité agoniste sélective de l'autorécepteur GABA, en association avec un excipient pharmaceutiquement acceptable, avec les conditions :
(i) lorsque E est alcoyle en $C_{1-6}$, A est alcoylène ayant 2 atomes de carbone liant Ar à N et $D^1$ est $-CONR^1R^2$, alors Ar est autre que phényle ou phényle substitué;
(ii) lorsque E est alcoyle en $C_{1-6}$ et $D^1$ est -COOH, alors Ar est autre que 3-pyridyle, et
(iii) lorsque A est méthylène et E est alcoyle en $C_{1-6}$, benzyle ou phényléthyle et $D^1$ est $-CONR^1R^2$, alors Ar est autre que phényle substitué.

**11.** Procédé suivant la revendication 10, dans lequel Ar et $Ar^1$ lorsqu'ils sont présents, sont indépendamment choisis parmi des radicaux aryle carbocycliques mono- ou bicycliques de 6 à 10 atomes de carbone et des radicaux aryle hétérocycliques mono- ou bicycliques de 5 à 10 atomes cycliques, dans lesquels le ou les hétéroatomes présents sont choisis parmi azote, oxygène et soufre.

**12.** Procédé suivant la revendication 10, dans lequel Ar et $Ar^1$, lorsqu'ils sont présents, sont choisis parmi phényle, naphtyle, pyridinyle, furannyle, thiényle, quinoléinyle et benzofurannyle, ces radicaux peuvent être facultativement substitués.

**13.** Procédé suivant la revendication 12, dans lequel le substituant facultatif est choisi parmi l'un des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**14.** Procédé suivant la revendication 10, dans lequel le composé de formule (Ia) est choisi parmi l'un des suivants :
l'acide 4-[N,N-bis(4-chlorobenzyl)amino]butyrique;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique;
l'acide 4-[N,N-bis(4-méthoxybenzyl)amino]butyrique;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque, ou
l'acide 4-[N-méthyl-N-(p-chlorobenzyl)amino]butanoïque; ou un sel pharmaceutiquement acceptable de ceux-
ci.

**15.** Procédé de préparation d'un composé de formule (Ia) ou (Ib) :

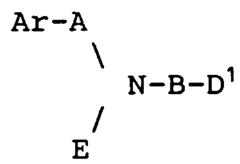

(Ia)

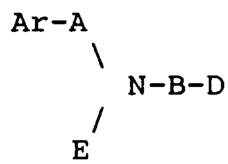

(Ib)

dans lequel E, B, Ar, Ar[1], A, D[1] et A[1] sont tels que définis à la revendication 4 et D représente -CN, -COhal, $-CH_2OH$, -CHO ou une fonction ester, -COOR, où R est un radical organique, pourvu que :

(i) lorsque D est -COOR, alors E est autre que alcoyle en $C_{1-6}$;

(ii) lorsque D est -COOR, alors Ar-A et Ar[1]-A[1] ne sont pas tous deux p-nitrobenzyle;

(iii) ni Ar-A, ni E n'est benzyle non substitué;

(iv) lorsque E est alcoyle en $C_{1-6}$, Ar n'est pas phényle, phényle substitué ou un radical hétéroaromatique à 5 ou 6 membres, et

(v) lorsque E est phénéthyle, Ar n'est pas benzyle substitué, qui comprend l'une des étapes suivantes :

a) réaction d'un composé de formule (II) :

Ar-A-NH-E (II)

dans lequel :

Ar, E et A sont tels que définis ci-dessus, avec un composé de formule (III) :

hal-B-CN (III)

dans lequel :

B est tel que défini ci-dessus et hal représente chlore ou brome, pour donner un composé de formule (Ib) dans lequel D est -CN, ou

b) la réalisation d'une alcoylation réductrice d'un composé de formule (II) tel que défini ci-dessus au moyen d'un composé de formule (IV) :

OHC-$B^1$-CN (IV)

dans lequel :

$B^1$ est une chaîne alcoylène de 2 atomes de carbone, facultativement substitués par alcoyle en $C_{1-6}$, en présence d'un agent réducteur pour donner un compose correspondant de formule (Ib) dans lequel D est -CN et B représente $-CH_2B^1-$, ou

c) la réduction d'un composé de formule (V) :

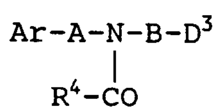

(V)

dans lequel :

$R^4$ est alcoyle en 1 à 5 atomes de carbone ou $Ar^1$-$A^2$-;

Ar, Ar[1], A et B sont tels que définis ci-dessus,

$D^3$ représente -CN, -COOR ou -COOH, et

$A^2$ représente un lien direct ou un alcoylène de 1 atome de carbone, facultativement substitué par alcoyle en $C_{1-6}$ et/ou aryle, pour donner un composé correspondant de formule (Ib), dans lequel D est -CN ou -COOR ou de formule (Ia), dans lequel $D^1$ est -COOH et E est $R^4CH_2$, où $R^4$ est tel que défini ci-dessus, ou

d) la réduction d'un composé de formule (VI) :

```
Ar-A-N-CO-B¹-D²
     |
     E
```
(VI)

dans lequel :

Ar, A et E sont tels que définis ci-dessus;

$B^1$ est tel que défini par rapport à la formule (IV), et

$D^2$ représente -CN, -COOR ou -CHO, pour donner un composé correspondant de formule (Ib) dans lequel B est $-CH_2B^1-$ et D est -CN, -COOR ou -CHO, ou

e) l'hydrolyse partielle d'un composé de formule (Ib) :

```
Ar-A
    \
     N-B-D
    /
   E
```
(Ib)

dans lequel D est -CN et Ar, A, E et B sont tels que définis ci-dessus, pour donner un composé correspondant de formule (Ia) dans lequel $D^1$ est $-CONH_2$, ou

f) l'hydrolyse d'un composé de formule (Ib), dans lequel D est -CN ou de formule (Ia), dans lequel $D^1$ est $-CONH_2$, pour donner un composé correspondant de formule (Ia) dans lequel $D^1$ est -COOH, ou

g) la réaction d'un composé de formule (Ib) dans lequel D est -COOR où -COOR est une fonction ester, ou -COhal où hal est un halogène, avec de l'ammoniac ou une amine de formule $HNR^1R^2$, pour donner un composé correspondant de formule (Ia) dans lequel $D^1$ est $-CONR^1R^2$, ou

h) l'hydrolyse d'un ester de formule (Ib), dans lequel D est -COOR, pour donner un acide carboxylique de formule (Ia) dans lequel $D^1$ est -COOH, ou

i) l'oxydation d'un aldéhyde de formule (Ib), dans lequel D est -CHO, pour donner un acide de formule (Ia), où $D^1$ est -COOH, ou

j) l'oxydation d'un alcool de formule (Ib), dans lequel D est $-CH_2OH$, pour donner un aldéhyde de formule (Ib), dans lequel D est -CHO ou un acide de formule (Ia), dans lequel $D^1$ est -COOH, ou

k) l'oxydation d'un composé de formule (VII) :

```
Ar-A
    \
     N-B-D³
    /
   E
```
(VII)

dans lequel :

Ar, A, E et B sont tels que définis ci-dessus, et

$D^3$ est $-C\equiv CH$, $-COCH_3$ ou $-CH=CH_2$, pour donner un composé de formule (Ia), dans lequel $D^1$ est -COOH, ou

(l) l'hydrolyse d'un composé de formule (VIII) :

```
Ar-A              T1
    \            /
     N-B1-CH                    (VIII)
    /            \
   E              T2
```

dans lequel :

Ar, A et E sont tels que définis ci-dessus;

$T^1$ et $T^2$ sont chacun, indépendamment, une fonction ester, un nitrile ou un radical acyle, et

$B^1$ est un radical alcoylène de 2 atomes de carbone, facultativement substitué par alcoyle en $C_{1-6}$, pour donner un composé correspondant de formule (Ia), dans lequel B est $-B^1-CH_2$ et $D^1$ est -COOH, ou

m) l'estérification d'un acide de formule (Ia), dans lequel $D^1$ est -COOH ou d'un halogénure d'acide de formule (Ib), dans lequel D est -COhal pour donner un ester de formule (Ib), dans lequel D est -COOR, ou

n) l'halogénation d'un acide de formule (Ia) pour donner un halogénure d'acide de formule (Ib), dans lequel D est -COhal, ou

o) la réduction d'un acide de formule (Ia) dans lequel $D^1$ est -COOH, pour donner un alcool de formule (Ib), dans lequel D est $-CH_2OH$, ou

p) l'acidification d'un composé de formule (Ia), pour donner un sel d'addition d'acide de celui-ci ou la neutralisation d'un sel d'addition d'acide pour donner la forme base libre.

**16.** Procédé suivant la revendication 15, dans lequel Ar et $Ar^1$, lorsqu'ils sont présents, sont indépendamment choisis parmi les radicaux aryle carbocycliques mono- ou bicycliques de 6 à 10 atomes de carbone et les radicaux aryle hétérocycliques mono- ou bicycliques de 5 à 10 atomes cycliques, dans lesquels le ou les hétéroatomes présents sont choisis parmi azote, oxygène et soufre.

**17.** Procédé suivant la revendication 16, dans lequel Ar et $Ar^1$, lorsqu'ils sont présents sont choisis parmi phényle, naphtyle, pyridinyle, furannyle, thiényle, quinoléinyle et benzofurannyle, ces radicaux peuvent être facultativement substitués.

**18.** Procédé suivant la revendication 17, dans lequel le substituant facultatif est choisi parmi l'un des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**19.** Procédé suivant l'une quelconque des revendications 15 à 18, dans lequel A et $A^1$, lorsqu'ils sont présents sont indépendamment choisis parmi $-CHR^3-$ où $R^3$ est hydrogène, alcoyle en $C_{1-6}$ ou aryle facultativement substitué tel que défini pour Ar.

**20.** Procédé suivant la revendication 15, dans lequel le produit est l'un des suivants :

le 4-[N,N-bis(4-chlorobenzyl)amino]butyronitrile;
le 4-[N,N-bis(4-méthylbenzyl)amino]butyronitrile;
le 4-[N,N-bis(3,4-dichlorobenzyl)amino]butyronitrile;
le 4-[N,N-bis(4-méthoxybenzyl)amino]butyronitrile;
le 4-[N,N-bis(2-(4-chlorophényl)éthyl)amino]butyronitrile;
le 4-[N,N-bis(4-chlorobenzyl)amino]butanoate d'éthyle;
le 4-[N,N-bis(4-bromobenzyl)amino]butanol;
le 4-[N,N-bis(3-bromobenzyl)amino]butyronitrile;
le 4-[N,N-bis(3-chlorobenzyl)amino]butyronitrile;
le 4-[N,N-bis(2-thiénylméthyl)amino]butyronitrile;
le 4-[N,N-bis(1-naphtalèneméthyl)amino]butyronitrile;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique ;

l'acide 4-[N,N-bis(4-méthoxybenzyl)amino]butyrique;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque, ou
l'acide 4-[N-méthyl-N-(p-chlorobenzyl)amino]butanoïque ou un sel pharmaceutiquement acceptable de celui-ci.

**Revendications pour l'Etat contractant suivant : GR**

**1.** Utilisation d'un composé qui agit sélectivement comme agoniste de l'acide gamma-aminobutyrique (GABA) sur les autorécepteurs GABA, dans la préparation d'un médicament pour le traitement de la dépression ou de la démence sénile ou d'autres formes de troubles de la mémoire, à la condition que le composé n'est pas la fengabine ou le progabide.

**2.** Utilisation suivant la revendication 1, dans laquelle la sélectivité pour l'autorécepteur GABA par rapport au récepteur $GABA_A$ est supérieure ou égale à 100.

**3.** Utilisation suivant la revendication 1 ou 2, dans laquelle l'agoniste de l'autorécepteur GABA est une amine trisubstituée dans laquelle un substituant est un radical alcoyle de 3 ou 4 atomes de carbone portant un groupe fonctionnel acide carboxylique ou amide.

**4.** Utilisation suivant les revendications 1 à 3, dans laquelle l'agoniste de l'autorécepteur GABA présente la formule :

```
Ar-A
    \
     N-B-D¹                    (Ia)
    /
  E
```

dans laquelle :

E est alcoyle en $C_{1-6}$ ou $Ar^1$-$A^1$;

Ar et $Ar^1$ sont des radicaux aryle identiques ou différents, facultativement substitués;

A et $A^1$ sont des radicaux alcoylène identiques ou différents, ayant 1 ou 2 atomes de carbone liant Ar ou $Ar^1$ à N, chacun étant facultativement substitué par alcoyle en $C_{1-6}$ ou aryle facultativement substitué;

B est un radical alcoylène de 3 atomes de carbone qui peut être substitué par alcoyle en $C_{1-6}$;

$D^1$ représente -COOH ou -$CONR^1R^2$, où $R^1$ et $R^2$ sont indépendamment, hydrogène, alcoyle en $C_{1-6}$ ou aralcoyle en 7 à 12 atomes de carbone, ou un sel pharmaceutiquement acceptable de celui-ci.

**5.** Utilisation suivant la revendication 4, dans laquelle Ar et $Ar^1$, lorsqu'ils sont présents, sont indépendamment choisis parmi les radicaux aryle carbocycliques mono- ou bicycliques de 6 à 10 atomes de carbone et les radicaux aryle hétérocycliques mono- ou bicycliques de 5 à 10 atomes cycliques dans lesquels le ou les hétéroatomes présents sont choisis parmi azote, oxygène et soufre.

**6.** Utilisation suivant la revendication 5, dans laquelle Ar et $Ar^1$, lorsqu'ils sont présents, sont choisis parmi phényle, naphtyle, pyridinyle, furannyle, thiényle, quinoléinyle et benzofurannyle, ces radicaux étant facultativement substitués.

**7.** Utilisation suivant la revendication 6, dans laquelle le substituant facultatif est choisi parmi un ou plusieurs des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitue.

**8.** Utilisation suivant l'une quelconque des revendications 4 à 7, dans laquelle A et $A^1$, lorsqu'ils sont présents, sont indépendamment choisis parmi $-CHR^3-$ où $R^3$ est hydrogène, alcoyle en $C_{1-6}$ ou aryle facultativement substitué, comme défini pour Ar.

**9.** Utilisation suivant la revendication 4, dans laquelle le composé de formule (Ia) est choisi parmi l'un des suivants :

l'acide 4-[N,N-bis(4-chlorobenzyl)amino]butyrique;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique;
le 4-[N,N-bis(4-méthoxybenzyl)amino]butyramide;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque;
l'acide 4-[N-méthyl-N-(p-chlorobenzyl)amino]butanoïque;
l'acide 4-(N,N-dibenzylamino)butyrique;
l'acide 4-[(N-(4-méthylphénylméthyl)-N-phénylméthyl)amino]butanoïque;
l'acide 4-[(N-(1,1-diphényl)méthyl-N-benzyl)]amino]butyrique;
le 4-[(N-(p-chlorobenzyl)-N-méthyl)amino]butyramide, ou
le 4-(N,N-dibenzylamino)-N',N'-diméthylbutanamide ou un sel pharmaceutiquement acceptable de ceux-ci.

**10.** Procédé de préparation d'une composition pharmaceutique qui comprend la mise en commun d'un compose de formule (Ia) tel que présenté et défini à la revendication 4 ou d'un sel pharmaceutiquement acceptable de celui-ci, possédant une activité agoniste sélective de l'autorécepteur GABA, en association avec un excipient pharmaceutiquement acceptable, avec les conditions :

(i) lorsque E est alcoyle en $C_{1-6}$, A est alcoylène ayant 2 atomes de carbone liant Ar à N et $D^1$ est $-CONR^1R^2$, alors Ar est autre que phényle ou phényle substitué;
(ii) lorsque E est alcoyle en $C_{1-6}$ et $D^1$ est -COOH, alors Ar est autre que 3-pyridyle, et
(iii) lorsque A est méthylène et E est alcoyle en $C_{1-6}$, benzyle ou phényléthyl et $D^1$ est $-CONR^1R^2$, alors Ar est autre que phényle substitué.

**11.** Procédé suivant la revendication 10, dans lequel Ar et $Ar^1$ lorsqu'ils sont présents, sont indépendamment choisis parmi des radicaux aryle carbocycliques mono- ou bicycliques de 6 à 10 atomes de carbone et des radicaux aryle hétérocycliques mono- ou bicycliques de 5 à 10 atomes cycliques, dans lesquels le ou les hétéroatomes présents sont choisis parmi azote, oxygène et soufre.

**12.** Procédé suivant la revendication 10, dans lequel Ar et $Ar^1$, lorsqu'ils sont présents, sont choisis parmi phényle, naphtyle, pyridinyle, furannyle, thiényle, quinoléinyle et benzofurannyle, ces radicaux peuvent être facultativement substitués.

**13.** Procédé suivant la revendication 12, dans lequel le substituant facultatif est choisi parmi l'un des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**14.** Procédé suivant la revendication 10, dans lequel le composé de formule (Ia) est choisi parmi l'un des suivants :

l'acide 4-[N,N-bis(4-chlorobenzyl)amino]butyrique;
le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)amino]butyrique;
l'acide 4-[N,N-bis(4-méthoxybenzyl)amino]butyrique;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;

l'acide 4-[N,N-bis(1-naphtalèneméthyl)amino]butanoïque, ou
l'acide 4-[N-méthyl-N-(p-chlorobenzyl)amino]butanoïque;
ou un sel pharmaceutiquement acceptable de ceux-ci.

**15.** Procédé de préparation d'un composé de formule (Ia) ou (Ib) :

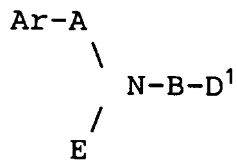

(Ia)

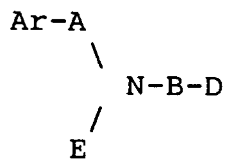

(Ib)

dans lequel E, B, Ar, $Ar^1$, A, $D^1$ et $A^1$ sont tels que définis à la revendication 4 et D représente -CN, -COhal, $-CH_2OH$, -CHO ou une fonction ester, -COOR, où R est un radical organique, pourvu que :

(i) lorsque D est -COOR, alors E est autre que alcoyle en $C_{1-6}$;

(ii) lorsque D est -COOR, alors Ar-A et $Ar^1$-$A^1$ ne sont pas tous deux p-nitrobenzyle;

(iii) ni Ar-A, ni E n'est benzyle non substitué;

(iv) lorsque E est alcoyle en $C_{1-6}$, Ar n'est pas phényle, phényle substitué ou un radical hétéroaromatique à 5 ou 6 membres, et

(v) lorsque E est phénéthyle, Ar n'est pas benzyle substitué, qui comprend l'une des étapes suivantes :

a) réaction d'un composé de formule (II) :

Ar-A-NH-E (II)

dans lequel :

Ar, E et A sont tels que définis ci-dessus, avec un composé de formule (III) :

hal-B-CN (III)

dans lequel :

B est tel que défini ci-dessus et hal représente chlore ou brome, pour donner un composé de formule (Ib) dans lequel D est CN, ou

b) la réalisation d'une alcoylation réductrice d'un composé de formule (II) tel que défini ci-dessus au moyen d'un composé de formule (IV) :

OHC-$B^1$-CN (IV)

dans lequel :

$B^1$ est une chaîne alcoylène de 2 atomes de carbone, facultativement substitués par alcoyle en $C_{1-6}$, en présence d'un agent réducteur pour donner un composé correspondant de formule (Ib) dans lequel D est -CN et B représente $-CH_2B^1-$, ou

c) la réduction d'un composé de formule (V) :

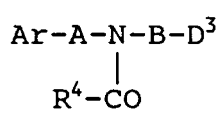

(V)

dans lequel :

$R^4$ est alcoyle en 1 à 5 atomes de carbone ou $Ar^1$-$A^2$-;

Ar, $Ar^1$, A et B sont tels que définis ci-dessus,

$D^3$ représente -CN, -COOR ou -COOH, et

$A^2$ représente un lien direct ou un alcoylène de 1 atome de carbone, facultativement substitué par alcoyle en $C_{1-6}$ et/ou aryle, pour donner un composé correspondant de formule (Ib), dans lequel D est -CN ou -COOR ou de formule (Ia), dans lequel $D^1$ est -COOH et E est $R^4CH_2$, où $R^4$ est tel que défini ci-dessus, ou

d) la réduction d'un composé de formule (VI) :

```
Ar-A-N-CO-B¹-D²
     |                      (VI)
     E
```

dans lequel :

Ar, A et E sont tels que définis ci-dessus;

$B^1$ est tel que défini par rapport à la formule (IV), et

$D^2$ représente -CN, -COOR ou -CHO, pour donner un composé correspondant de formule (Ib) dans lequel B est $-CH_2B^1$- et D est -CN, -COOR ou -CHO, ou

e) l'hydrolyse partielle d'un composé de formule (Ib) :

```
Ar-A
    \
     N-B-D                 (Ib)
    /
   E
```

dans lequel D est -CN et Ar, A, E et B sont tels que définis ci-dessus, pour donner un composé correspondant de formule (Ia) dans lequel $D^1$ est $-CONH_2$, ou

f) l'hydrolyse d'un composé de formule (Ib), dans lequel D est -CN ou de formule (Ia), dans lequel $D^1$ est $CONH_2$, pour donner un composé correspondant de formule (Ia) dans lequel $D^1$ est -COOH, ou

g) la réaction d'un composé de formule (Ib) dans lequel D est -COOR où -COOR est une fonction ester, ou -COhal où hal est un halogène, avec de l'ammoniac ou une amine de formule $HNR^1R^2$, pour donner un composé correspondant de formule (Ia) dans lequel $D^1$ est $-CONR^1R^2$, ou

h) l'hydrolyse d'un ester de formule (Ib), dans lequel D est -COOR, pour donner un acide carboxylique de formule (Ia) dans lequel $D^1$ est -COOH, ou

i) l'oxydation d'un aldéhyde de formule (Ib), dans lequel D est -CHO, pour donner un acide de formule (Ia), où $D^1$ est -COOH, ou

j) l'oxydation d'un alcool de formule (Ib), dans lequel D est $-CH_2OH$, pour donner un aldéhyde de formule (Ib), dans lequel D est -CHO ou un acide de formule (Ia), dans lequel $D^1$ est -COOH, ou

k) l'oxydation d'un composé de formule (VII) :

```
Ar-A
    \
     N-B-D³               (VII)
    /
   E
```

dans lequel :

Ar, A, E et B sont tels que définis ci-dessus, et

$D^3$ est -C≡CH; $-COCH_3$ ou $-CH=CH_2$, pour donner un composé de formule (Ia), dans lequel

$D^1$ est -COOH, ou

(l) l'hydrolyse d'un composé de formule (VIII) :

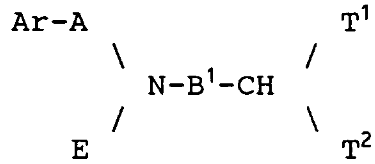

(VIII)

dans lequel :

Ar, A et E sont tels que définis ci-dessus;

$T^1$ et $T^2$ sont chacun, indépendamment, une fonction ester, un nitrile ou un radical acyle, et

$B^1$ est un radical alcoylène de 2 atomes de carbone, facultativement substitué par alcoyle en $C_{1-6}$, pour donner un composé correspondant de formule (Ia), dans lequel B est -$B^1$-$CH_2$ et $D^1$ est -COOH, ou

m) l'estérification d'un acide de formule (Ia), dans lequel $D^1$ est -COOH ou d'un halogénure d'acide de formule (Ib), dans lequel D est -COhal pour donner un ester de formule (Ib), dans lequel D est -COOR, ou

n) l'halogénation d'un acide de formule (Ia) pour donner un halogénure d'acide de formule (Ib), dans lequel D est -COhal, ou

o) la réduction d'un acide de formule (Ia) dans lequel $D^1$ est -COOH, pour donner un alcool de formule (Ib), dans lequel D est -$CH_2OH$, ou

p) l'acidification d'un composé de formule (Ia), pour donner un sel d'addition d'acide de celui-ci ou la neutralisation d'un sel d'addition d'acide pour donner la forme base libre.

**16.** Procédé suivant la revendication 15, dans lequel Ar et $Ar^1$, lorsqu'ils sont présents, sont indépendamment choisis parmi les radicaux aryle carbocycliques mono- ou bicycliques de 6 à 10 atomes de carbone et les radicaux aryle hétérocycliques mono- ou bicycliques de 5 à 10 atomes cycliques, dans lesquels le ou les hétéroatomes présents sont choisis parmi azote, oxygène et soufre.

**17.** Procédé suivant la revendication 16, dans lequel Ar et $Ar^1$, lorsqu'ils sont présents sont choisis parmi phényle, naphtyle, pyridinyle, furannyle, thiényle, quinoléinyle et benzofurannyle, ces radicaux peuvent être facultativement substitués.

**18.** Procédé suivant la revendication 17, dans lequel le substituant facultatif est choisi parmi l'un des radicaux suivants : alcoyle en $C_{1-6}$, alcoxy en $C_{1-6}$, halogène, haloalcoyle en $C_{1-6}$, haloalcoxy en $C_{1-6}$, nitro, cyano et amino facultativement substitué.

**19.** Procédé suivant l'une quelconque des revendications 15 à 18, dans lequel A et $A^1$, lorsqu'ils sont présents sont indépendamment choisis parmi -$CHR^3$- où $R^3$ est hydrogène, alcoyle en $C_{1-6}$ ou aryle facultativement substitué tel que défini pour Ar.

**20.** Procédé suivant la revendication 15, dans lequel le produit est l'un des suivants :

le 4-[N,N-bis(4-chlorobenzyl)amino]butyramide;
le 4-[N,N-bis(4-méthylbenzyl)amino]butyramide;
l'acide 4-[N,N-bis(3,4-dichlorobenzyl)]aminobutyrique;
l'acide 4-[N,N-bis(4-méthoxybenzyl)amino]butyrique;
l'acide 4-[N,N-bis(2-(4-chlorophényl)éthyl)amino]butyrique;
l'acide 4-[N,N-bis(3-bromobenzyl)amino]butanoïque;
l'acide 4-[N,N-bis(3-chlorobenzyl)amino]butanoïque;
l'acide 4-[N,N-bis(2-thiénylméthyl)amino]butanoïque;
l'acide 4-[N,N-bis(1-naphtalèneméthyl)amino]butanoïque, ou
l'acide 4-[N-méthyl-N-(p-chlorobenzyl)amino]butanoïque, ou
un sel pharmaceutiquement acceptable de ceux-ci.

**21.** Intermédiaire pour un composé de formule (Ia) suivant la revendication 15, l'intermédiaire ayant la formule (Ib) :

```
Ar-A
    \
     N-B-D                (Ib)
    /
   E
```

ou un sel de celui-ci, dans lequel E, B, Ar, $Ar^1$, A et $A^1$ sont tels que définis à la revendication 15 et D représente -CN, -COhal, $-CH_2OH$, -CHO ou une fonction ester -COOR, où R est un radical organique pourvu que :

(i) lorsque D est -COOR, alors E est autre que alcoyle en $C_{1-6}$, et

(ii) lorsque D est -COOR, alors Ar-A et $Ar^1$-$A^1$ ne sont pas tous deux p-nitrobenzyle.

**22.** Composé de formule (Ib) suivant la revendication 21, qui est l'un des suivants :

le 4-[N,N-bis(4-chlorobenzyl)amino]butyronitrile;
le 4-[N,N-bis(4-méthylbenzyl)amino]butyronitrile;
le 4-[N,N-bis(3,4-dichlorobenzyl)amino]butyronitrile;
le 4-[N,N-bis(4-méthoxybenzyl)amino]butyronitrile;
le 4-[N,N-bis(2-(4-chlorophényl)éthyl)amino]butyronitrile;
le 4-[N,N-bis(4-chlorobenzyl)amino]butanoate d'éthyle;
le 4-[N,N-bis(4-chlorobenzyl)amino]butanol;
le 4-[N,N-bis(3-bromobenzyl)amino]butyronitrile;
le 4-[N,N-bis(3-chlorobenzyl)amino]butyronitrile;
le 4-[N,N-bis(2-thiénylméthyl)amino]butyronitrile,
le 4-[N,N-bis(1-naphtalèneméthyl)amino]butyronitrile; ou
un sel pharmaceutiquement acceptable de ceux-ci.